# EUROPEAN PATENT APPLICATION

(11) **EP 1 197 504 A1**
(43) Date of publication of application: **17.04.2002**
(21) Application number: 00944326.8
(22) Date of filing: 07.07.2000
(51) Int. Cl.: C08F 291/00, C08L 101/00, C08G 81/02, C08F 12/22, C07D 211/26, C07D 303/22, C07D 307/89, C07D 405/12, C07C 309/15, C07C 305/04, C07C 233/38, C07C 233/44, C07C 235/10, C07C 255/37, C07C 255/42, C07C 255/29, C07C 265/04, C07C 265/10, C07C 265/12, C07C 217/08, C07C 229/08

(54) **THERMOPLASTIC POLYMER HAVING POLAR GROUP, USE THEREOF, AND UNSATURATED COMPOUNDS HAVING POLAR GROUP**

(30) Priority: 08.07.1999 JP 19469399; 15.10.1999 JP 29418199; 15.10.1999 JP 29419399; 15.10.1999 JP 29436199; 24.04.2000 JP 2000122977; 12.05.2000 JP 2000144629; 12.05.2000 JP 2000144630
(71) Applicant: Mitsui Chemicals, Inc., Tokyo 100-6070 (JP)
(72) Inventor: OTA, Seiji, Mitsui Chemicals, Inc., Kuga-gun, Yamaguchi 740-0061 (JP); MATSUNAGA, Shinya, Mitsui Chemicals, Inc., Ichihara-shi, Chiba 299-0108 (JP); MIYAZAKI, Kazuhisa, Mitsui Chemicals, Inc., Sodegaura-shi, Chiba 299-0265 (JP); ISHITOKU, Takeshi, Mitsui Chemicals, Inc., Sodegaura-shi, Chiba 299-0265 (JP); HATA, Eiichiro, Mitsui Chemicals, Inc., Sodegaura-shi, Chiba 299-0265 (JP)
(74) Representative: Cresswell, Thomas Anthony
(86) International application number: JP0004537
(87) International publication number: WO0104170

(57) **Abstract**

The present invention is intended to provide a polar group-containing thermoplastic polymer having high adhesion to metals and polar polymers, uses thereof and a polar group-containing unsaturated compound. The polar group-containing thermoplastic polymer is one obtained by graft modifying a thermoplastic polymer with at least one kind of a polar group-containing unsaturated compound represented by the following formula (I):

A-X-R²-(Y)ₚ-R¹ (I)

wherein A is a group containing a double bond, X is -COO-, -OCO-, -CO-, -O- or the like, R² is a divalent group containing a carbon atom or a single bond, Y is -CO-, -COO-, -OCO-, -O- or the like, p is 0 or 1, R¹ is a polar group selected from a carboxylic anhydride group, an epoxy group and the like or an alkyl, cycloalkyl or aryl group containing a polar group, and the bond chain linking the double bond in A to the polar group in R¹ is constituted of 6 or more atoms.

## Description

### TECHNICAL FIELD

The present invention relates to polar group-containing thermoplastic polymers, uses thereof and polar group-containing unsaturated compounds. More particularly, the invention relates to polar group-containing thermoplastic polymers modified with specific polar group-containing unsaturated compounds, compositions comprising the polar group-containing thermoplastic polymers and other thermoplastic resin, molded products comprising the compositions, adhesive resin compositions, polar group-containing unsaturated compounds having polymerizability, compounds for radical modification comprising the polar group-containing unsaturated compounds, and polymer modifiers.

### BACKGROUND ART

Introduction of various polar groups into thermoplastic polymers such as polyolefins to enhance adhesion to metals and other resins or compatibility therewith has been generally carried out. In particular, thermoplastic polymers containing a group derived from a carboxylic acid or its derivative in the molecule and thermoplastic polymers containing an epoxy group in the molecule are improved in the properties of adhesion to metals, nylon and ethylene/vinyl alcohol copolymers. Therefore, they have been widely used for adhesive layers of various laminates, and the laminates have been molded into films used as food packaging materials.

As the thermoplastic polymers containing a group derived from a carboxylic acid or its derivative in the molecule, there are known a high-pressure radical copolymer of ethylene and acrylic acid and a modified polyolefin obtained by grafting a polyolefin with maleic anhydride. As the thermoplastic polymers containing an epoxy group in the molecule, there are known a high-pressure radical copolymer of ethylene and glycidyl methacrylate and a modified polyolefin obtained by graft polymerizing a polyolefin with glycidyl methacrylate. In some uses, however, it cannot be said that these polar group-containing thermoplastic polymers have sufficient adhesion properties.

Under such circumstances, the present inventors have earnestly studied, and as a result, they have found that a polar group-containing thermoplastic polymer obtained by modifying a thermoplastic polymer with a polar group-containing unsaturated compound of specific structure, which contains a double bond having polymerizability and a functional group and in which atoms of not less than a certain number are present between the double bond and the functional group, have high adhesion strength to metals and polar polymers.

Because of their excellent properties, engineering plastics such as polyamide resins and polycarbonate resins have been widely used in the fields of automobile parts and electric/electronic parts. These automobile parts and electric/electronic parts sometimes need to have impact resistance in views of their functions. In this case, a method of melting the engineering plastics and modified polyolefins, and extruding them is generally conducted. For example, there have been proposed a method of adding an ionomer to a mixture of a polyolefin and a polyamide (Japanese Patent Publication No. 6529/1968) and a method of mixing a polyolefin with a polyamide in the presence of a maleic anhydride-modified polyolefin (Polymer Chemistry, 29, 265 (1972)). In these methods, however, there resides problems in that the compatibility between the polyolefin and the engineering plastic is insufficient and satisfactory mechanical properties cannot be obtained.

Under such circumstances, the present inventors have earnestly studied, and as a result, they have found that a composition comprising a polar group-containing thermoplastic polymer obtained by modifying with a polar group-containing unsaturated compound of specific structure, which contains a double bond having polymerizability and a functional group and in which atoms of not less than a certain number are present between the double bond and the functional group, and another thermoplastic polymer has solved the above problems and is excellent in mechanical properties such as impact strength.

In various fields, compounds containing an unsaturated bond having polymerizability and a polar group in their molecules are used as materials of various functional polyolefins, paints, adhesives and pressure sensitive adhesives. For example, polar group-containing unsaturated compounds, such as a (meth)acryloyl compound having a glycidyl group or a polyoxyalkylene group and maleic anhydride, are used as grafting agents in the preparation of functional polyolefins which are used as additives, compatibilizing agents or adhesive resins, and in the field of paints, these compounds are used as starting monomers of copolymer resins.

Recently, production of novel polar group-containing unsaturated compounds has been tried. For example, unsaturated compounds of novel structure containing polar groups have been proposed for the purpose of improving properties as adhesive resins and compatibilizing agents. However, available compounds having both an unsaturated bond and a polar group in their molecules are restricted, so that novel polar group-containing unsaturated compounds are still desired.

Under such circumstances, the present inventors have earnestly studied, and as a result, they have found that a polar group-containing unsaturated compound of specific structure, which contains a double bond having polymerizability and a functional group and in which atoms of not less than a certain number are present between the double bond and the polyfunctional group, is useful as a material of copolymerizable monomers, grafting agents for polyolefins, paints, adhesives and pressure sensitive adhesives.

### DISCLOSURE OF THE INVENTION

A polar group-containing thermoplastic polymer according to the invention is obtained by graft modifying a thermoplastic polymer with at least one kind of a polar group-containing unsaturated compound represented by the following formula (I):

A-X-R²-(Y)ₚ-R¹ (I)

wherein A is a group containing a double bond,
X is -COO-, -OCO-, -NHCO-, -CO-, -O-, -CONR¹⁰-or-NR¹⁰-, R¹⁰ is a hydrogen atom or a hydrocarbon group, and R¹⁰ may be bonded to A or R² to form a cyclic structure (in this case, R¹⁰ may be a single bond),
R² is a divalent group containing a carbon atom or a single bond,
Y is -CO-, -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
p is 0 or 1,
R¹ is at least one polar group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group, or any one of an alkyl group of 1 to 20 carbon atoms, a cycloalkyl group of 3 to 20 carbon atoms and an aryl group of 6 to 20 carbon atoms each of which contains at least one group selected from these polar groups, and
the bond chain linking the double bond in A to at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group in R¹ is constituted of 6 or more atoms.

In the formula (I) representing the polar group-containing unsaturated compound, the bond chain linking C=O in the carboxylic acid group or the derivative thereof or C=O in the acid anhydride group in R¹ to X, or the bond chain linking the amino group, the cyano group, the isocyanate group, the sulfonic acid group, the metallic salt thereof, the phosphoric acid group, the metallic salt thereof or the epoxy group in R¹ to X is preferably constituted of 5 or more atoms.

In the formula (I) representing the polar group-containing unsaturated compound, R¹ is preferably at least one polar group selected from the group consisting of a carboxylic acid group, a derivative thereof, an acid anhydride group, an amino group, a cyano group, an isocyanate group, a sulfonic acid group, a metallic salt thereof, a phosphoric acid group and a metallic salt thereof, or any one of an alkyl group of 1 to 20 carbon atoms, a cycloalkyl group of 3 to 20 carbon atoms and an aryl group of 6 to 20 carbon atoms each of which contains at least one group selected from these polar groups.

In the formula (I) representing the polar group-containing unsaturated compound, R¹ is also preferably an epoxy group, or any one of an alkyl group of 1 to 20 carbon atoms, a cycloalkyl group of 3 to 20 carbon atoms and an aryl group of 6 to 20 carbon atoms each of which contains an epoxy group. When X is a group other than -NR¹⁰-, R² is preferably a divalent group containing a carbon atom, and when X is -NR¹⁰-, R² is preferably an alkylene group, a cycloalkylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

In the polar group-containing unsaturated compound, A is preferably a group which contains a double bond and is selected from the following groups: 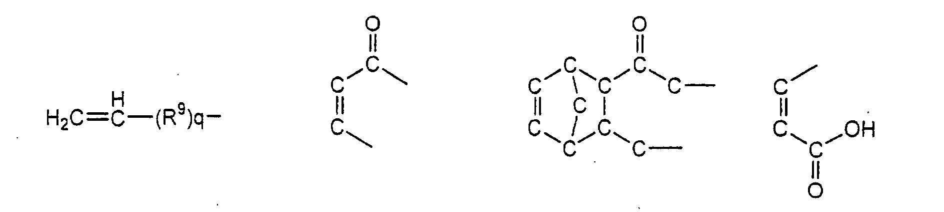 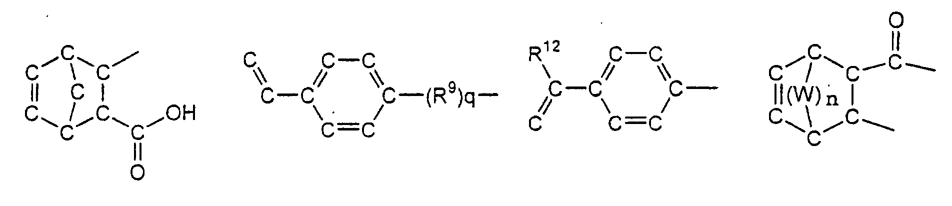 wherein R⁹ is an alkylene group of 1 to 10 carbon atoms, an arylene group of 6 to 10 carbon atoms or a cycloalkylene group of 3 to 10 carbon atoms,
q is 0 or 1,
R¹² is a hydrogen group or a methyl group,
W is -CH(R¹³)- (with the proviso that R¹³ is a hydrogen atom or an alkyl group of 1 to 5 carbon atoms), and
n is 0 or 1.

The polar group-containing unsaturated compound represented by the formula (I) is specifically a compound represented by any one of the following formulas (I-a) to (I-f): 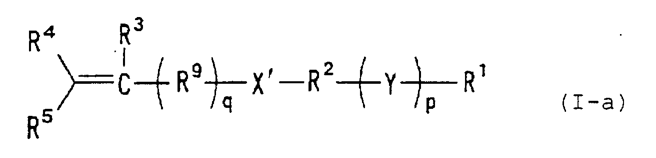 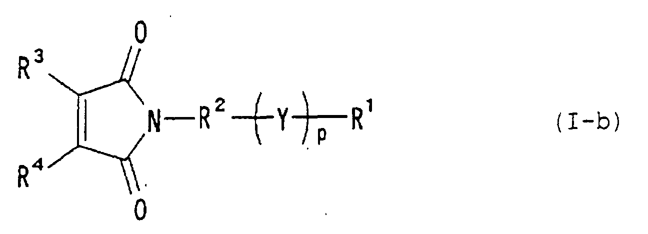 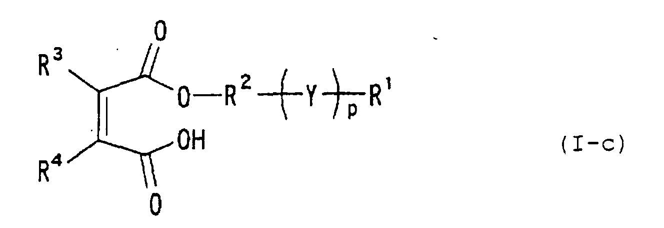 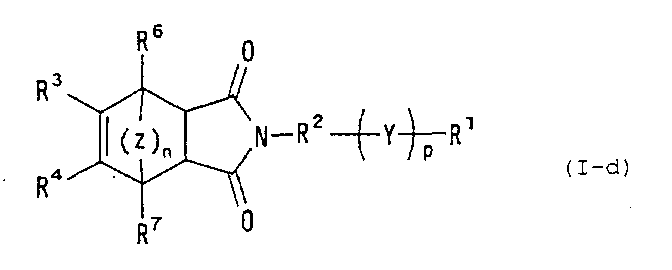 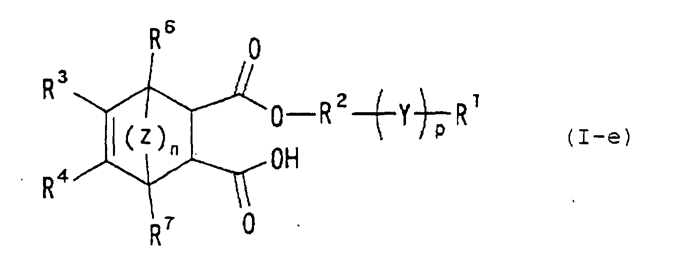 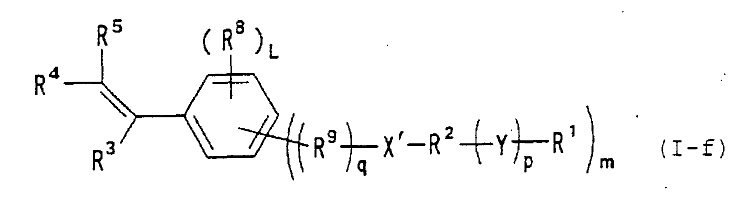 wherein R³, R⁴, R⁵, R⁶, R⁷ and R⁸ are each a hydrogen atom, an alkyl group of 1 to 10 carbon atoms or an aryl group of 6 to 15 carbon atoms,
R⁹ is an alkylene group of 1 to 10 carbon atoms, an arylene group of 6 to 10 carbon atoms or a cycloalkylene group of 3 to 10 carbon atoms,
p is 0 or 1,
q is 0 or 1,
Z is -CH₂- or -O-,
n is an integer of 0 to 2, and in case of n = O, there is no linkage between the carbon to which R⁶ is bonded and the carbon to which R⁷ is bonded,
X' is -COO-, -OCO-, -NHCO-, -CO-, -O- or -CONR¹⁰-, R¹⁰ is a hydrogen atom or a hydrocarbon group, and R¹⁰ may be bonded to a group to which X' is linked or R² to form a cyclic structure (in this case, R¹⁰ may be a single bond),
R² is a divalent group containing a carbon atom,
Y is -CO-, -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
R¹ is any one of an alkyl group of 1 to 20 carbon atoms, a cycloalkyl group of 3 to 20 carbon atoms and an aryl group of 6 to 20 carbon atoms each of which contains at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group,
L is an integer of 0 to 4,
m is an integer of 1 to 5, L+m = 5, and in case of L≥2, each R⁸ may be the same or different, and
the bond chain linking the double bond R⁴R⁵C=CR³ in the formula (I-a), the double bond R⁴C=CR³ in the formula (I-b), the double bond R⁴C=CR³ in the formula (I-c), the double bond R⁴C=CR³ in the formula (I-d), the double bond R⁴C=CR³ in the formula (I-e) or the double bond R⁴R⁵C=CR³ in the formula (I-f) to at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group in R¹ is constituted of 6 or more atoms.

In the polar group-containing unsaturated compound represented by any one of the formulas (I-a) to (I-f), the bond chain linking C=O in the carboxylic acid group, the derivative thereof or the acid anhydride group in R¹ to each of X' in the formula (I-a), N in the formula (I-b), COO of -COOR²- in the formula (I-c), N in the formula (I-d), COO of -COOR²- in the formula (I-e) and X' in the formula (I-f), or the bond chain linking the amino group, the cyano group, the isocyanate group, the sulfonic acid group, the metallic salt thereof, the phosphoric acid group, the metallic salt thereof or the epoxy group in R¹ to each of X' in the formula (I-a), N in the formula (I-b), COO of -COOR²- in the formula (I-c), N in the formula (I-d), COO of -COOR²- in the formula (I-e) and X' in the formula (I-f) is preferably constituted of 5 or more atoms.

In the formula (I) representing the polar group-containing unsaturated compound, R² is preferably a single bond, an alkylene group, a cycloalkylene group, an arylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

In the formula (I), R² is preferably an alkylene group of 2 to 20 carbon atoms, a cycloalkylene group of 3 to 20 carbon atoms, an arylene group of 6 to 20 carbon atoms, a polyoxyalkylene group of 2 to 20 carbon atoms, -R^{a}COOR^{b}- or -R^{a}OCOR^{b}- (with the proviso that R^{a} and R^{b} may be the same or different and are each a group selected from an alkylene group of 2 to 20 carbon atoms, an arylene group of 6 to 20 carbon atoms, a cycloalkylene group of 3 to 20 carbon atoms and a polyoxyalkylene group of 2 to 20 carbon atoms).

The polar group-containing thermoplastic polymer according to the invention is preferably a polymer selected from a polyolefin, a polyester, a polyamide and a polystyrene.

The polar group-containing thermoplastic polymer according to the invention, in another embodiment, has in a polymer side chain a structural part represented by the following formula (I'):

-A'-X-R²-(Y)ₚ-R¹ (I')

wherein A' is a group linking the polymer chain to X or a single bond,
X is -COO-, -OCO-, -NHCO-, -CO-, -O-, -CONR¹⁰-or-NR¹⁰-, R¹⁰ is a hydrogen atom or a hydrocarbon group, and R¹⁰ may be bonded to A or R² to form a cyclic structure (in this case, R¹⁰ may be a single bond),
R² is a divalent group containing a carbon atom or a single bond,
Y is -CO-, -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
p is 0 or 1,
R¹ is any one of an alkyl group of 1 to 20 carbon atoms, a cycloalkyl group of 3 to 20 carbon atoms and an aryl group of 6 to 20 carbon atoms each of which contains at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group, and
the bond chain linking the carbon atom of the polymer main chain, at which the side chain is bonded to the main chain, to at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group in R¹ is constituted of 6 or more atoms.

In the formula (I') in the polar group-containing thermoplastic polymer, the bond chain linking C=O in the carboxylic acid group or the derivative thereof or C=O in the acid anhydride group in R¹ to X, or the bond chain linking the amino group, the cyano group, the isocyanate group, the sulfonic acid group, the metallic salt thereof, the phosphoric acid group, the metallic salt thereof or the epoxy group in R¹ to X is preferably constituted of 5 or more atoms.

In the formula (I') in the polar group-containing thermoplastic polymer, X is preferably-COO-, -OCO-, -NHCO-, -CO-, -O- or -CONR¹⁰-, and R² is preferably an alkylene group of 3 to 20 carbon atoms, an arylene group, a cycloalkylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

An adhesive resin according to the invention comprises any one of the above-mentioned polar group-containing thermoplastic polymers.

The polar group-containing thermoplastic polymer composition according to the invention is a composition comprising:
(A) any one of the above-mentioned polar group-containing thermoplastic polymers, and
(B) a thermoplastic polymer other than the polar group-containing thermoplastic polymer (A),
wherein the polar group-containing thermoplastic polymer (A) is contained in an amount of 1 to 99 % by weight and the thermoplastic polymer (B) is contained in an amount of 99 to 1 % by weight.

In the polar group-containing thermoplastic polymer composition, the thermoplastic polymer (B) is preferably at least one polymer selected from an olefin polymer and a diene rubber.

In the polar group-containing thermoplastic polymer composition, the thermoplastic polymer (B) is preferably a thermoplastic polymer containing, as main recurring units, recurring units derived from a monomer containing a polar group. The thermoplastic polymer (B) is, for example, a polyamide resin, a polyester resin, an acrylic resin, a polyphenylene sulfide resin, a polycarbonate resin, a polyacetal resin, a polyphenylene oxide resin, a polyarylate resin or a polysulfone resin.

The molded product according to the invention is obtained by molding the above-mentioned polar group-containing thermoplastic polymer composition.

The adhesive resin composition according to the invention comprises the above-mentioned polar group-containing thermoplastic polymer composition.

The polar group-containing unsaturated compound according to the invention is represented by the following formula (I):

A-X-R²-(Y)ₚ-R¹ (I)

wherein A is a group containing a double bond,
X is -COO-, -OCO-, -NHCO-, -CO-, -O-, -CONR¹⁰-or-NR¹⁰-, R¹⁰ is a hydrogen atom or a hydrocarbon group, and R¹⁰ may be bonded to A or R² to form a cyclic structure (in this case, R¹⁰ may be a single bond),
R² is a divalent group containing a carbon atom or a single bond,
Y is -CO-, -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
p is 0 or 1,
R¹ is any one of an alkyl group of 1 to 20 carbon atoms, a cycloalkyl group of 3 to 20 carbon atoms and an aryl group of 6 to 20 carbon atoms each of which contains at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group, and
the bond chain linking the double bond in A to at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group in R¹ is constituted of 6 or more atoms.

In the formula (I) representing the polar group-containing unsaturated compound, the bond chain linking C=O in the carboxylic acid group or the derivative thereof or C=O in the acid anhydride group in R¹ to X, or the bond chain linking the amino group, the cyano group, the isocyanate group, the sulfonic acid group, the metallic salt thereof, the phosphoric acid group, the metallic salt thereof or the epoxy group in R¹ to X is preferably constituted of 5 or more atoms.

In the formula (I) representing the polar group-containing unsaturated compound, R¹ is preferably an epoxy group, or any one of an alkyl group of 1 to 20 carbon atoms, a cycloalkyl group of 3 to 20 carbon atoms and an aryl group of 6 to 20 carbon atoms each of which contains an epoxy group. In the formula (I), when X is a group other than -NR¹⁰-, R² is preferably a divalent group containing a carbon atom, and when X is -NR¹⁰-, R² is preferably an alkylene group, a cycloalkylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

In the formula (I) representing the polar group-containing unsaturated compound, R¹ is preferably any one of an alkyl group of 1 to 20 carbon atoms, a cycloalkyl group and an aryl group each of which contains at least one group selected from a carboxylic acid group, a derivative of a carboxylic acid group and an acid anhydride group.

In the formula (I) representing the polar group-containing unsaturated compound, A is preferably a group which contains a double bond selected from the following groups: 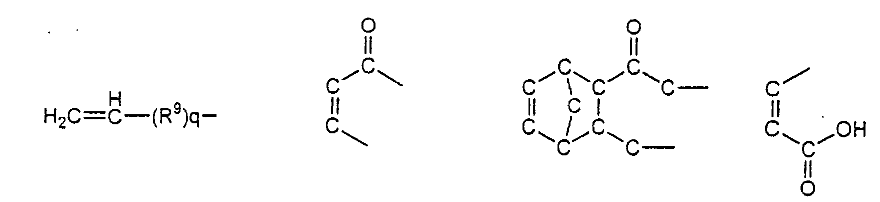 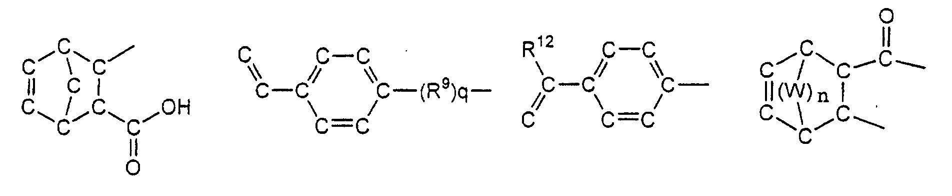 wherein R⁹ is an alkylene group of 1 to 10 carbon atoms, an arylene group of 6 to 10 carbon atoms or a cycloalkylene group of 3 to 10 carbon atoms,
q is 0 or 1,
R¹² is a hydrogen atom or a methyl group,
W is -CH(R¹³)- (with the proviso that R¹³ is a hydrogen atom or an alkyl group of 1 to 5 carbon atoms), and
n is 0 or 1.

The polar group-containing unsaturated compound is, for example, a compound represented by any one of the following formulas (I-a) to (I-f): 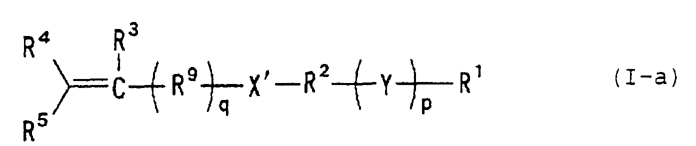 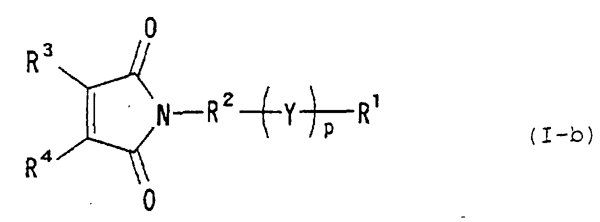 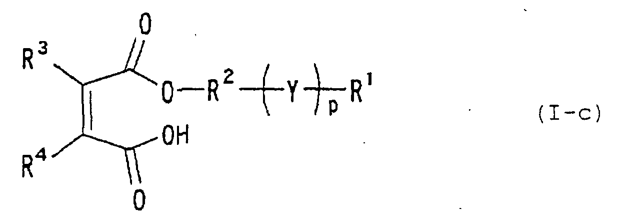 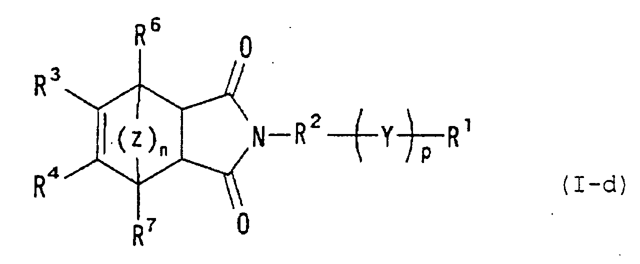 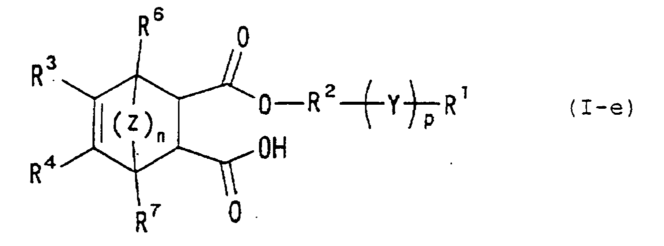 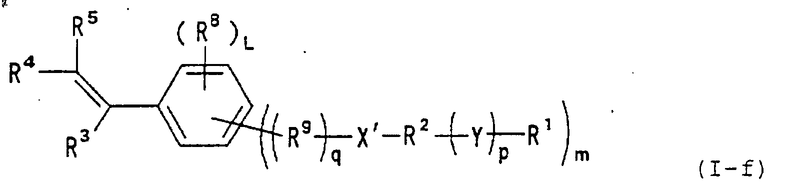 wherein R³, R⁴, R⁵, R⁶, R⁷ and R⁸ are each a hydrogen atom, an alkyl group of 1 to 10 carbon atoms or an aryl group of 6 to 10 carbon atoms,
R⁹ is an alkylene group of 1 to 10 carbon atoms, an arylene group of 6 to 10 carbon atoms or a cycloalkylene group of 3 to 10 carbon atoms,
p is 0 or 1,
q is 0 or 1,
Z is -CH₂- or -O-,
n is an integer of 0 to 2, and in case of n = 0, there is no linkage between the carbon to which R⁶ is bonded and the carbon to which R⁷ is bonded,
X' is -COO-, -OCO-, -NHCO-, -CO-, -O- or -CONR¹⁰-, R¹⁰ is a hydrogen atom or a hydrocarbon group, and R¹⁰ may be bonded to a group to which X' is linked or R² to form a cyclic structure (in this case, R¹⁰ may be a single bond),
R² is a divalent group containing a carbon atom,
Y is -CO-, -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
R¹ is any one of an alkyl group of 1 to 20 carbon atoms, a cycloalkyl group of 3 to 20 carbon atoms and an aryl group of 6 to 20 carbon atoms each of which contains at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group,
L is an integer of 0 to 4,
m is an integer of 1 to 5, L+m = 5, and in case of L≥2, each R⁸ may be the same or different, and
the bond chain linking the double bond R⁴R⁵C=CR³ in the formula (I-a), the double bond R⁴C=CR³ in the formula (I-b), the double bond R⁴C=CR³ in the formula (I-c), the double bond R⁴C=CR³ in the formula (I-d), the double bond R⁴C=CR³ in the formula (I-e) or the double bond R⁴R⁵C=CR³ in the formula (I-f) to at least one group selected from the group consisting of a carboxylic acid group, a derivative thereof, an acid anhydride group, an amino group, a cyano group, an isocyanate group, a sulfonic acid group, ametallic salt thereof, a phosphoric acid group, a metallic salt thereof and an epoxy group in R¹ is constituted of 6 or more atoms.

In the polar group-containing unsaturated compound represented by any one of the formulas (I-a) to (I-f), the bond chain linking C=O in the carboxylic acid group, the derivative thereof or the acid anhydride group in R¹ to each of X' in the formula (I-a), N in the formula (I-b), COO of -COOR²- in the formula (I-c), N in the formula (I-d), COO of -COOR²- in the formula (I-e) and X' in the formula (I-f), or the bond chain linking the amino group, the cyano group, the isocyanate group, the sulfonic acid group, the metallic salt thereof, the phosphoric acid group, the metallic salt thereof or the epoxy group in R¹ to each of X' in the formula (I-a), N in the formula (I-b), COO of -COOR²- in the formula (I-c), N in the formula (I-d), COO of -COOR²- in the formula (I-e) and X' in the formula (I-f) is preferably constituted of 5 or more atoms.

In the formula (I) representing the polar group-containing unsaturated compound, R² is preferably a single bond, an alkylene group, a cycloalkylene group, an arylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

In the formula (I) representing the polar group-containing unsaturated compound, R² is preferably an alkylene group of 2 to 20 carbon atoms, a cycloalkylene group of 3 to 20 carbon atoms, an arylene group of 6 to 20 carbon atoms, a polyoxyalkylene group of 2 to 20 carbon atoms, -R^{a}COOR^{b}- or -R^{a}OCOR^{b}- (with the proviso that R^{a} and R^{b} may be the same or different and are each a group selected from an alkylene group of 2 to 20 carbon atoms, an arylene group of 6 to 20 carbon atoms, a cycloalkylene group of 3 to 20 carbon atoms and a polyoxyalkylene group of 2 to 20 carbon atoms).

Specifically, an example of the polar group-containing unsaturated compound is a polar group-containing unsaturated imide compound represented by the following formula (I-g): 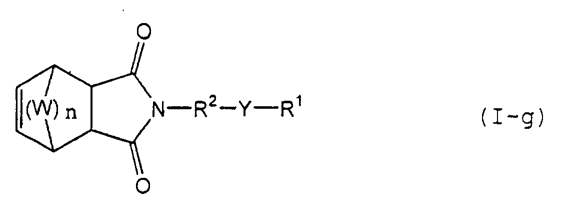 wherein W is -CH(R¹³)- (with the proviso that R¹³ is a hydrogen atom or an alkyl group of 1 to 5 carbon atoms),
n is 0 or 1,
R² is a straight-chain or branched alkylene group or an arylene group,
Y is -O₂C-, and
R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group.

In the formula (I-g) representing the polar group-containing unsaturated imide compound, R¹ is preferably a phthalic anhydride group.

In the formula (I-g) representing the polar group-containing unsaturated imide compound, it is preferable that W is -CH₂-, n is 1, R² is a straight-chain or branched alkylene group, and R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group.

In the formula (I-g) representing the polar group-containing unsaturated imide compound, R² is more preferably a straight-chain alkylene group of 2 to 10 carbon atoms.

Specifically, another example of the polar group-containing unsaturated compound is a polar group-containing vinylbenzene derivative represented by the following formula (I-h): 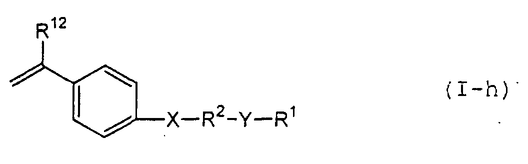 wherein R¹² is a hydrogen atom or a methyl group,
X is an oxygen atom,
R² is single bond or a (poly)oxyalkylene group,
Y is a carbonyl group, and
R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group.

In the formula (I-h) representing the polar group-containing vinylbenzene derivative, R¹² is preferably a methyl group.

In the formula (I-h) representing the polar group-containing vinylbenzene derivative, the (poly)oxyalkylene group indicated by R² is preferably a group represented by the following formula (1) or (2):

-(CH₂CH₂O)ₙ- (1)

wherein n is an integer of 1 to 10,

-(CH₂CHCH₃O)ₘ- (2)

wherein m is an integer of 1 to 10.

In the formula (I-h) representing the polar group-containing vinylbenzene derivative, R¹ is preferably any one of an alkyl group of 1 to 20 carbon atoms, a cycloalkyl group of 3 to 20 carbon atoms and an aryl group of 6 to 20 carbon atoms each of which contains a phthalic anhydride group.

The polar group-containing thermoplastic polymer according to the invention, in another embodiment, has a recurring unit represented by the following formula (I-h') : 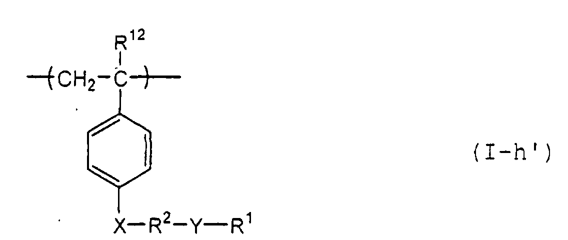 wherein R¹² is a hydrogen atom or a methyl group,
X is an oxygen atom
R² is single bond or a (poly)oxyalkylene group,
Y is a carbonyl group,
R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group, and
the bond chain linking the carbon of a carbonyl group in the carboxylic anhydride group or the carbon of a carbonyl group in the carboxyl group to X is constituted of 5 or more atoms.

Specifically, a further example of the polar group-containing unsaturated compound is a glycidyl polyester acrylate compound represented by the following formula (I-i): 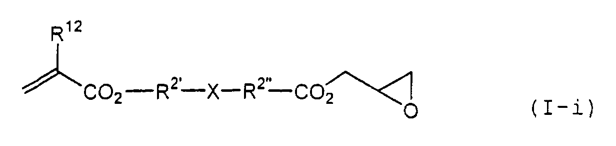 wherein R¹² is a hydrogen atom or a methyl group,
R^{2'} is a straight-chain or branched alkylene group, an oxyalkylene group, an arylene group or a cycloalkylene group,
X is -CO₂- or -O₂C-, and
R^{2"} is a straight-chain or branched alkylene group, an oxyalkylene group, an arylene group or a cycloalkylene group.

In the formula (I-i) representing the glycidyl polyester acrylate compound, it is preferable that R¹² is a hydrogen atom or a methyl group, R^{2'} is a straight-chain or branched alkylene group, an oxyalkylene group or a cycloalkylene group, X is -O₂C-, and R^{2"} is a straight-chain alkylene group.

In the formula (I-i) representing the glycidyl polyester acrylate compound, it is also preferable that R¹² is a hydrogen atom, R²' is a straight-chain alkylene group of 2 to 4 carbon atoms, X is -O₂C-, and R^{2"} is a straight-chain alkylene group of 2 to 4 carbon atoms.

In the formula (I-i) representing the glycidyl polyester acrylate compound, it is more preferable that R¹² is a hydrogen atom, R^{2'} is ethylene, X is -O₂C-, and R^{2"} is ethylene.

A process for preparing a polar group-containing unsaturated compound according to the invention comprises:
allowing acrylic acid or methacrylic acid to react with an alkylene oxide or an alkylene carbonate or allowing an acryloyl halide to react with an alkanediol to obtain a terminal hydroxyl group-containing acrylate,
allowing a terminal hydroxyl group of the terminal hydroxyl group-containing acrylate to react with an acid anhydride to obtain a carboxyl group,
then allowing to react with epichlorohydrin in the presence of a base to obtain the glycidyl polyester acrylate compound represented by the formula (I-i).

A compound for radical reaction according to the invention comprises any one of the above-mentioned polar group-containing unsaturated compounds.

A polymer modifier according to the invention comprises any one of the above-mentioned polar group-containing unsaturated compounds.

### BRIEF DESCRIPTION OF THE DRAWINGS

Fig. 1 is a chart of a NMR spectrum of p-isopropenylphenyltrimellitic anhydride (compound (a)).
Fig. 2 is a chart of a NMR spectrum of p-isopropenylphenoxyethyltrimellitic anhydride (compound (b)).

### BEST MODE FOR CARRYING OUT THE INVENTION

The polar group-containing thermoplastic polymer according to the invention, uses thereof and the polar group-containing unsaturated compound are described hereinafter.

The polar group-containing thermoplastic polymer of the invention is obtained by graft modifying a thermoplastic polymer with at least one kind of a polar group-containing unsaturated compound represented by the formula (I) described below.

First, the polar group-containing unsaturated compound used for preparing the polar group-containing thermoplastic polymer of the invention is described. The polar group-containing thermoplastic polymer also is the polar group-containing unsaturated compound of the invention.

### Polar group-containing unsaturated compound

The polar group-containing unsaturated compound used in the invention and the polar group-containing unsaturated compound of the invention are each represented by the following formula (I).

A-X-R²-(Y)ₚ-R¹ (I)

In the formula (I), A is a group containing a double bond. This double bond is preferably one capable of performing radical reaction. The radical reaction is, for example, radical polymerization. The double bond capable of performing radical reaction is, for example, a carbon double bond.

The atoms for forming the double bond may have substituent groups, and the substituent groups may be bonded to form a cyclic structure. The cyclic structure may be a cyclic structure containing a double bond or a cyclic structure containing X.

Examples of A include -C(R^{A})=CR^{B}R^{C}, -COC(R^{D}) =CR^{E}R^{F}, -C(R^{G})=CR^{H}COOH,

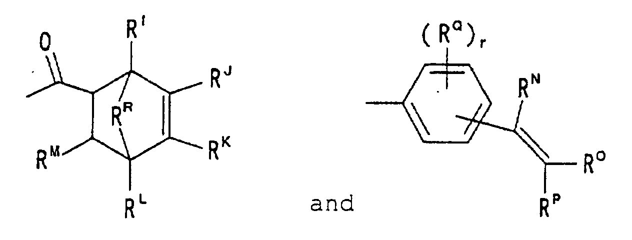

In the above formulas, R^{A} to R^{P} are each a hydrogen atom or a hydrocarbon group, R^{Q} is a hydrocarbon group, R^{R} is a hydrocarbon group or an oxygen atom, and r is an integer of 1 to 4.

Specifically, an example of A is the following one.

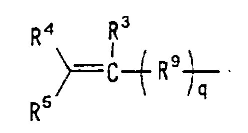

In this case, the compound corresponds to a polar group-containing unsaturated compound represented by the later-described formula (I-a).

Specifically, another example of A is the following one.

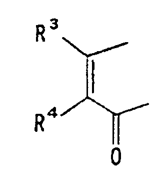

In this case, the compound wherein X is -CONR¹⁰- and R¹⁰ is a single bond and is bonded to A to form a cyclic structure corresponds to a polar group-containing unsaturated compound represented by the later-described formula (I-b).

Specifically, a further example of A is the following one.

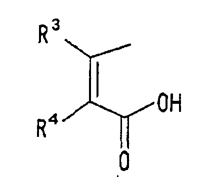

In this case, the compound wherein X is -COO- corresponds to a polar group-containing unsaturated compound represented by the later-described formula (I-c).

Specifically, a still further example of A is the following one.

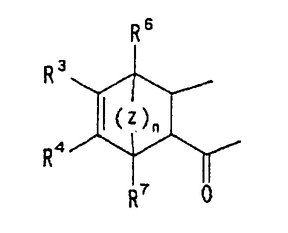

In this case, the compound wherein X is -CONR¹⁰- and R¹⁰ is a bond and is bonded to A to form a cyclic structure corresponds to a polar group-containing unsaturated compound represented by the later-described formula (I-d).

Specifically, a still further example of A is the following one.

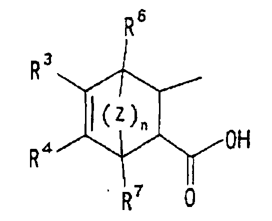

In this case, the compound wherein X is -COO- corresponds to a polar group-containing unsaturated compound represented by the later-described formula (I-e).

Specifically, a still further example of A is the following one.

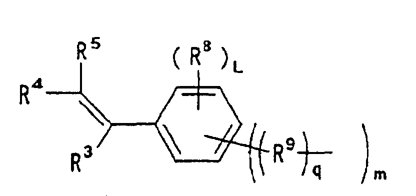

In this case, the compound corresponds to a polar group-containing unsaturated compound represented by the later-described formula (I-f).

In the above formulas, R³, R⁴, R⁵, R⁶, R⁷, R⁸, R⁹, Z, n, L and q have the same meanings as those in the later-described formulas (I-a) to (I-f), and their meanings will be described later.

A is preferably a group which contains a double bond and is selected from the following groups.

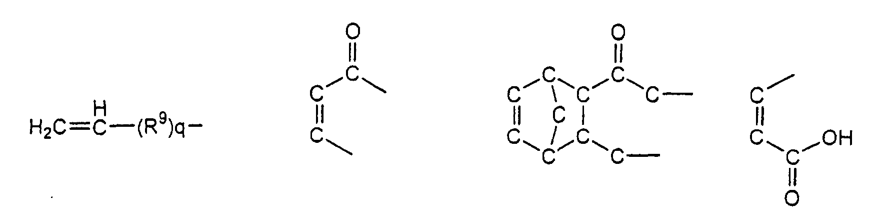

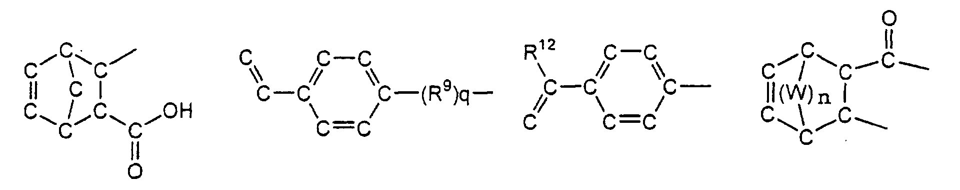

In the above formulas, R⁹ is an alkylene group of 1 to 10 carbon atoms, an arylene group of 6 to 10 carbon atoms or a cycloalkylene group of 3 to 10 carbon atoms,
q is 0 or 1,
R¹² is a hydrogen atom or a methyl group,
W is -CH(R¹³)- (with the proviso that R¹³ is a hydrogen atom or an alkyl group of 1 to 5 carbon atoms), and
n is 0 or 1.
X is -COO-, -OCO-, -NHCO-, -CO-, -O-, -CONR¹⁰- or -NR¹⁰-.
R¹⁰ is a hydrogen atom or a hydrocarbon group such as an alkyl group, an aryl group or a cycloalkyl group.

Specific examples of the hydrocarbon groups include alkyl groups of 1 to 10 carbon atoms, such as methyl, ethyl, n-propyl, isopropyl, n-butyl, isobutyl, sec-butyl, tert-butyl, neopentyl and n-hexyl; aryl groups of 6 to 10 carbon atoms, such as phenyl and benzyl; and cycloalkyl groups of 3 to 10 carbon atoms, such as cyclohexyl, cyclopentyl and cyclohexylmethyl.

R¹⁰ may be bonded to A or R² to form a cyclic structure. In this case, R¹⁰ is a single bond or a divalent hydrocarbon group such as an alkylene group, an arylene group or a cycloalkylene group.

When R¹⁰ is bonded to A to form a cyclic structure, examples of such cyclic structures include: 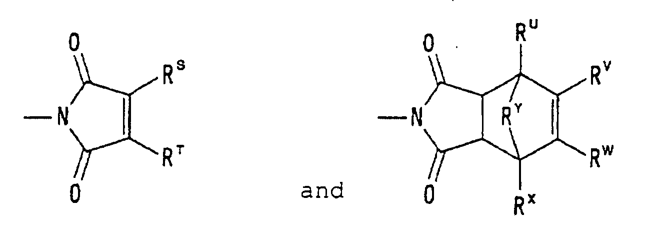 wherein R^{S} to R^{X} are each a hydrogen atom or a hydrocarbon group, and R^{Y} is a hydrocarbon group or an oxygen atom.

R² is a divalent group containing a carbon atom or a single bond, preferably a straight-chain or branched divalent group. Preferred examples of R² include a single bond, an alkylene group, an arylene group, a cycloalkylene group, a polyoxyalkylene group, -R^{a}COOR^{b}- and -R^{a}OCOR^{b}- (with the proviso that R^{a} and R^{b} may be the same or different and are each a group selected from an alkylene group, an arylene group, a cycloalkylene group and a polyoxyalkylene group) . More preferred examples of R² include an alkylene group of 1 to 20 carbon atoms, preferably 3 to 20 carbon atoms, an arylene group of 6 to 20 carbon atoms, preferably 6 to 10 carbon atoms, a cycloalkylene group of 3 to 20 carbon atoms, preferably 3 to 10 carbon atoms, a polyoxyalkylene group of 2 to 20 carbon atoms, -R^{a}COOR^{b}- and -R^{a}OCOR^{b}-. R^{a} and R^{b} are each preferably a group selected from an alkylene group of 1 to 20 carbon atoms, preferably 1 to 10 carbon atoms, an alkylene group of 6 to 20 carbon atoms, preferably 6 to 10 carbon atoms, a cycloalkylene group of 3 to 20 carbon atoms, preferably 3 to 10 carbon atoms and a polyoxyalkylene group of 2 to 20 carbon atoms. Specific examples of such groups include methylene, ethylene, propylene, butylene, hexamethylene, octamethylene, decamethylene, cyclopropylene, cyclobutylene, cyclopentylene, cyclohexylene and cyclooctylene. Of these, ethylene and cyclohexylene are particularly preferable. In one preferred embodiment, R^{a} and R^{b} are each an alkylene group of 1 to 10 carbon atoms.

In the present invention, when R¹ is an epoxy group or any one of an alkyl group of 1 to 20 carbon atoms, a cycloalkyl group of 3 to 20 carbon atoms and an aryl group of 6 to 20 carbon atoms each of which contains an epoxy group, it is preferable that X is a group other than -NR¹⁰-(X is a group of -COO-, -OCO-, -NHCO-, -CO-, -O- or -CONR¹⁰-) and R² is a divalent group containing a carbon atom, and it is also preferable that X is -NR¹⁰- and R² is an alkylene group, a cycloalkylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group, preferably an alkylene group of 1 to 10 carbon atoms).

In the formula (I) representing the polar group-containing unsaturated compound, R² is preferably a single bond, an alkylene group, a cycloalkylene group, an arylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group, preferably an alkylene group of 1 to 10 carbon atoms).

In the formula (I) , R² is preferably an alkylene group of 1 to 20 carbon atoms, preferably 2 to 20 carbon atoms, more preferably 3 to 20 carbon atoms, a cycloalkylene group of 3 to 20 carbon atoms, preferably 3 to 10 carbon atoms, an arylene group of 6 to 20 carbon atoms, preferably 6 to 10 carbon atoms, a polyoxyalkylene group of 2 to 20 carbon atoms, -R^{a}COOR^{b}- or -R^{a}OCOR^{b}- (with the proviso that R^{a} and R^{b} may be the same or different and are each a group selected from an alkylene group of 1 to 20 carbon atoms, preferably 1 to 10 carbon atoms, an arylene group of 6 to 20 carbon atoms, a cycloalkylene group of 3 to 20 carbon atoms and a polyoxyalkylene group of 2 to 20 carbon atoms).

Y is -CO-, -COO-, -OCO-, -O-, -CONR¹¹-, -OCONR¹¹- or -NR¹¹CO-.

R¹¹ is a hydrogen atomor a hydrocarbon group. Examples of the hydrocarbon groups include the same ones as previously described with respect to R¹⁰. R¹¹ may be bonded to R² or R¹ to form a cyclic structure. In this case, R¹¹ is a single bond or a divalent hydrocarbon group that is exemplified with respect to R¹⁰.

p is 0 or 1, preferably 1.

R¹ is at least one polar group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group, or any one of an alkyl group of 1 to 20 carbon atoms, a cycloalkyl group of 3 to 20 carbon atoms and an aryl group of 6 to 20 carbon atoms each of which contains at least one group selected from these polar groups . In one preferred embodiment, R¹ is any one of an alkyl group of 1 to 20 carbon atoms, a cycloalkyl group of 3 to 20 carbon atoms and an aryl group of 6 to 20 carbon atoms each of which contains at least one group selected from the above polar groups.

Examples of the derivatives of the carboxylic acid group include an acid halide, an amide, an imide, an ester and a carboxylic acid metallic salt. In the present invention, R¹ is preferably any one an alkyl group of 1 to 20 carbon atoms, a cycloalkyl group of 3 to 20 carbon atoms and an aryl group of 6 to 20 carbon atoms each of which contains at least one group selected from a carboxylic acid group, a derivative thereof and an acid anhydride group.

The epoxy group is

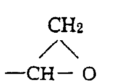

In the polar group-containing unsaturated compound represented by the formula (I), it is an important requisite that the bond chain linking the double bond in A to at least one group selected from the group consisting of a carboxylic acid group, a derivative thereof, an acid anhydride group, an amino group, a cyano group, an isocyanate group, a sulfonic acid group, a metallic salt thereof, a phosphori cacid group, a metallic salt thereof and an epoxy group in R¹ is constituted of 6 or more atoms, preferably 7 or more atoms, more preferably 8 or more atoms, still more preferably 9 or more atoms, particularly preferably 11 or more atoms. The upper limit of the number of atoms is not specifically restricted, but the number of atoms is usually 55 or less, preferably 35 or less.

The bond chain linking C=O in the carboxylic acid group or the derivative thereof or C=O in the acid anhydride group in R¹ to X, or the bond chain linking the amino group, the cyano group, the isocyanate group, the sulfonic acid group, the metallic salt thereof, the phosphoric acid group, the metallic salt thereof or the epoxy group in R¹ to X is desirably constituted of 5 or more atoms, preferably 7 or more atoms, more preferably 8 or more atoms, particularly preferably 9 or more atoms. The upper limit of the number of atoms is not specifically restricted, but the number of atoms is usually 50 or less, preferably 30 or less.

According to the invention, in the polar group-containing unsaturated compound represented by the formula (I), it is preferable that R¹ is at least one polar group selected from the group consisting of a carboxylic acid group, a derivative thereof, an acid anhydride group, an amino group, a cyano group, an isocyanate group, a sulfonic acid group, a metallic salt thereof, a phosphoric acid group and a metallic salt thereof, or any one of an alkyl group of 1 to 20 carbon atoms, a cycloalkyl group of 3 to 20 carbon atoms and an aryl group of 6 to 20 carbon atoms each of which contains at least one group selected from these polar groups.

It is also preferable that R¹ is an epoxy group or any one of an alkyl group of 1 to 20 carbon atoms, a cycloalkyl group of 3 to 20 carbon atoms and an aryl group of 6 to 20 carbon atoms each of which contains an epoxy group. In this case, it is desirable that when X is a group other than -NR¹⁰-, R² is a divalent group containing a carbon atom, and when X is -NR¹⁰-, R² is an alkylene group, a cycloalkylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group, preferably an alkylene group of 1 to 10 carbon atoms).

The "bond chain" used in the invention means a linkage of atomic bonds composed of the smallest number of atoms. In case of, for example, R², among the linkages of atoms linking X to Y, a linkage composed of the smallest number of atoms is a bond chain. Therefore, when R² contains a cyclic bond, there are plural linkages by way of atoms for forming the cyclic bond (e.g., a linkage of atoms of a cyclohexylene group in the right-handed rotation, and a linkage thereof in the left-handed rotation), and of the plural linkages, a linkage composed of the smallest number of atoms is taken as a bond chain. When R¹ contains two or more polar groups selected from C=O that is derived from a carboxylic acid group, its derivative or an acid anhydride group, an amino group, a cyano group, an isocyanate group, a sulfonic acid group, a metallic salt thereof, a phosphoric acid group, a metallic salt thereof and an epoxy group, the bond chain is chosen as a linkage to such a polar group that the number of atoms constituting the bond chain becomes the largest.

The way to count the number of atoms of the bond chain is explained below. The polar group-containing unsaturated compound has, for example, the following formula.

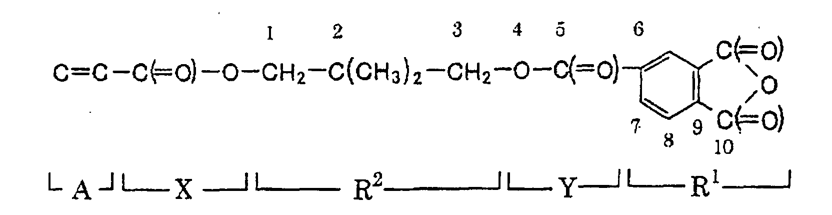

In this case, X is -COO-, and as the C=O of the carboxylic acid group, its derivative or the acid anhydride group in R¹, C=O of numeral 10 in the above formula is chosen. Therefore, the bond chain therebetween is a linkage of atoms consisting of numerals 1-2-3-4-5-6-7-8-9 in the above formula, so that the bond chain is constituted of 9 atoms (carbon, oxygen) . The bond chain in R² is constituted of 3 carbon atoms consisting of numerals 1-2-3 in the above formula. The bond chain linking the double bond in A to C=O of the carboxylic anhydride group in R¹ is constituted of 11 atoms.

The polar group-containing unsaturated compound has, for example, the following formula.

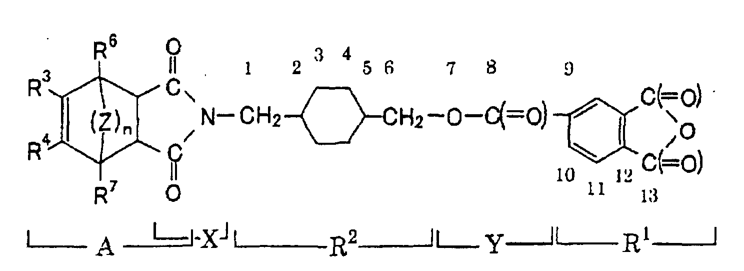

In this case, X is -CON(-)- ((-) designates a single bond), and as the C=O of the carboxylic acid group, its derivative or the acid anhydride group in R¹, C=O of numeral 13 in the above formula is chosen. Therefore, the bond chain is a linkage of atoms consisting of numerals 1-2-3-4-5-6-7-8-9-10-11-12 in the above formula, so that the bond chain is constituted of 12 atoms (carbon, oxygen) . The bond chain in R² is constituted of 6 carbon atoms consisting of numerals 1-2-3-4-5-6 in the above formula. The bond chain linking the double bond in A to C=O of the carboxylic anhydride group in R¹ is constituted of 16 atoms.

The polar group-containing unsaturated compound has, for example, the following formula.

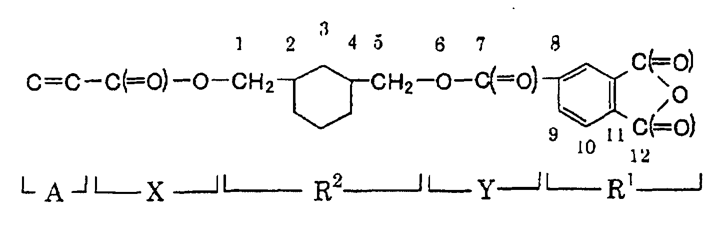

In this case, X is -COO-, and as the C=O of the carboxylic acid group, its derivative or the acid anhydride group in R¹, C=O of numeral 12 in the above formula is chosen. Therefore, the bond chain is a linkage of atoms consisting of numerals 1-2-3-4-5-6-7-8-9-10-11 in the above formula, so that the bond chain is constituted of 11 atoms (carbon, oxygen) . The bond chain in R² is constituted of 5 carbon atoms consisting of numerals 1-2-3-4-5 in the above formula. The bond chain linking the double bond in A to C=O of the carboxylic anhydride group in R¹ is constituted of 13 atoms.

The polar group-containing unsaturated compound has, for example, the following formula.

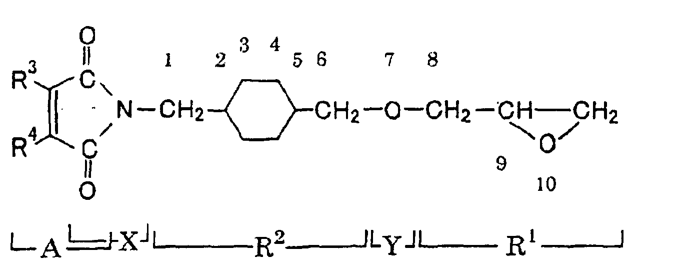

In this case, X is -CON(-)- ((-) designates a single bond), and as the epoxy group in R¹, that of numeral 9 in the above formula is chosen. Therefore, the bond chain is a linkage of atoms consisting of numerals 1-2-3-4-5-6-7-8 in the above formula, so that the bond chain is constituted of 8 atoms (carbon, oxygen). The bond chain in R² is constituted of 6 carbon atoms consisting of numerals 1-2-3-4-5-6 in the above formula. The bond chain linking the double bond in A to the epoxy group in R¹ is constituted of 10 atoms.

In the present invention, of the polar group-containing unsaturated compounds, those represented by the following formulas (I-a) to (I-f) are preferable.

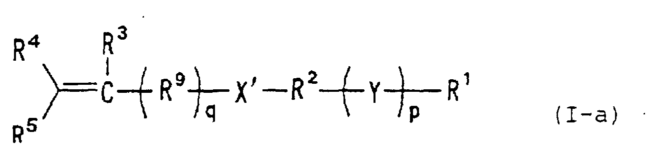

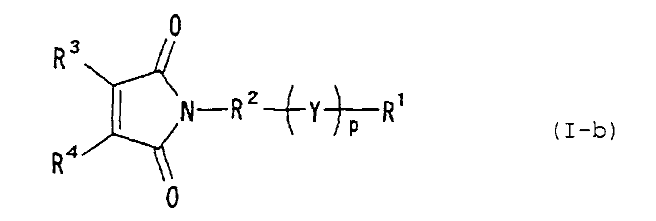

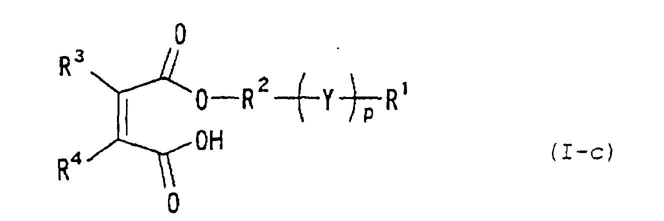

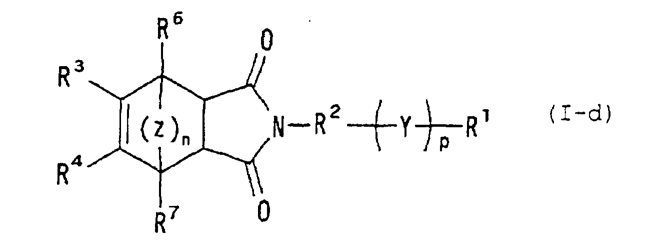

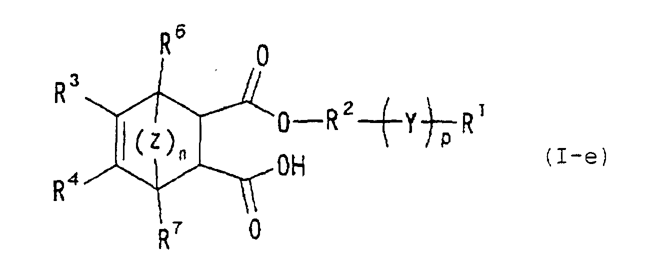

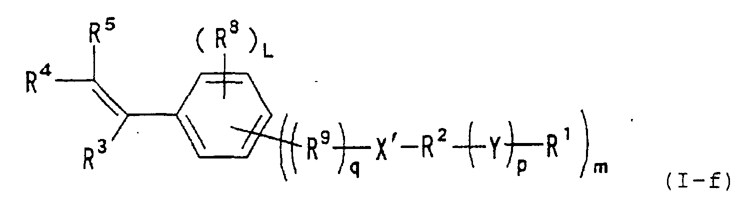

In the formulas (I-a) to (I-f), R³, R⁴, R⁵, R⁶, R⁷ and R⁸ are each a hydrogen atom, a straight-chain or branched alkyl group of 1 to 10 carbon atoms or an aryl group of 6 to 15 carbon atoms, preferably 6 to 10 carbon atoms.

Examples of the alkyl groups include methyl, ethyl, n-propyl, isopropyl, n-butyl, isobutyl, sec-butyl, tert-butyl, neopentyl and n-hexyl.

Examples of the aryl groups include phenyl and benzyl.

q is 0 or 1.

R⁹ is a divalent hydrocarbon group, such as an alkylene group of 1 to 10 carbon atoms, an arylene group of 6 to 10 carbon atoms or a cycloalkylene group of 3 to 10 carbon atoms.

Examples of the alkylene groups include trimethylene, tetramethylene, propylene and ethylethylene. Examples of the arylene groups include phenylene and naphthylene. Examples of the cycloalkylene groups include 1, 4-cyclohexylene and 1,3-cyclohexylene.

n is an integer of 0 to 2.

Z is -CH₂- or -O-, and in case of n = 0, there is no linkage between the carbon to which R⁶ is bonded and the carbon to which R⁷ is bonded.

X' is -COO-, -OCO-, -NHCO-, -CO-, -O- or -CONR¹⁰-.

R¹⁰ is a hydrogen atom or a hydrocarbon group.

The hydrocarbon group is, for example, an alkyl group of 1 to 10 carbon atoms, an aryl group of 6 to 10 carbon atoms or a cycloalkyl group of 3 to 10 carbon atoms. Specific examples thereof include alkyl groups, such as methyl, ethyl, n-propyl, isopropyl, n-butyl, isobutyl, sec-butyl, tert-butyl, neopentyl and n-hexyl; aryl groups, such as phenyl and benzyl; and cycloalkyl groups, such as cyclohexyl, cyclopentyl and cyclohexylmethyl. R¹⁰ may be bonded to a group to which X' is linked or R² to form a cyclic structure. In this case, R¹⁰ is a single bond or the above-mentioned divalent hydrocarbon group such as an alkylene group, an arylene group or a cycloalkylene group.

R² is a single bond or a divalent group containing a carbon atom, preferably a straight-chain or branched divalent group in which the number of atoms of the bond chain is 1 or more, preferably 2 or more, more preferably 3 or more. Preferred examples of R² include a single bond, an alkylene group of 1 to 20 carbon atoms, preferably 2 to 20 carbon atoms, more preferably 3 to 20 carbon atoms, an arylene group of 6 to 20 carbon atoms, preferably 6 to 10 carbon atoms, a cycloalkylene group of 3 to 20 carbon atoms, preferably 3 to 10 carbon atoms, and a polyoxyalkylene group of 2 to 20 carbon atoms. Examples of the alkylene groups include trimethylene, tetramethylene, propylene and ethylethylene. Examples of the arylene groups include phenylene and naphthylene. Examples of the cycloalkylene groups include 1,4-cyclohexylene and 1,3-cyclhexylene. Examples of the polyoxyalkylene groups include polyethyleneoxy and polybutyleneoxy.

Other preferred examples of R² are -R^{a}COOR^{b}- and -R^{a}OCOR^{b}-. R^{a} and R^{b} are each desired to be an alkylene group of 1 to 20 carbon atoms, preferably 1 to 10 carbon atoms, an arylene group of 6 to 20 carbon atoms, preferably 6 to 10 carbon atoms, a cycloalkylene group of 3 to 20 carbon atoms, preferably 3 to 10 carbon atoms or a polyoxyalkylene group of 2 to 20 carbon atoms. Specific examples of such groups include methylene, ethylene, propylene, butylene, hexamethylene, octamethylene, decamethylene, cyclopropylene, cyclobutylene, cyclopentylene, cyclohexylene and cyclooctylene. Of these, ethylene and cyclohexylene are particularly preferable.

Y is -CO-, -COO-, -OCO-, -O-, -CONR¹¹-, -OCONR¹¹- or -NR¹¹CO-.

R¹¹ is a hydrogen atom or a hydrocarbon group. Examples of the hydrocarbon groups include the same ones as previously described with respect to R¹⁰. R¹¹ may be bonded to R² or R¹ to form a cyclic structure. In this case, R¹¹ is a single bond or a divalent hydrocarbon group that is exemplified as R¹⁰. p is 0 or 1, preferably 1.

R¹ is any one of an alkyl group of 1 to 20 carbon atoms, a cycloalkyl group of 3 to 20 carbon atoms and an aryl group of 6 to 20 carbon atoms each of which contains at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group.

Examples of the derivatives of the carboxylic acid group include an acid halide, an amide, an imide, an ester and a carboxylic acid metallic salt. In the present invention, R¹ is preferably any one of an alkyl group of 1 to 20 carbon atoms, a cycloalkyl group of 3 to 20 carbon atoms and an aryl group of 6 to 20 carbon atoms each of which contains at least one group selected from a carboxylic acid group, a derivative thereof and an acid anhydride group.

R¹ is also preferably any one of an alkyl group of 1 to 20 carbon atoms, a cycloalkyl group of 3 to 20 carbon atoms and an aryl group of 6 to 20 carbon atoms each of which contains at least one group selected from an amino group and an isocyanate group.

The alkyl group containing at least one group selected from the group consisting of a carboxylic acid group, a derivative thereof and an acid anhydride group is, for example, -CH₂CH₂-COOH derived from succinic anhydride.

Examples of the cycloalkyl groups containing at least one group selected from the group consisting of a carboxylic acid group, a derivative thereof and an acid anhydride group include:

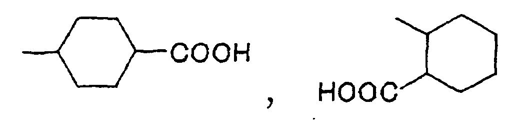

Examples of the aryl groups containing at least one group selected from the group consisting of a carboxylic acid group, a derivative thereof and an acid anhydride group include: 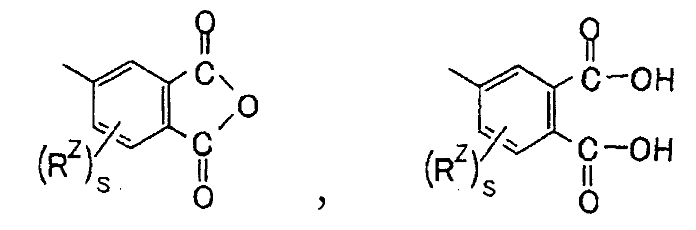 and esters or metallic salts of the above compounds.

Examples of R^{Z} include an alkyl group of 1 to 10 carbon atoms, an aryl group of 6 to 10 carbon atoms, a cycloalkyl group of 3 to 10 carbon atoms, a COOH group, a derivative thereof; groups wherein an alkyl group of 1 to 10 carbon atoms, an aryl group of 6 to 10 carbon atoms and a cycloalkyl group of 3 to 10 carbon atoms are substituted with a COOH group and a derivative thereof; an OH group, a derivative thereof; and groups wherein an alkyl group of 1 to 10 carbon atoms, an aryl group of 6 to 10 carbon atoms and a cycloalkyl group of 3 to 10 carbon atoms are substituted with an OH group and a derivative thereof. When s is more than 1, R^{Z} and another R^{Z} may form a cyclic structure.

As the alkyl groups of 1 to 10 carbon atoms, the aryl groups of 6 to 10 carbon atoms and the cycloalkyl groups of 3 to 10 carbon atoms, there can be mentioned alkyl groups, such as methyl, ethyl, n-propyl, isopropyl, n-butyl, isobutyl, sec-butyl, tert-butyl, neopentyl and n-hexyl; aryl groups, such as phenyl and benzyl; and cycloalkyl groups, such as cyclohexyl, cyclopentyl and cyclohexylmethyl.

Examples of the derivatives of COOH group include an alkyl ester group and a salt of a metal such as Li, Na or K.

Examples of the derivatives of OH group include an alkyl ether group and a silyl ether group, such as:

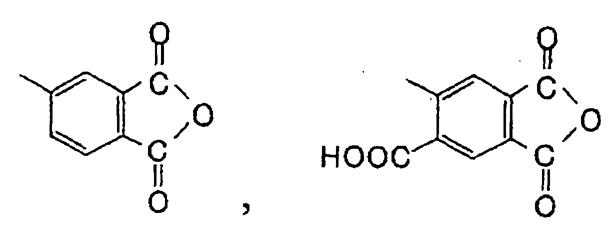

Between Y and any one of the above groups, an alkylene group of 1 to 20 carbon atoms may be bonded as a spacer.

In the formula (I-f), L is an integer of 0 to 4, m is an integer of 1 to 5, and L+M = 5. In case of L≥2, each R⁸ may be the same or different.

The bond chain linking the double bond R⁴R⁵C=CR³ in the formula (I-a), the double bond R⁴C=CR³ in the formula (I-b), the double bond R⁴C=CR³ in the formula (I-c), the double bond R⁴C=CR³ in the formula (I-d), the double bond R⁴C=CR³ in the formula (I-e) or the double bond R⁴R⁵C=CR³ in the formula (I-f) to at least one group selected from the group consisting of a carboxylic acid group, a derivative thereof, an acid anhydride group, an amino group, a cyano group, an isocyanate group, a sulfonic acid group, ametallic salt thereof, a phosphoric acid group, a metallic salt thereof and an epoxy group in R¹ is desirably constituted of 6 or more atoms, preferably 7 or more atoms, more preferably 8 or more atoms, particularly preferably 9 or more atoms. The upper limit of the number of atoms is not specifically restricted, but the number of atoms is usually 55 or less, preferably 35 or less.

The bond chain linking each of X' in the formula (I-a), N in the formula (I-b), COO of -COOR²- in the formula (I-c), N in the formula (I-d) ; COO of -COOR²- in the formula (I-e) and X' in the formula (I-f) to C=O in the carboxylic acid group, the derivative thereof or the acid anhydride group in R¹, or the bond chain linking each of X' in the formula (I-a), N in the formula (I-b), COO of -COOR²- in the formula (I-c), N in the formula (I-d), COO of -COOR²- in the formula (I-e) and X' in the formula (I-f) to the amino group, the cyano group, the isocyanate group, the sulfonic acid group, the metallic salt thereof, the phosphoric acid group, the metallic salt thereof or the epoxy group in R¹ is constituted of 5 ormore atoms, preferably 7 or more atoms, more preferably 8 or more atoms, particularly preferably 9 or more atoms. The upper limit of the number of atoms is not specifically restricted, but the number of atoms is usually 50 or less, preferably 30 or less.

Of the polar group-containing unsaturated compounds represented by the formulas (I-a) to (I-f), those represented by the formulas (I-a), (I-b), (I-c) and (I-f) are preferable. The polar group-containing unsaturated compound represented by the formula (I-a) is one of particularly preferred embodiments, and the compound represented by the formula (I-b), (I-c) or (I-f) also is a preferred embodiment.

When the polar group contained in R¹ is a carboxylic acid group, a derivative thereof, an acid anhydride group, an amino group or a cyano group, the polar group-containing unsaturated compound represented by the formula (I-a), (I-b), (I-c) or (I-f) is particularly preferable. In this case, the polar group-containing unsaturated compound represented by the formula (I-a) is one of particularly preferred embodiments, and the compound represented by the formula (I-b), (I-c) or (I-f) also is a preferred embodiment.

When the polar group contained in R¹ is an isocyanate group, a sulfonic acid group, a derivative thereof, a phosphoric acid group, a metallic salt thereof or an epoxy group, the polar group-containing unsaturated compound represented by the formula (I-a), (I-b), (I-c) or (I-f) is particularly preferable. In this case, the polar group-containing unsaturated compound represented by the formula (I-a) is one of particularly preferred embodiments, and the compound represented by the formula (I-b), (I-c) or (I-f) also is a preferred embodiment.

Examples of the polar group-containing unsaturated compounds represented by the formula (I-a) are given below. In those examples, "-O₂C-" means a -O-C(=O)- group, and "-Bu" means a tert-butyl group.

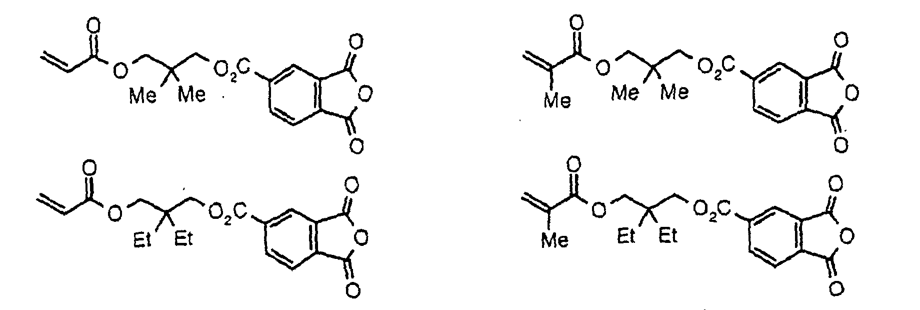

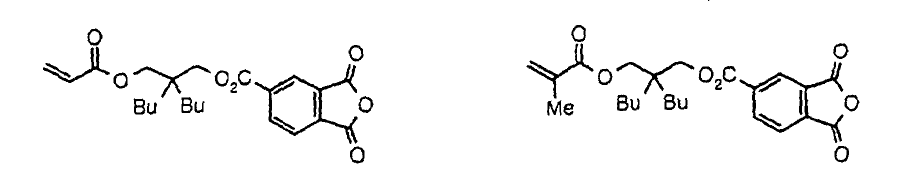

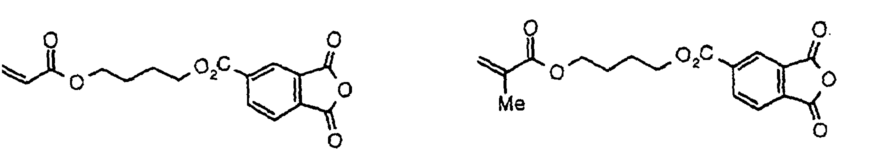

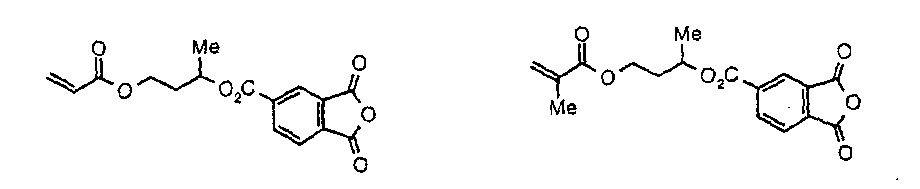

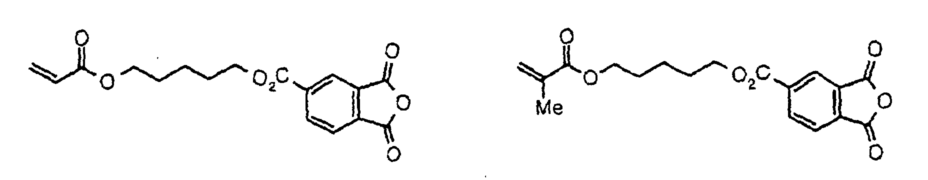

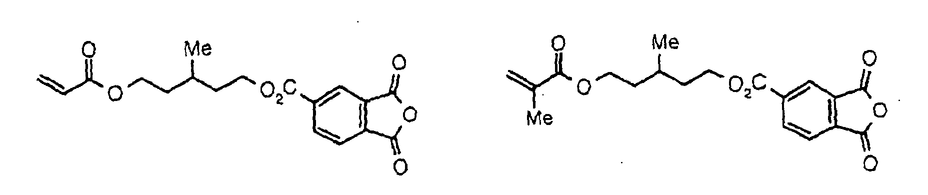

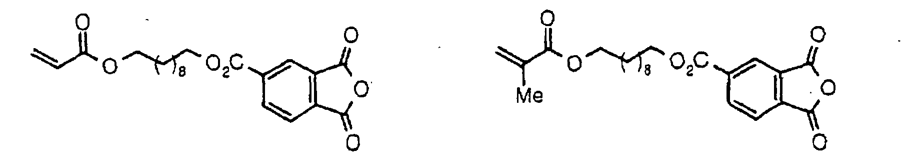

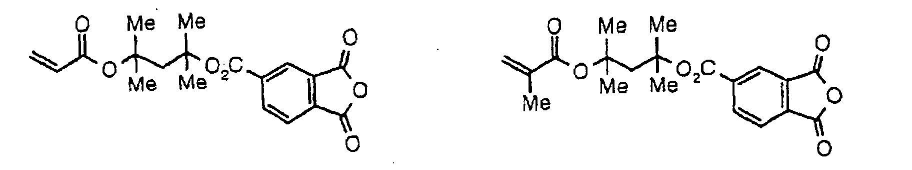

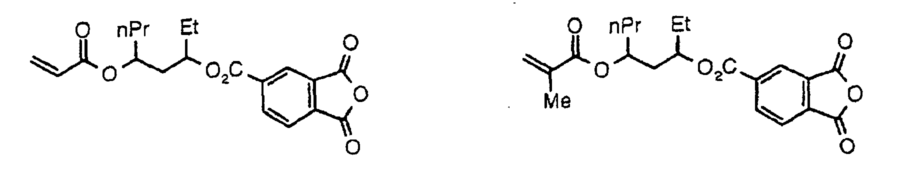

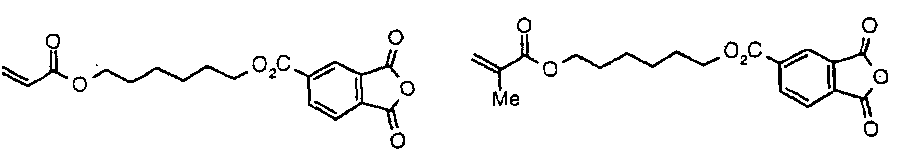

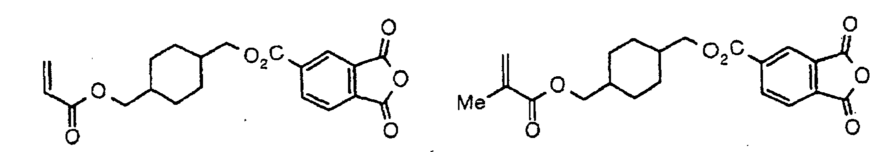

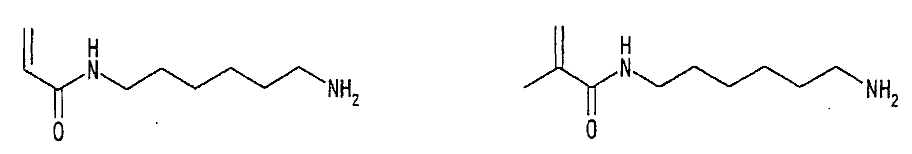

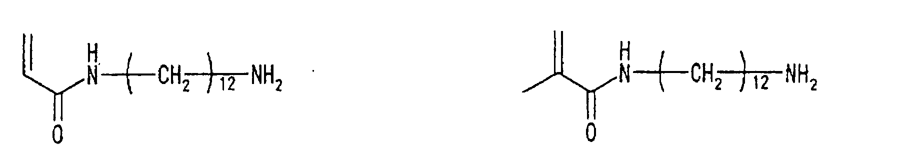

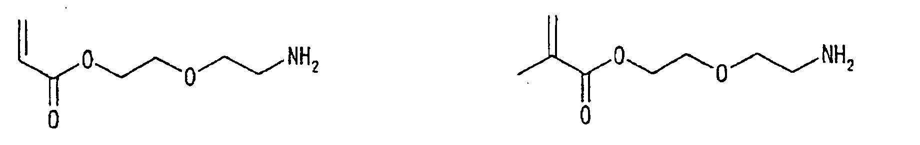

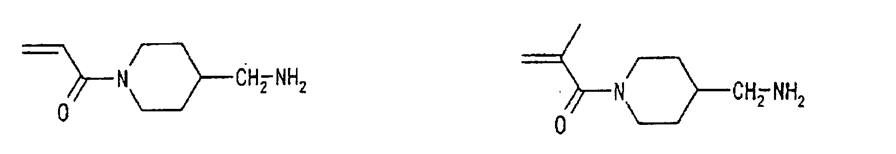

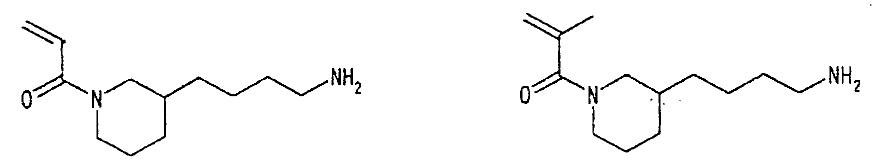

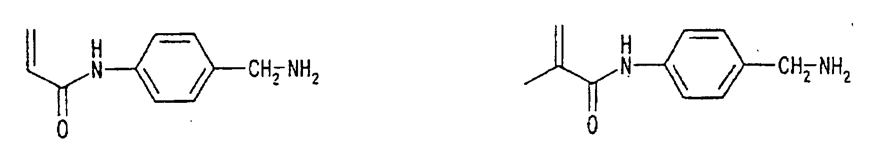

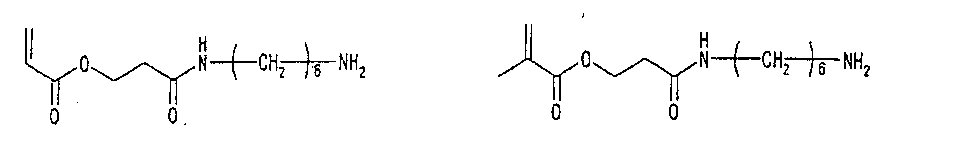

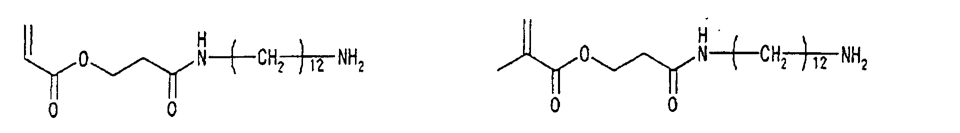

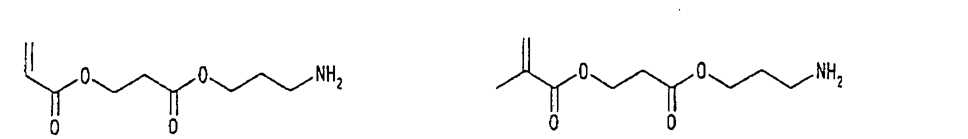

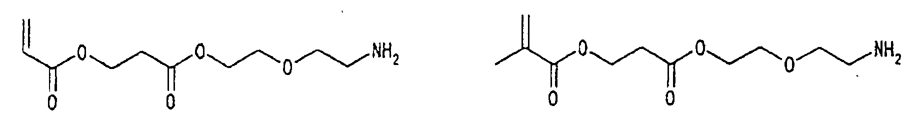

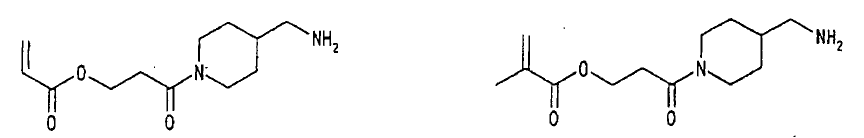

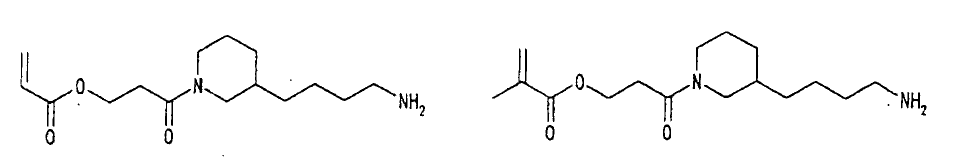

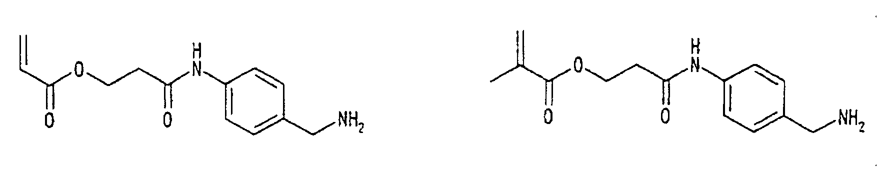

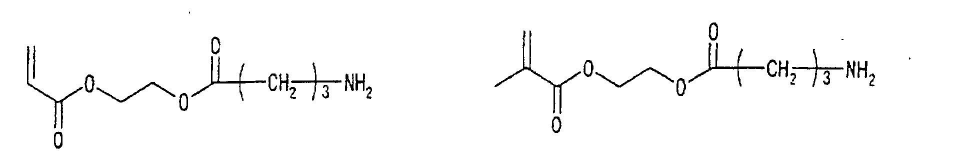

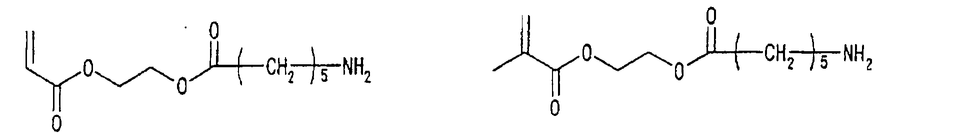

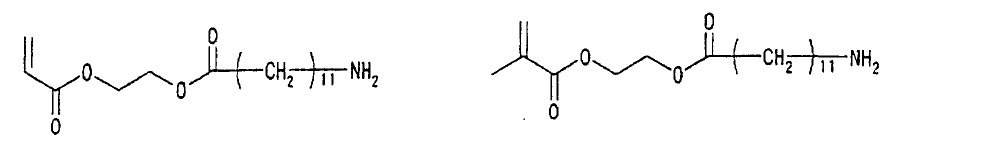

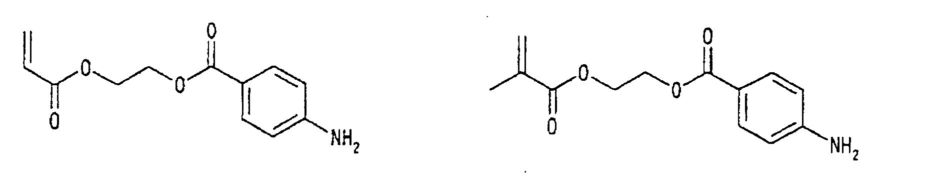

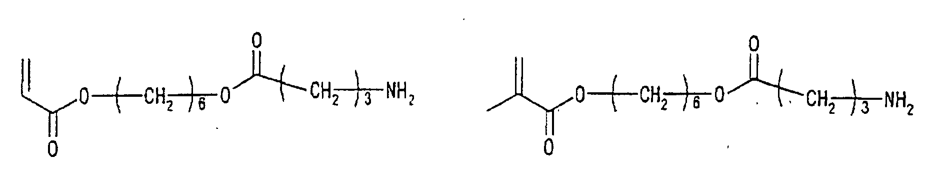

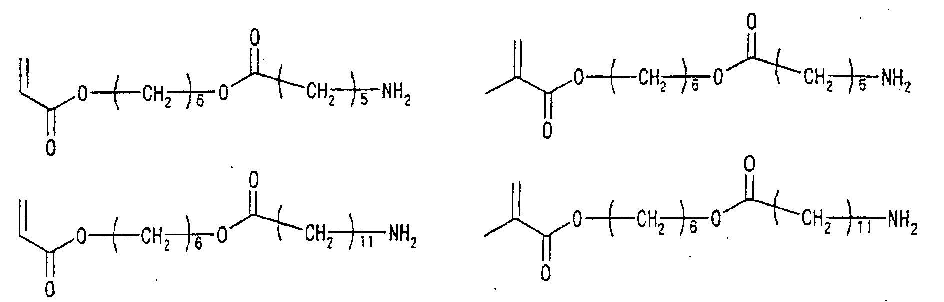

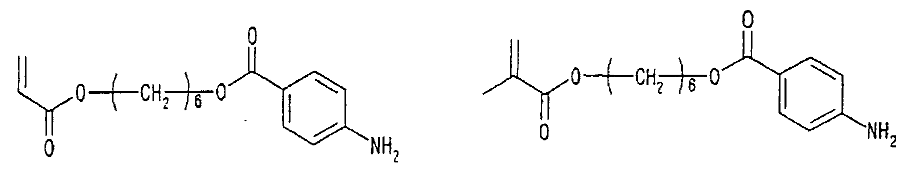

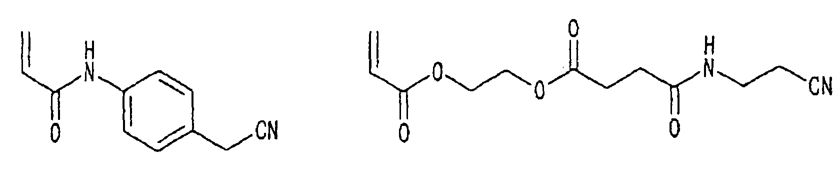

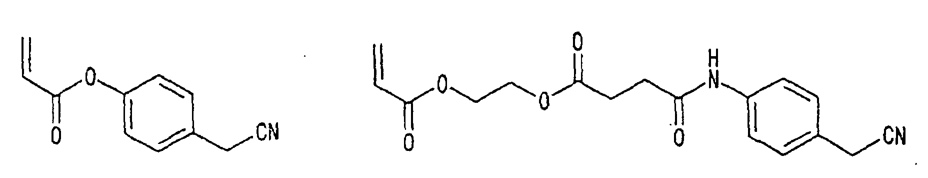

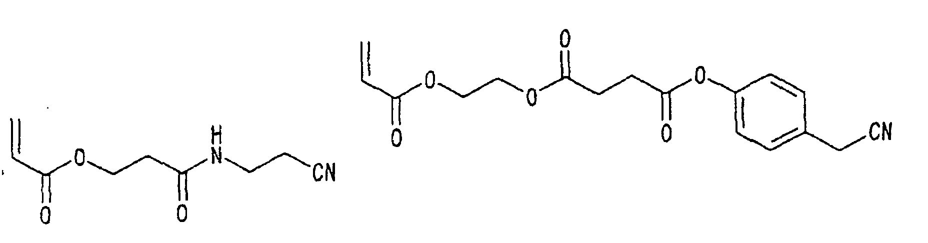

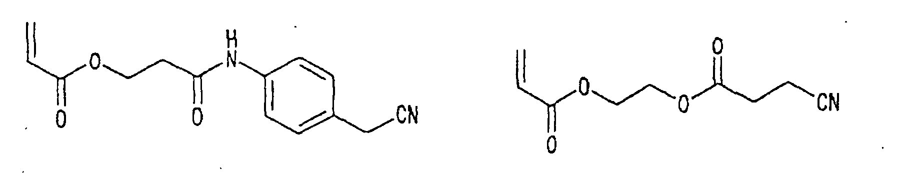

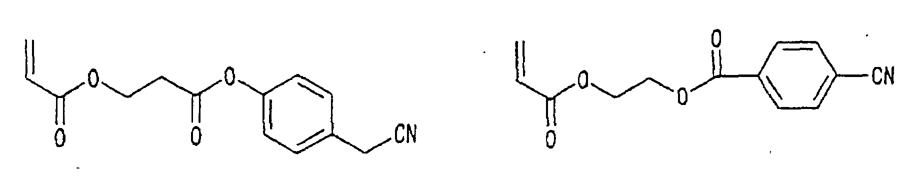

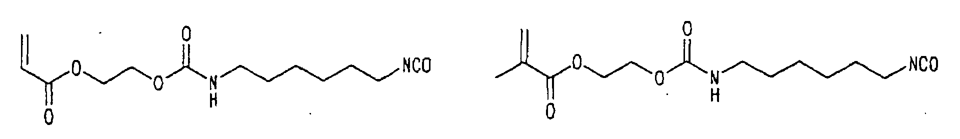

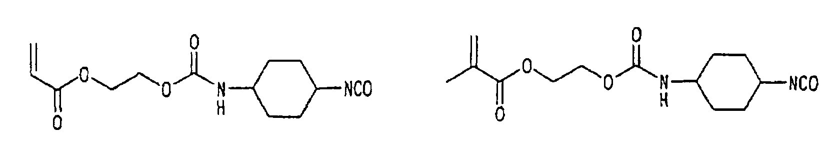

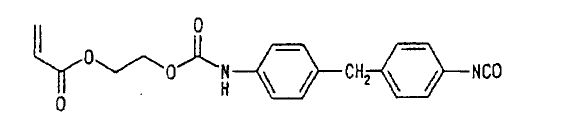

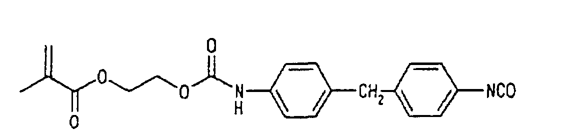

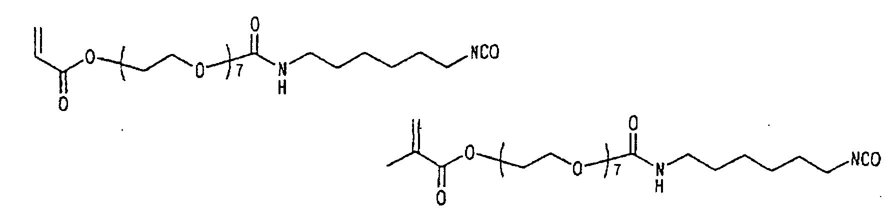

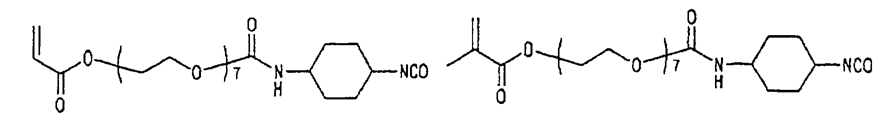

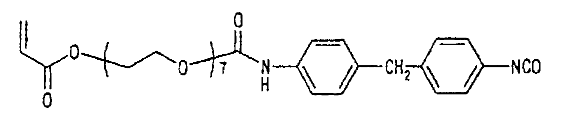

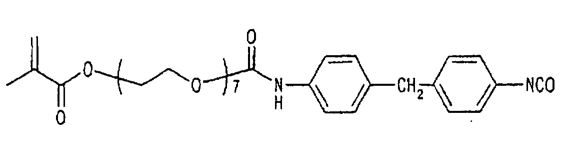

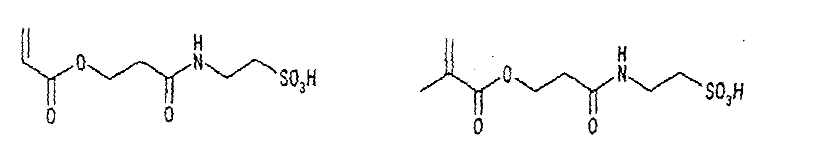

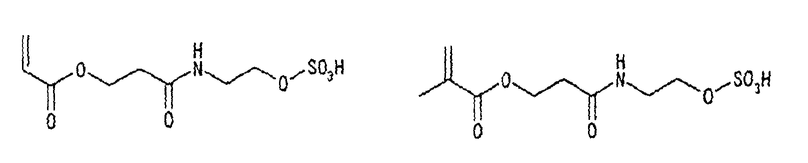

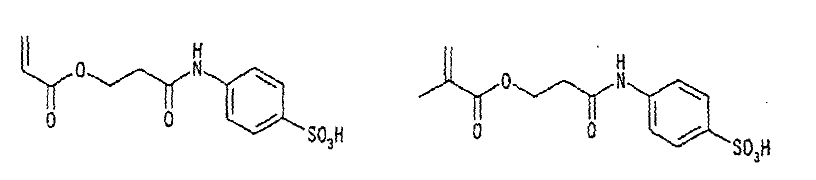

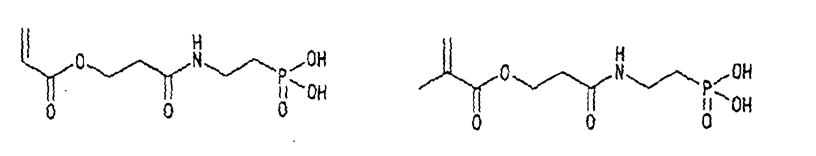

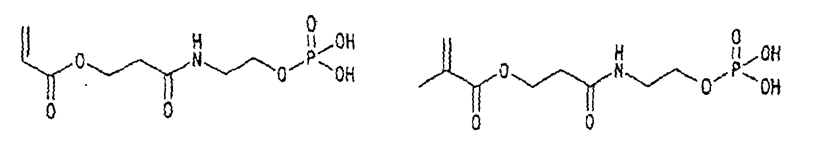

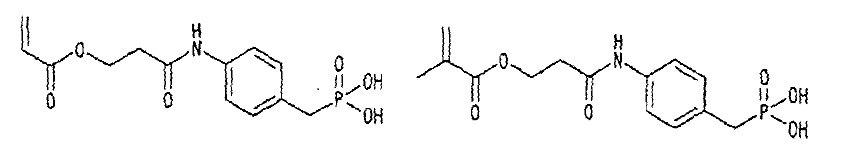

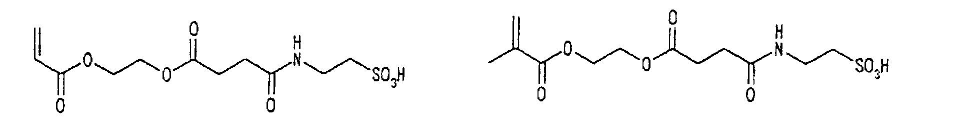

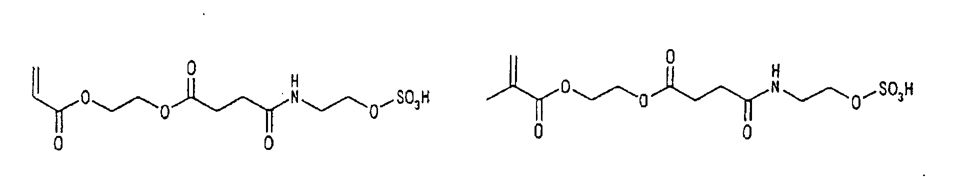

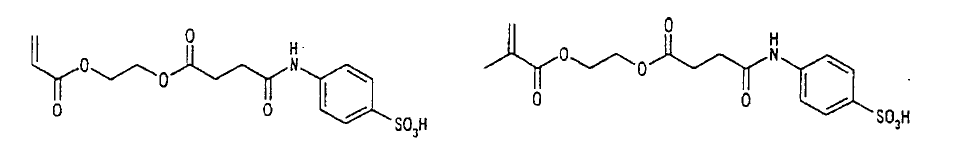

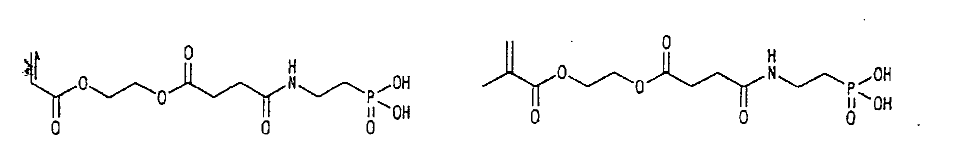

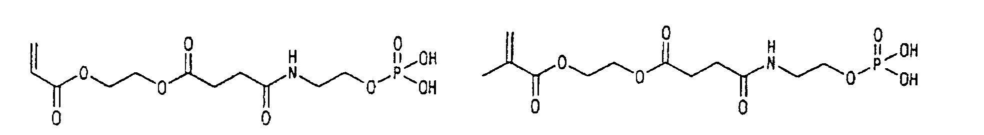

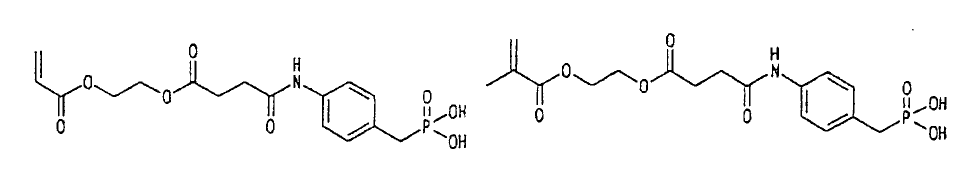

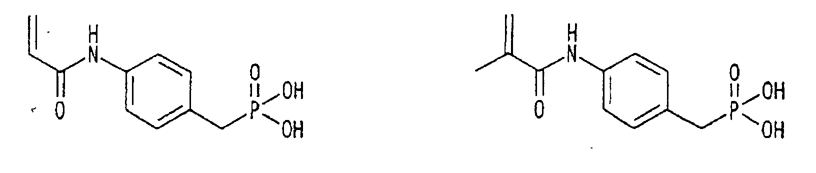

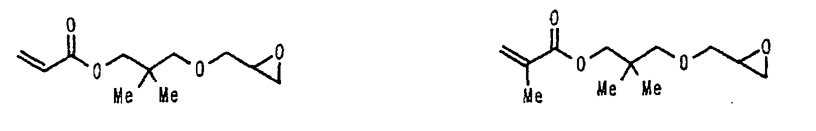

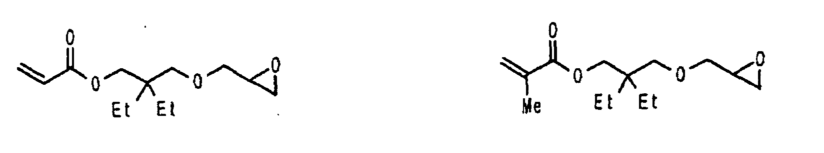

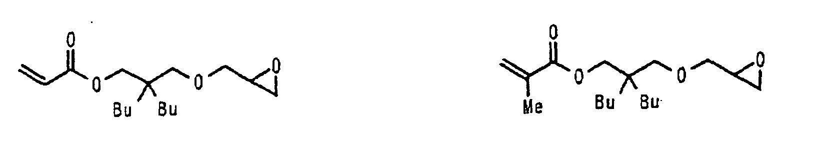

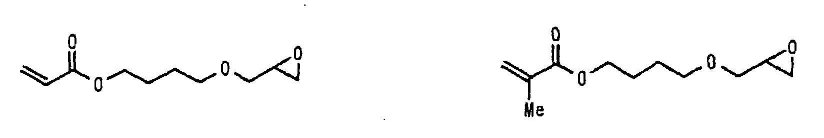

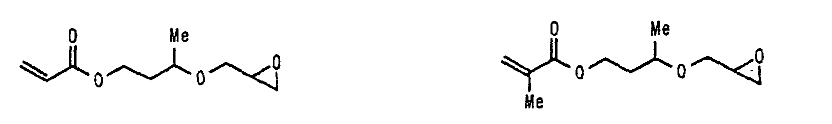

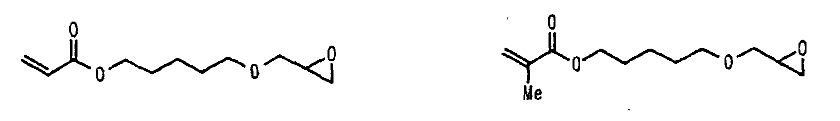

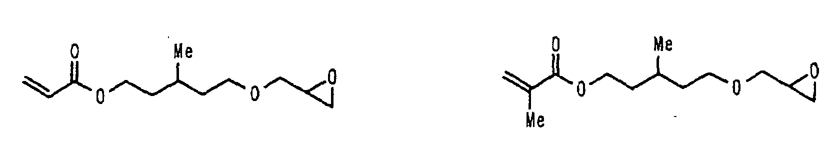

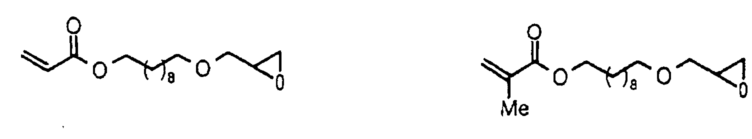

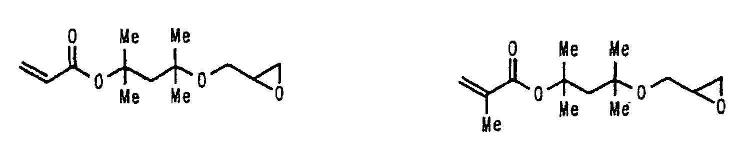

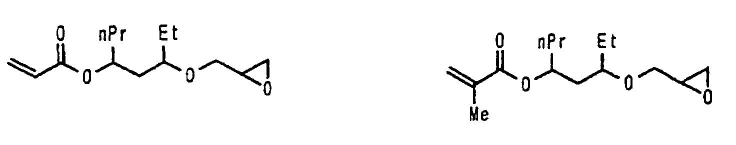

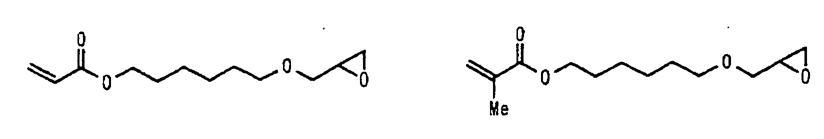

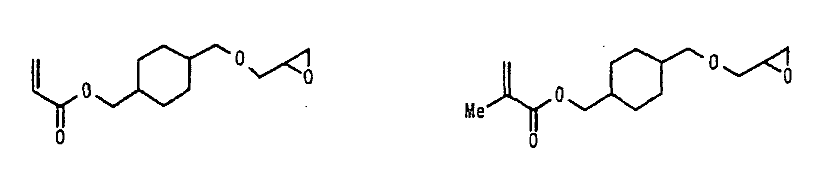

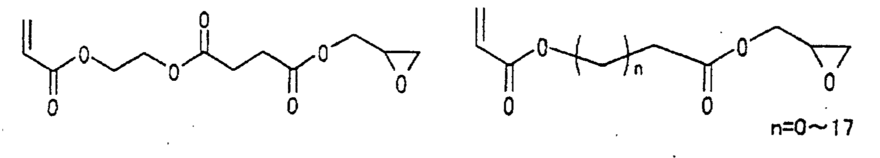

Specific examples thereof include the following ones.

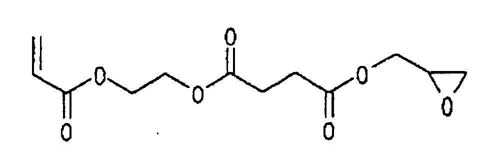

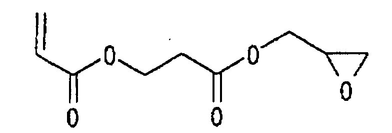

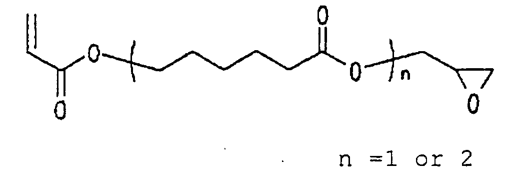

Examples of the polar group-containing unsaturated compounds represented by the formula (I-b) are given below.

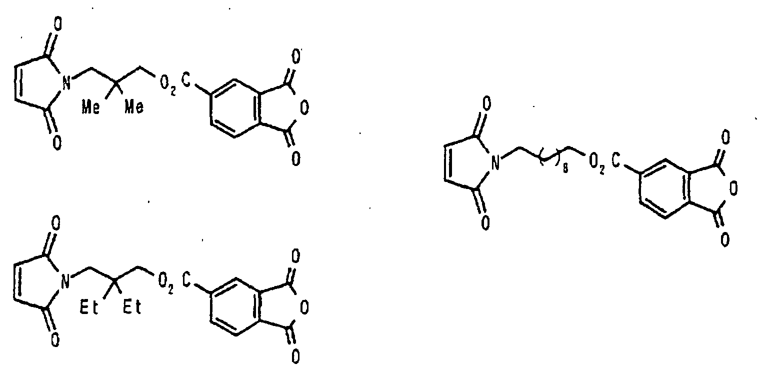

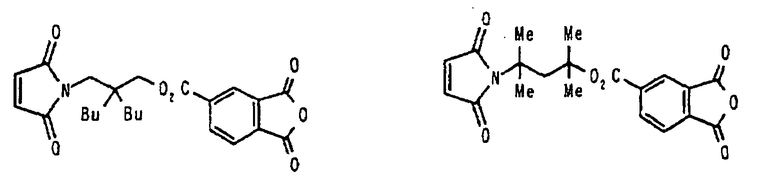

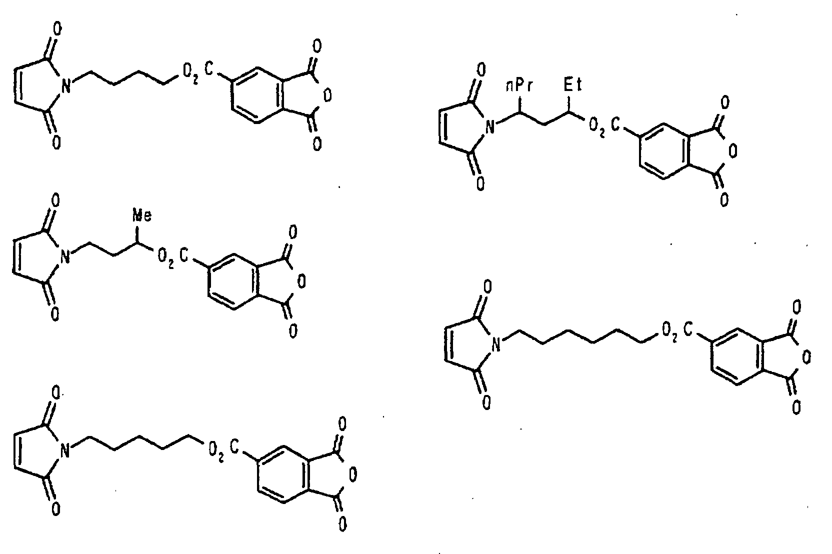

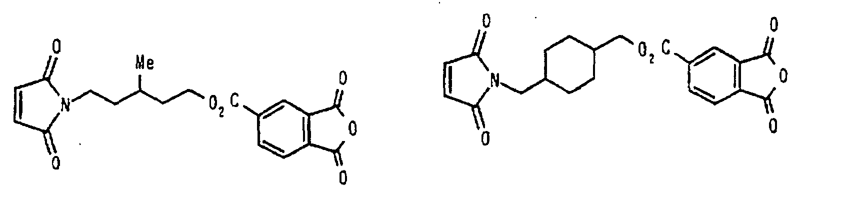

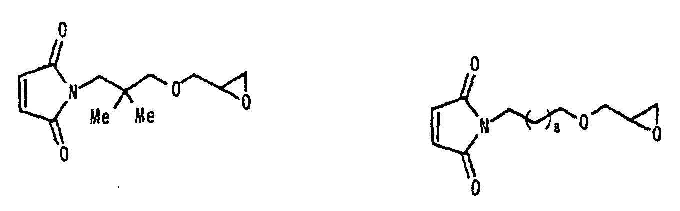

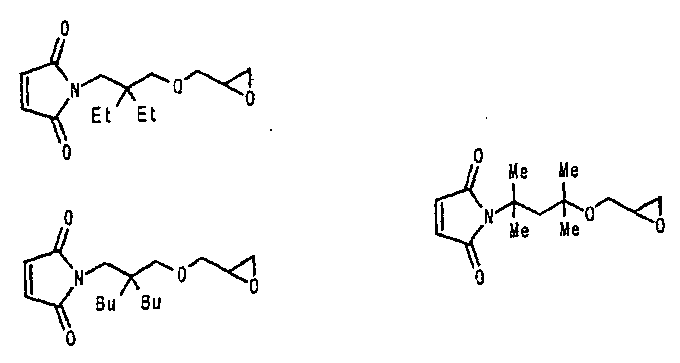

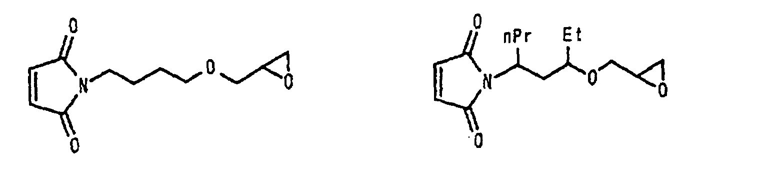

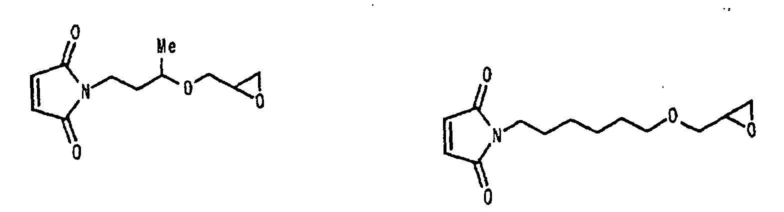

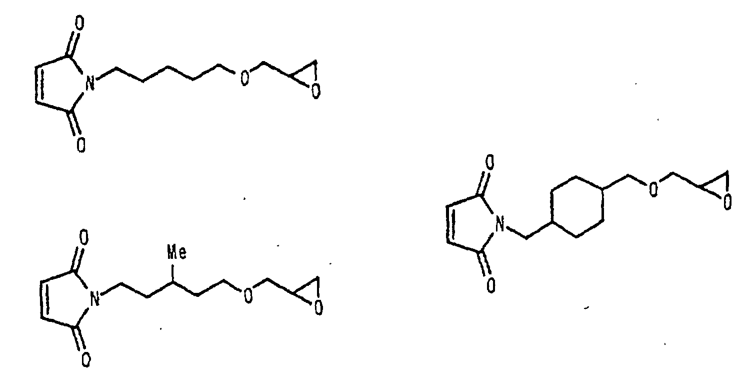

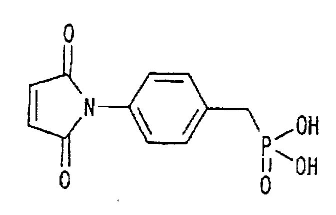

Examples of the polar group-containing unsaturated comp ounds represented by the formula (I-c) are given below.

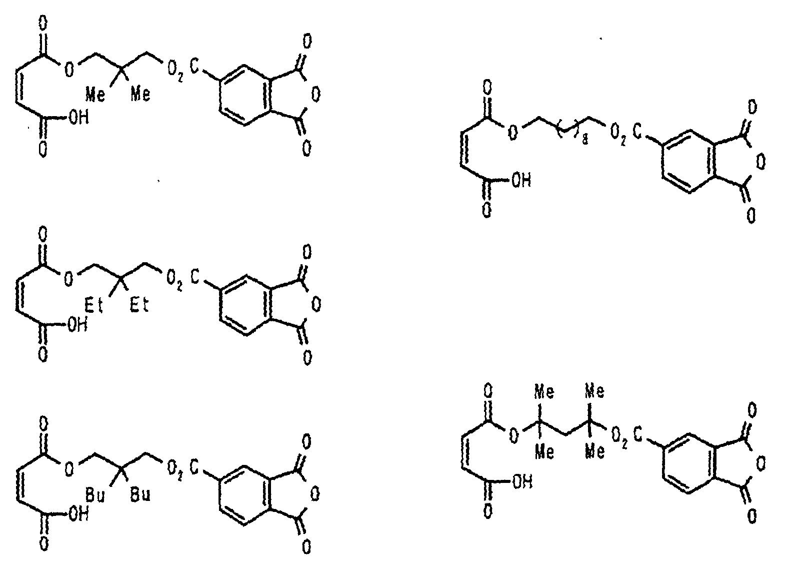

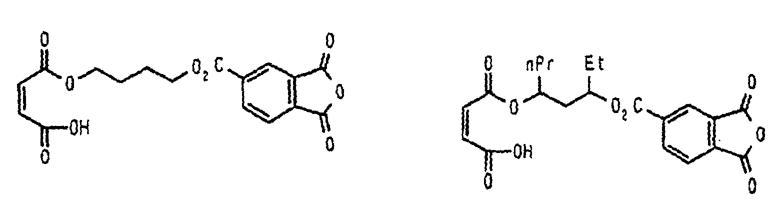

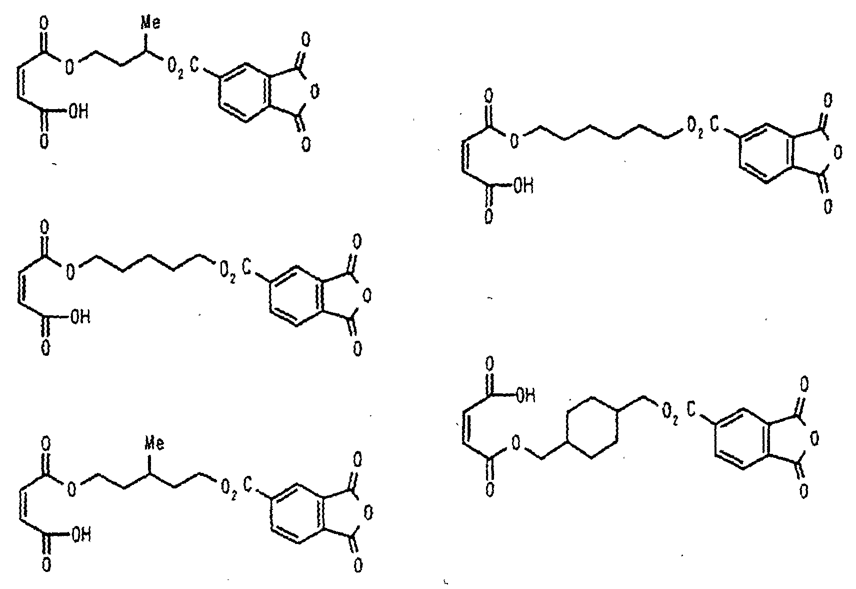

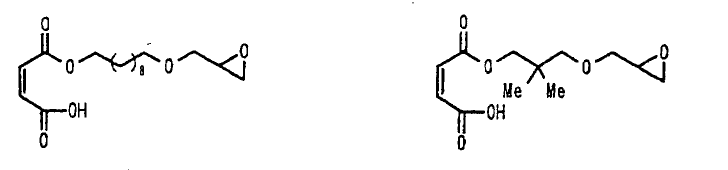

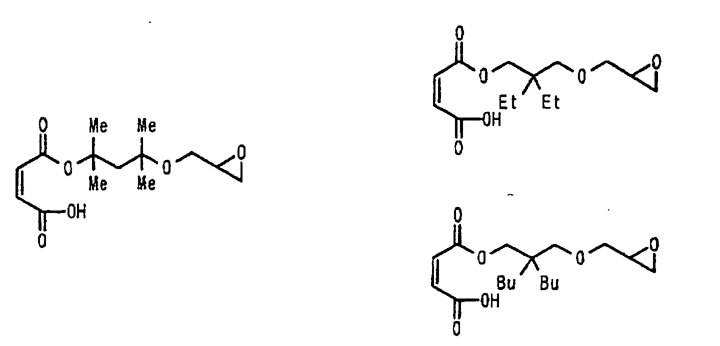

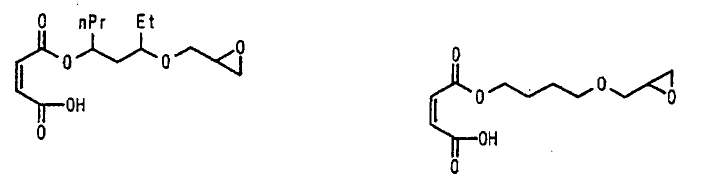

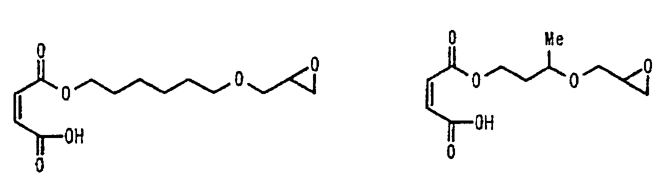

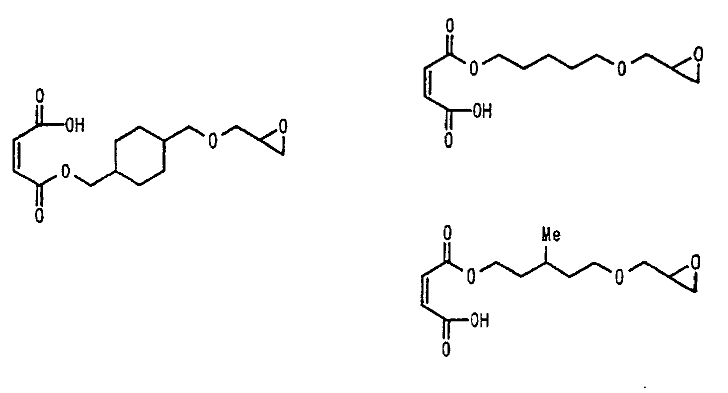

Examples of the polar group-containing unsaturated compounds represented by the formula (I-d) are given below.

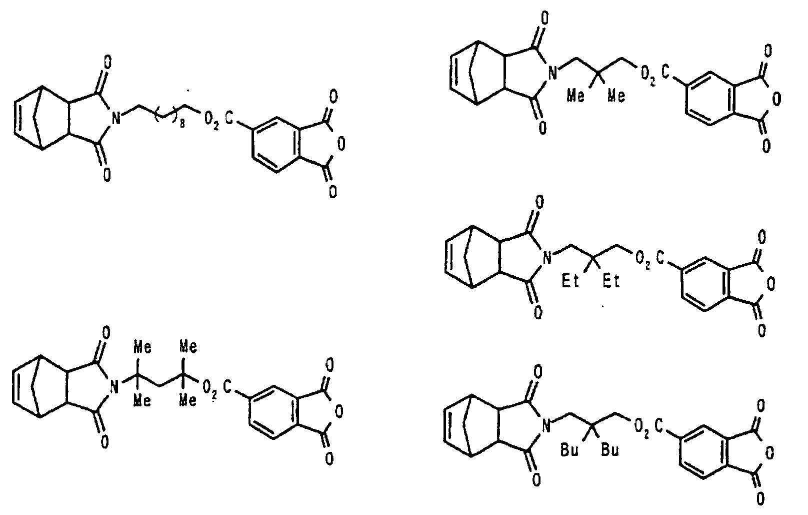

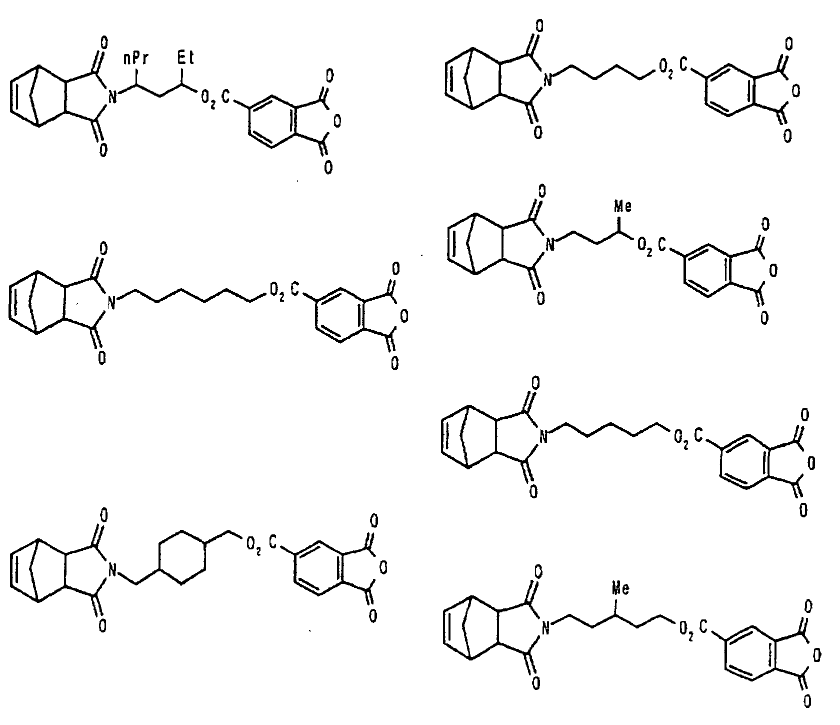

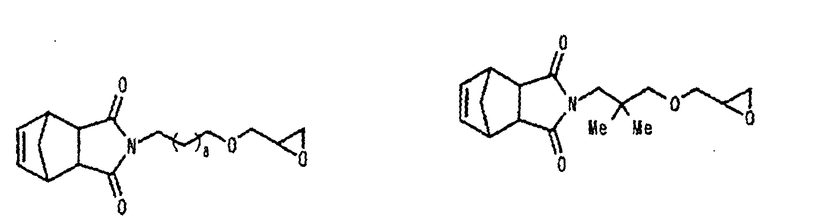

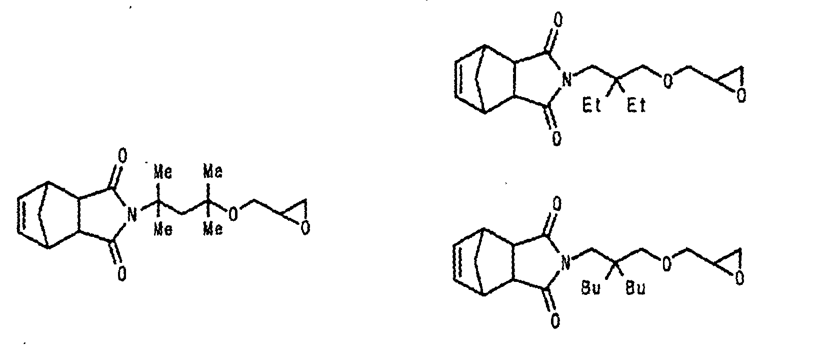

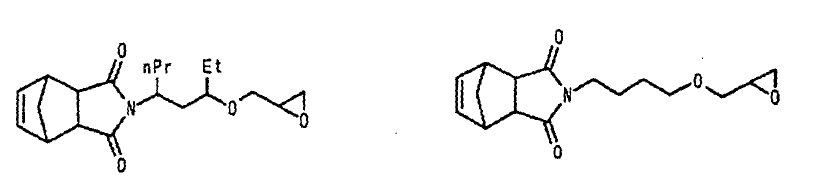

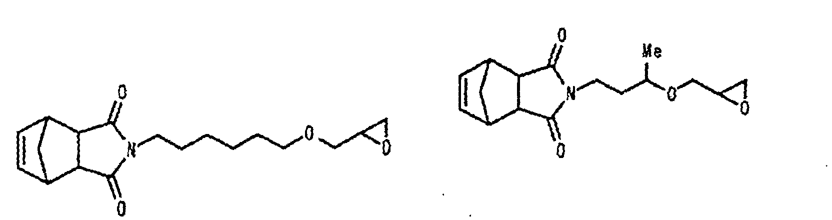

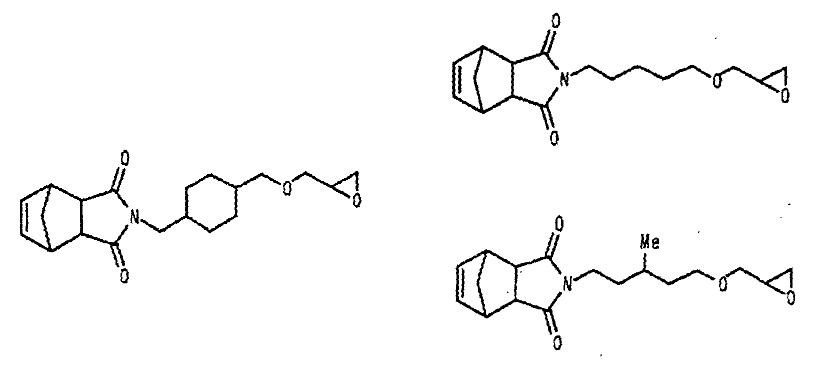

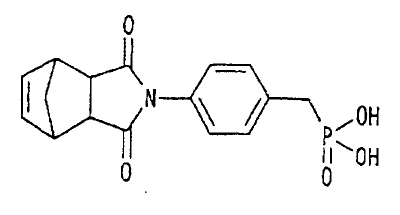

Examples of the polar group-containing unsaturated comp ounds represented by the formula (I-e) are given below.

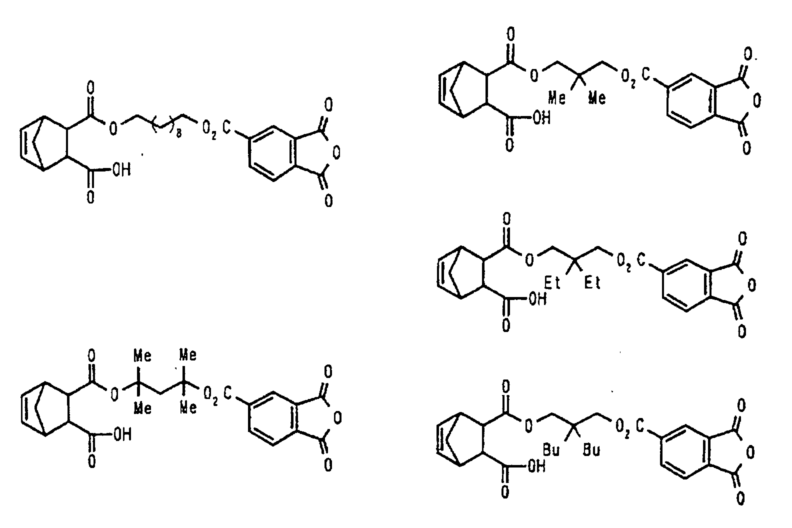

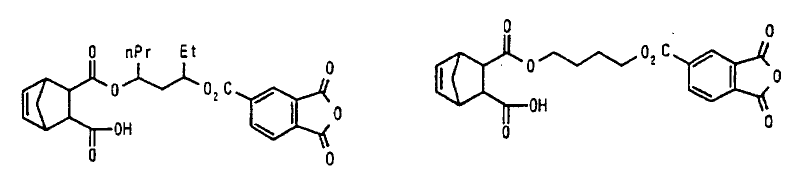

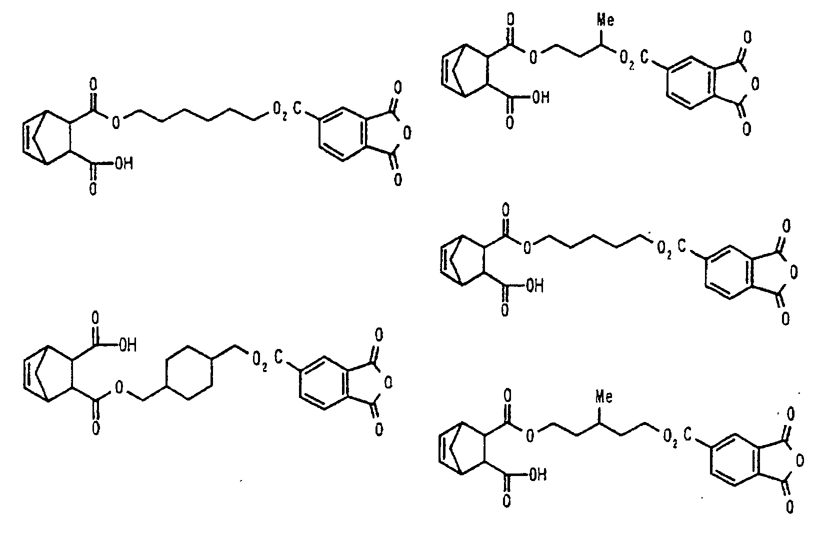

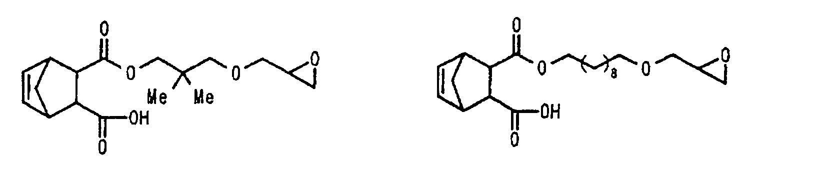

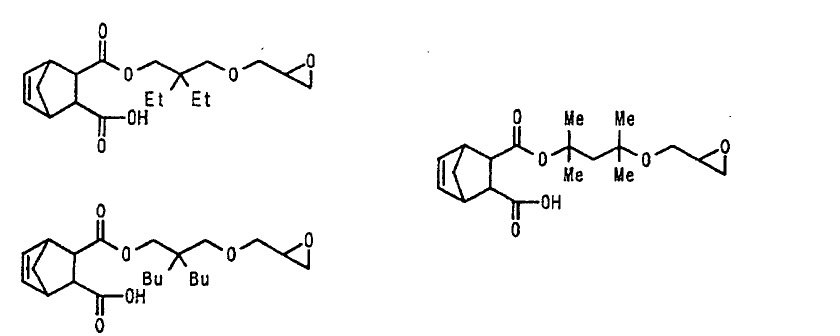

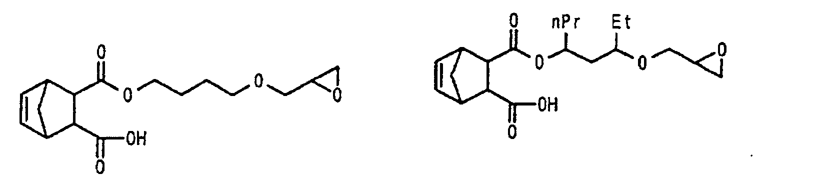

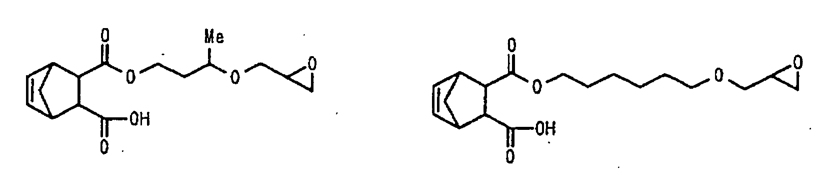

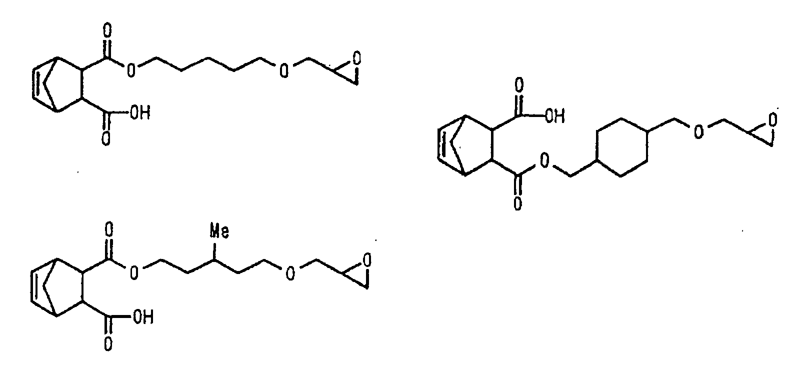

Examples of the polar group-containing unsaturated compounds represented by the formula (I-f) are given below.

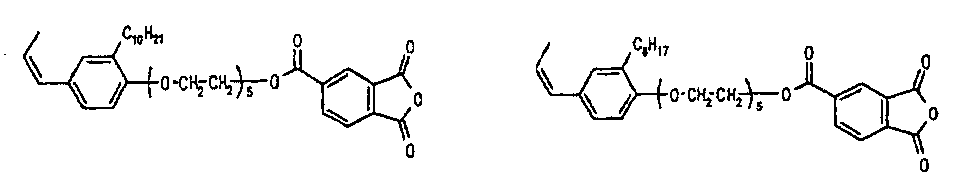

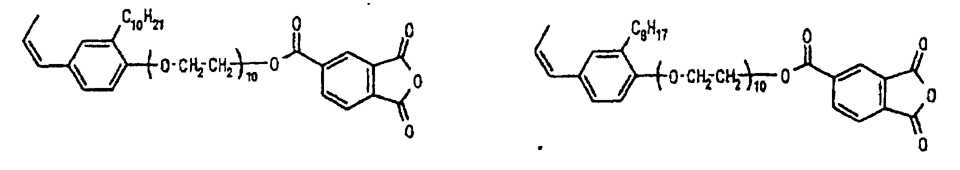

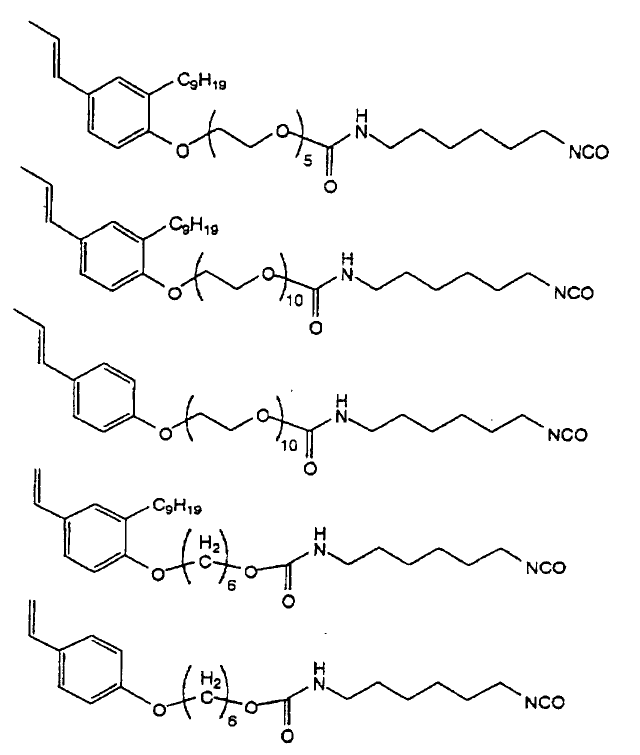

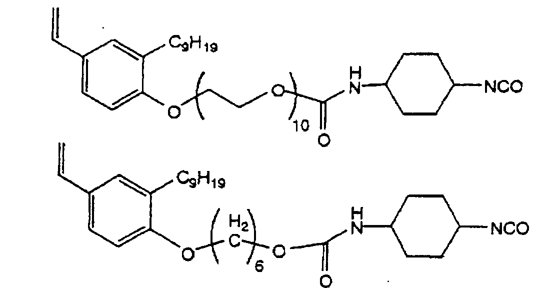

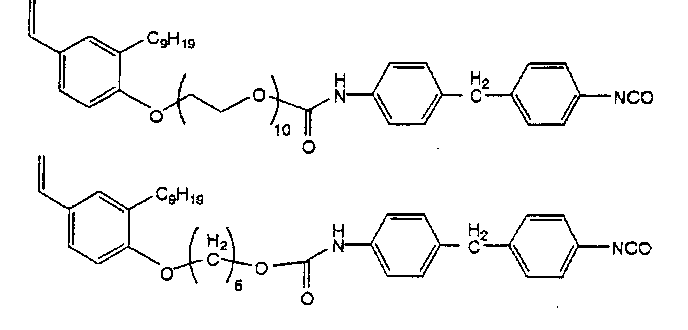

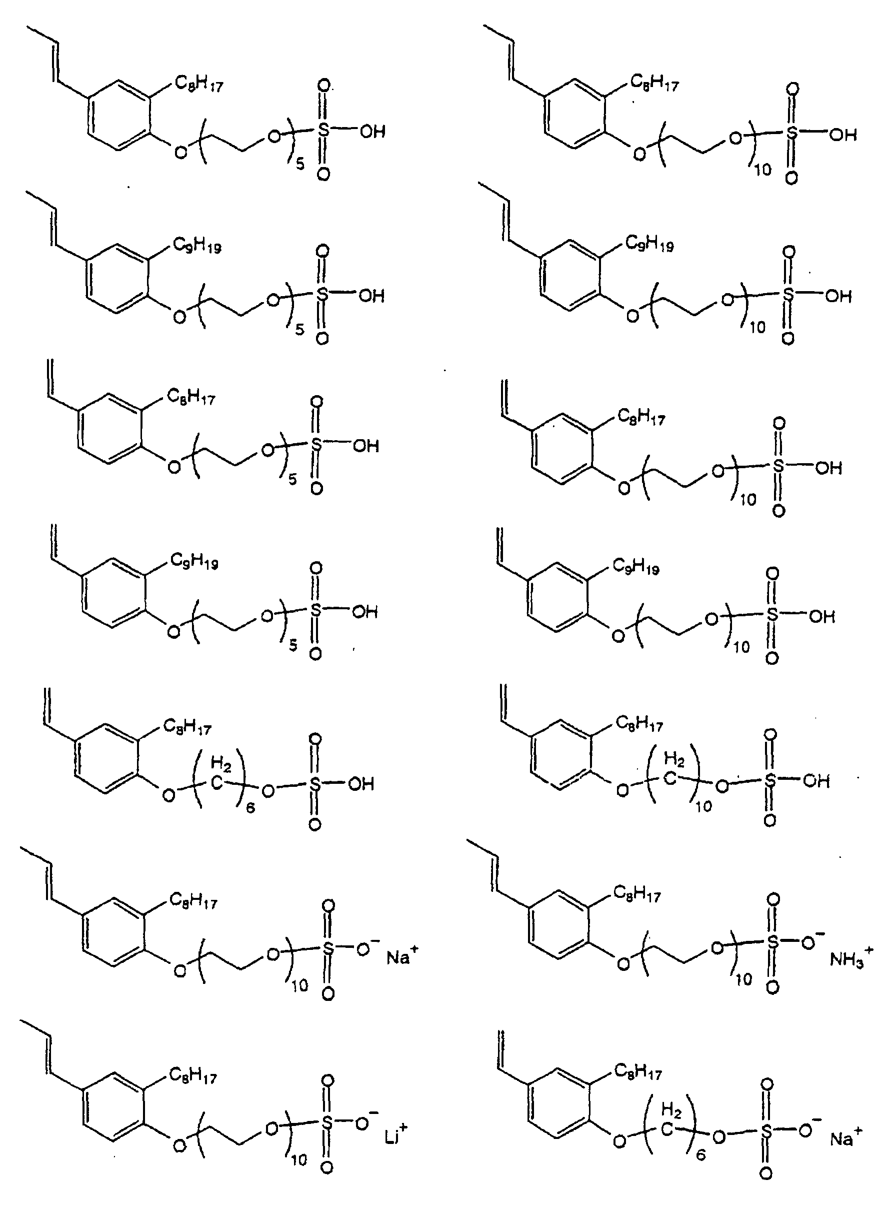

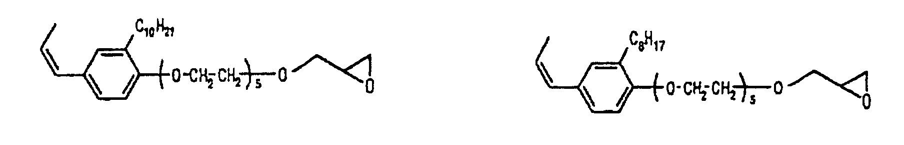

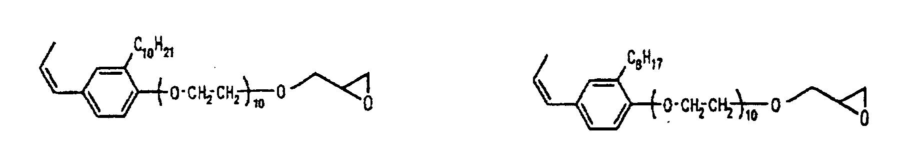

As an embodiment of the polar group-containing unsaturated compound of the invention, there is a compound represented by the following formula (II):

A-X-R²-(Y)ₚ-R¹ (II)

wherein A is a group containing a double bond,
X is -COO-, -OCO-, -NHCO-, -CO-, -O-, -CONR¹⁰-or-NR¹⁰-, R¹⁰ is a hydrogen atom or a hydrocarbon group, and R¹⁰ may be bonded to A or R² to form a cyclic structure (in this case, R¹⁰ may be a single bond),
R² is a divalent group containing a carbon atom,
Y is -COO-, -OCO-, -O-, -CONR¹¹-, -OCONR¹¹-or-NR¹¹CO-, R¹¹ is a hydrogen atom or a hydrocarbon group, and R¹¹ may be bonded to R² or R¹ to form a cyclic structure (in this case, R¹¹ may be a single bond),
p is 0 or 1,
R¹ is any one of an alkyl group of 1 to 20 carbon atoms, a cycloalkyl group and an aryl group each of which contains at least one group selected from the group consisting of a carboxylic acid group, a derivative thereof, an acid anhydride group, an amino group, a cyano group, an isocyanate group, a sulfonic acid group, a metallic salt thereof, a phosphoric acid group and a metallic salt thereof, and
the bond chain linking C=O in the carboxylic acid group, the derivative thereof or the acid anhydride group in R¹ to X, or the bond chain linking the amino group, the cyano group, the isocyanate group, the sulfonic acid group, the metallic salt thereof, the phosphoric acid group or the metallic salt thereof in R¹ to X is constituted of 5 or more atoms.

As another embodiment of the polar group-containing unsaturated compound of the invention, there is a compound of the formula (II) wherein R¹ is any one of an alkyl group of 1 to 20 carbon atoms, a cycloalkyl group and an aryl group each of which contains at least one group selected from the group consisting of a carboxylic acid group, a derivative thereof and an acid anhydride group, and the bond chain linking C=O in the carboxylic acid group, the derivative thereof or the acid anhydride group in R¹ to X is constituted of 5 or more atoms.

As a further embodiment of the polar group-containing unsaturated compound of the invention, there is a compound represented by the following formula (III):

A-X-R²-(Y)ₚ-R¹ (III)

wherein A is a group containing a double bond,
X is -COO-, -OCO-, -NHCO-, -CO-, -O-, -CONR¹⁰-or-NR¹⁰-, R¹⁰ is a hydrogen atom or a hydrocarbon group, and R¹⁰ may be bonded to A or R² to form a cyclic structure (in this case, R¹⁰ may be a single bond),
when X is a group other than -NR¹⁰-, R² is a divalent group containing a carbon atom, and when X is -NR¹⁰-, R² is an alkylene group, a cycloalkylene group, a polyalkyleneoxy group, -R^{a}COOR^{b}- or -R^{a}OCOR^{b}- (R^{a} and R^{b} are each an alkylene group),
Y is-COO-, -OCO-, -O-, -CONR¹¹-, -OCONR¹¹-or-NR¹¹CO-, R¹¹ is a hydrogen atom or a hydrocarbon group, and R¹¹ may be bonded to R² or R¹ to form a cyclic structure (in this case, R¹¹ may be a single bond),
p is 0 or 1, and
R¹ is any one of an alkyl group of 1 to 20 carbon atoms, a cycloalkyl group and an aryl group each of which contains at least one epoxy group, and the bond chain linking epoxy group in R' to X is constituted of 5 or more atoms.

As a still further embodiment of the polar group-containing unsaturated compound of the invention, there is a compound of the above formula (III) wherein, when X is -COO-, -OCO-, -NHCO-, -CO-, -O- or -CONR¹⁰-, R² is an alkylene group of 3 to 20 carbon atoms, an arylene group, a cycloalkylene group, a polyalkyleneoxy group, -R^{a}COOR^{b}- or -R^{a}OCOR^{b}- (R^{a} and R^{b} are each an alkylene group) .

Examples of the polar group-containing unsaturated compounds represented by the formula (I), other than those represented by the formulas (I-a) to (I-f), are given below.

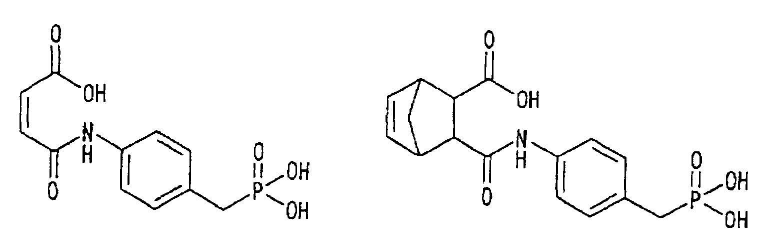

There is no specific limitation on the synthesis of the polar group-containing unsaturated compounds, and they can be synthesized by the processes described below.

The process for synthesizing the polar group-containing unsaturated compound wherein R¹ is at least one polar group selected from the group consisting of a carboxylic acid group, a derivative thereof, an acid anhydride group, an amino group, a cyano group, an isocyanate group, a sulfonic acid group, a metallic salt thereof, a phosphoric acid group and a metallic salt thereof, or any one of an alkyl group of 1 to 20 carbon atoms, a cycloalkyl group of 3 to 20 carbon atoms and an aryl group of 6 to 20 carbon atoms each of which contains at least one group selected from these polar groups is described first.

When X' is -C(=O)O- group, the compound of the formula (I-a) can be prepared by allowing a compound of the following formula (I-a') to react with HO-R²-OH and then with a halide containing a structure of the R¹ group such as an acid halide. 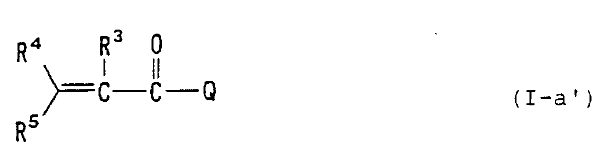 wherein Q is a halogen atom.

For example, an acrylic acid halide (or methacrylic acid halide) is allowed to react with an alkyldiol to synthesize a hydroxyalkyl acrylate, and the hydroxyalkyl acrylate is then allowed to react with, for example, a trimellitic anhydride halide.

The reaction of the acrylic acid halide (or methacrylic acid halide) with the alkyldiol is preferably carried out in the presence of a dehydrohalogenation agent. As the dehydrohalogenation agent, a basic material is used, and examples thereof include inorganic salts such as sodium carbonate and sodium hydrogencarbonate and organic bases such as pyridine and triethylamine. Of these, triethylamine is preferable. It is preferable to conduct the reaction in a solvent. Examples of the solvents employable for the reaction include aromatic hydrocarbons, such as benzene and toluene; aliphatic or alicyclic hydrocarbons, such as n-hexane andcyclohexane; halogenated hydrocarbons, such as chloroform and dichloromethane; and ethers, such as dioxane and tetrahydrofuran. Of these, dichloromethane is particularly preferable.

The reaction temperature is in the range of usually -20 to 50°C, preferably 0 to 25°C, though it depends upon the types of the diol and the catalyst used. It is preferable to use the acrylic acid halide and the diol substantially in equimolar amounts. After the reaction, a salt of the dehydrohalogenation agent and the hydrogen halide is usually precipitated, so that the salt is removed by filtration or the like. Then, the reaction solution is concentrated to remove the solvent, followed by separating and purifying the desired hydroxyalkyl acrylate by a known means such as column chromatography.

Then, the hydroxyalkyl acrylate is allowed to react with trimellitic anhydride chloride. The reaction of the hydroxyalkyl acrylate with the trimellitic anhydride chloride also is preferably carried out in the presence of a dehydrohalogenation agent. As the dehydrohalogenation agent, a basic material is used, and examples thereof include inorganic salts such as sodium carbonate and sodium hydrogencarbonate and organic bases such as pyridine and triethylamine. Of these, pyridine is preferable. This reaction is preferably carried out in a solvent, and examples of the solvents include the same ones as previously described. Of the solvents, dichloromethane is particularly preferably employed.

The reaction temperature is in the range of usually -20 to 50°C, preferably 0 to 25°C, though it depends upon the types of the diol and the catalyst used. The hydroxyalkyl acrylate and the trimellitic anhydride chloride are used substantially in equimolar amounts. After the reaction, a salt of the dehydrohalogenation agent and the hydrogen halide is usually deposited, so that the salt is removed by filtration or the like. Then, the reaction solution is concentrated to remove the solvent, followed by separating and purifying the desired acryloxyalkyltrimellitic anhydride by a known means such as column chromatography.

The compound of the formula (I-a) (X' is -COO- group) can be prepared by allowing a compound of the above formula (I-a') wherein Q is an OH group to react with HO-R²-OH and then with a halide containing a structure of the R¹ group such as an acid halide.

For example, acrylic acid is allowed to react with an alkyldiol to synthesize a hydroxyalkyl acrylate, and the hydroxyalkyl acrylate is then allowed to react with, for example, a trimellitic anhydride halide. An example of the reaction formula is given below.

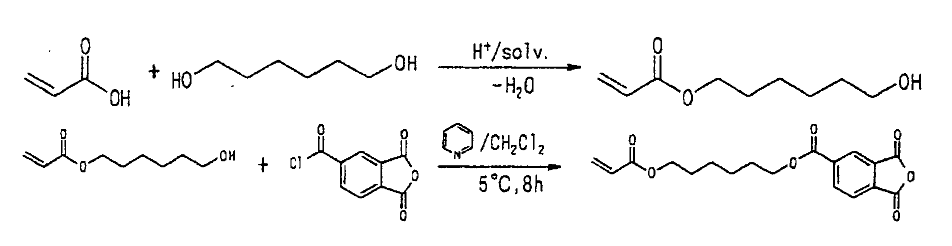

The hydroxyalkyl acrylate can be synthesized by dehydration reaction of acrylic acid with an alkyldiol in the presence of an acid catalyst. Examples of the acid catalysts employable for the reaction include inorganic acids such as sulfuric acid and phosphoric acid, organic acids such as acetic acid and p-toluenesulfonic acid, and solid acids such as activated clay. Of these, p-toluenesulfonic acid is particularly preferable. The reaction is preferably carried out in a solvent, and examples of the solvents employable include the same ones as previously described. Of those, toluene is particularly preferable.

The reaction temperature is in the range of usually 50 to 180°C, preferably 110 to 140°C, though it depends upon the types of the diol, the catalyst and the solvent used. The acrylic acid and the diol are used substantially in equimolar amounts. With a progress of the reaction, water is produced, so that the water is removed from the reaction system using a Dean and Stark tube or the like. Thereafter, the acid catalyst is removed by a method of using an adsorbent such as calcium oxide or magnesium oxide or a method of filtration or washing with water, and then the solvent is distilled off, followed by separating and purifying the desired hydroxyalkyl acrylate by a known means such as column chromatography.

The reaction of the hydroxyalkyl acrylate with the trimellitic anhydride chloride can be carried out in accordance with the aforesaid synthesis process.

In case of the compound of the formula (I-a) wherein X' is -OCO- group, an allyl ester can be prepared by such a reaction of an allyl halide with a long-chain dicarboxylic acid as described below.

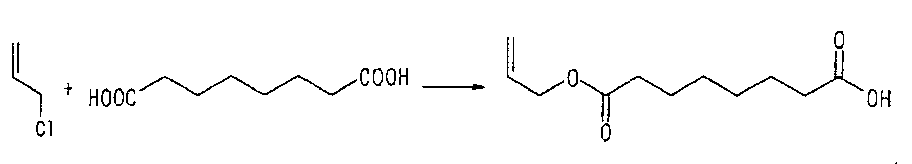

The reaction of the allyl halide with the long-chain dicarboxylic acid is carried out in the presence of a dehydrohalogenation agent. As the dehydrohalogenation agent, a basic material is used, and examples thereof include inorganic salts such as sodium carbonate and sodium hydrogencarbonate and organic bases such as pyridine and triethylamine. Of these, triethylamine is preferable. This reaction is preferably carried out in a solvent, and examples of the solvents employable for the reaction include the same ones as previously described. Of the solvents, dichloromethane is particularly preferable.

The reaction temperature is in the range of usually -20 to 50°C, preferably -5 to 25°C, though it depends upon the types of the starting materials and the catalyst used. The allyl halide and the dicarboxylic acid are used substantially in equimolar amounts. After the reaction, a salt of the dehydrohalogenation agent and the hydrogen halide is usually deposited, so that the salt is removed by filtration or the like. Then, the reaction solution is concentrated to remove the solvent, followed by separating and purifying the desired allyl ester by a known means such as column chromatography.

It is also possible to synthesize the allyl ester from an alcohol having a terminal vinyl group and a dicarboxylic acid through the following reaction.

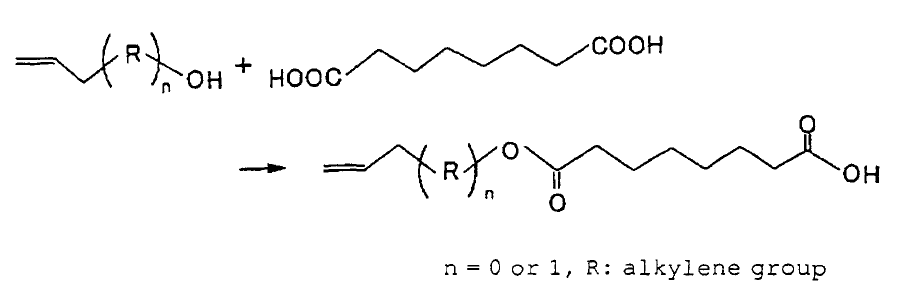

This synthesis reaction can be carried out by dehydration reaction of an alcohol having a terminal vinyl group with a dicarboxylic acid in the presence of an acid catalyst. Examples of the acid catalysts employable for the reaction include inorganic acids such as sulfuric acid and phosphoric acid, organic acids such as acetic acid and p-toluenesulfonic acid, and solid acids such as activated clay. Of these, p-toluenesulfonic acid is particularly preferable. This reaction is preferably carried out in a solvent, and examples of the solvents employable for the reaction include the same ones as previously described. Of those, toluene is particularly preferable.

The reaction temperature is in the range of usually 50 to 180°C, preferably 110 to 140°C, though it depends upon the types of the starting materials, the catalyst and the solvent used. The alcohol having a terminal vinyl group and the dicarboxylic acid are used substantially in equimolar amounts.

With a progress of the reaction, water is produced, so that the water is removed from the reaction system using a Dean and Stark tube or the like. Thereafter, the acid catalyst is removed by a method of using an adsorbent such as calcium oxide or magnesium oxide or a method of filtration or washing with water and so on, and then the solvent is distilled off, followed by separating and purifying the desired product by a known means such as column chromatography.

In case of the compound of the formula (I-a) wherein R² is a polyethyleneoxy group, commercially available acryloxypolyethylene glycol can be used as a starting materiel.

The compound of the formula (I-a) wherein X' is -COO- group or -CONH- group and R¹ contains an amino group can be synthesized by allowing a compound of the formula (I-a') wherein Q is OH group or a halogen atom to react with H₂N-R-NH₂ or HO-R-NH₂ (R is a group containing a part of R¹, a structure of R² or a structure of Y).

For example, an acrylic acid halide (or methacrylic acid halide) is allowed to react with an alkyldiamine to synthesize an aminoalkylacrylamide. An example of the reaction formula is given below.

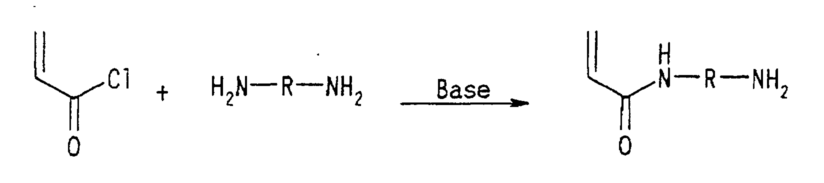

This reaction is carried out in the presence of a dehydrohalogenation agent. As the dehydrohalogenation agent, a basic material is used, and examples thereof include inorganic salts such as sodium carbonate and sodium hydrogencarbonate and organic bases such as pyridine and triethylamine. Of these, triethylamine is preferable.

This reaction is preferably carried out in a solvent, and examples of the solvents include the same ones as previously described. Of the solvents, dichloromethane is particularly preferable.

The reaction temperature is in the range of usually -20 to 50°C, preferably 0 to 25°C, though it depends upon the types of the diamine and the catalyst used. The acrylic acid halide and the diamine are used substantially in equimolar amounts. After the reaction is completed, a salt of the dehydrohalogenation agent and the hydrogen halide is usually deposited, so that the salt is removed by filtration or the like. Then, the reaction solution is concentrated to remove the solvent, followed by separating and purifying the desired aminoalkylacrylamide by a known means such as column chromatography.

The synthesis of the aminoalkylacrylamide can also be carried out in the presence of an acid catalyst. An example of the reaction formula is given below.

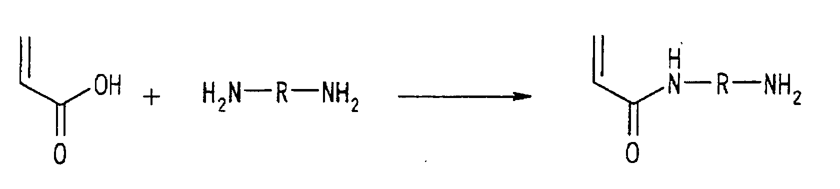

Examples of the acid catalysts employable for the reaction include inorganic acids such as sulfuric acid and phosphoric acid, organic acids such as acetic acid and p-toluenesulfonic acid, and solid acids such as activated clay. Of these, p-toluenesulfonic acid is particularly preferable.

This reaction is preferably carried out in a solvent, and examples of the solvents employable for the reaction include the same ones as previously described. Of the solvents, toluene is particularly preferable.

The reaction temperature is in the range of usually 50 to 180°C, preferably 110 to 140°C, though it depends upon the types of the diamine, the catalyst and the solvent used. The acrylic acid and the diamine are used substantially in equimolar amounts. With a progress of the reaction, water is produced, so that the water is removed from the reaction system using a Dean and Stark water separation tube or the like. After the reaction, the acid catalyst is removed by a method of using an adsorbent such as calcium oxide or magnesium oxide or a method of filtration or washing with water and so on, and then the solvent is distilled off, followed by separating and purifying the desired aminoalkylacrylamide by a known means such as column chromatography.

The compound of the formula (1-a) wherein R¹ contains an amino group can also be synthesized by, for example, a reaction of a hydroxyalkyl acrylate with an aminoalkylcarboxylic acid, said reaction being represented by the following reaction formula. 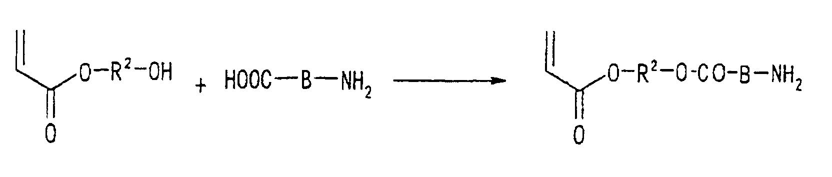 wherein B is a part of a structure of R¹.

An example of the above reaction is given below.

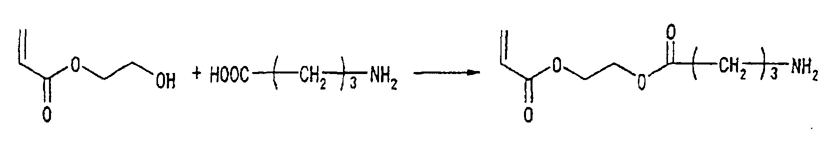

This reaction can be carried out in the presence of an acid catalyst. Examples of the acid catalysts employable for the reaction include inorganic acids such as sulfuric acid and phosphoric acid, organic acids such as acetic acid and p-toluenesulfonic acid, and solid acids such as activated clay. Of these, p-toluenesulfonic acid is particularly preferable.

This reaction is preferably carried out in a solvent, and examples of the solvents employable for the reaction include the same ones as previously described. Of the solvents, toluene is particularly preferable.

The reaction temperature is in the range of usually 50 to 180°C, preferably 110 to 140°C, though it depends upon the types of the aminoalkylcarboxylic acid, the catalyst and the solvent used. The hydroxyalkyl acrylate and the aminoalkylcarboxylic acid are used substantially in equimolar amounts. With a progress of the reaction, water is produced, so that the water is removed from the reaction system using a Dean and Stark water separation tube or the like. After the reaction, the acid catalyst is removed by a method of using an adsorbent such as calcium oxide or magnesium oxide or a method of filtration or washing with water and so on, and then the solvent is distilled off, followed by separating and purifying the desired terminal amino group-containing acrylic acid by a known means such as column chromatography.

The compound of the formula (I-a) wherein X' is -COO- group or -CONH- group and R¹ contains a cyano group can be synthesized by, for example, a reaction represented by the following reaction formula.

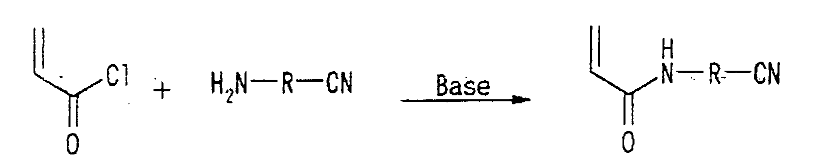

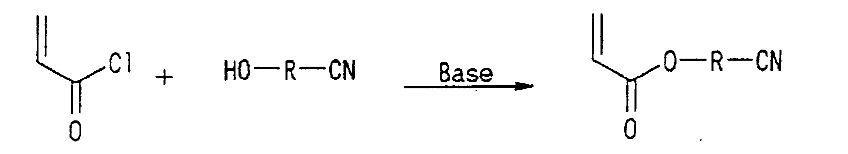

The reaction of an acrylic acid halide (or methacrylic acid halide) with a cyanoalkylamine (or cyanoalkyl alcohol) is carried out in the presence of a dehydrohalogenation agent. As the dehydrohalogenation agent, a basic material is used, and examples thereof include inorganic salts such as sodium carbonate and sodium hydrogencarbonate and organic bases such as pyridine and triethylamine. Of these, triethylamine is particularly preferable.

This reaction is preferably carried out in a solvent, and examples of the solvents include the same ones as previously described. Of the solvents, dichloromethane is particularly preferable.

The reaction temperature is in the range of usually -20 to 50°C, preferably 0 to 25°C, though it depends upon the types of the cyanoalkylamine (or cyanoalkyl alcohol) and the catalyst used. The acrylic acid halide and the cyanoalkylamine (or cyanoalkyl alcohol) are used substantially in equimolar amounts. After the reaction is completed, a salt of the dehydrohalogenation agent and the hydrogen halide is usually deposited, so that the salt is removed by filtration or the like. Then, the reaction solution is concentrated to remove the solvent, followed by separating and purifying the desired cyanoalkylacrylamide (or cyanoalkyl acrylate) by a known means such as column chromatography.

The terminal cyano group-containing acrylate can also be synthesized through the following two steps.

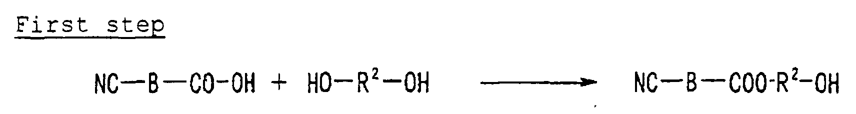

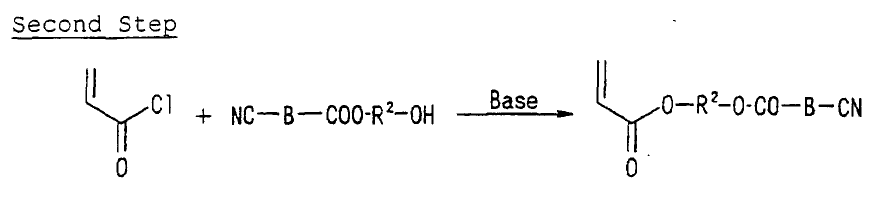

In the reaction of the first step, a hydroxyalkylcyanoacetic ester can be synthesized by, for example, a reaction of cyanoacetic acid with an alkyldiol in the presence of an acid catalyst, said reaction being represented by the following reaction formula.

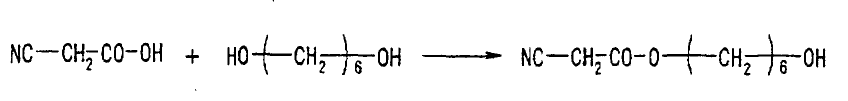

Examples of the acid catalysts employable for the reaction include inorganic acids such as sulfuric acid and phosphoric acid, organic acids such as acetic acid and p-toluenesulfonic acid, and solid acids such as activated clay. Of these, p-toluenesulfonic acid is particularly preferable.

This reaction is preferably carried out in a solvent, and examples of the solvents employable for the reaction include the same ones as previously described. Of the solvents, toluene is particularly preferable.

The reaction temperature is in the range of usually 50 to 180°C, preferably 110 to 140°C, though it depends upon the types of the diol, the catalyst and the solvent used. The cyanoacetic acid and the alkyldiol are used substantially in equimolar amounts. With a progress of the reaction, water is produced, so that the water is removed from the reaction system using a Dean and Stark water separation tube or the like. After the reaction, the acid catalyst is removed by a method of using an adsorbent such as calcium oxide or magnesium oxide or a method of filtration or washing with water and so on, and then the solvent is distilled off, followed by separating and purifying the desired hydroxyalkylcyanoacetic ester by a known means such as column chromatography.

As the reaction of the second step, for example, a reaction of an acrylic acid halide (or methacrylic acid halide) with the hydroxyalkylcyanoacetic ester is carried out in the presence of a dehydrohalogenation agent, said reaction being represented by the following reaction formula.

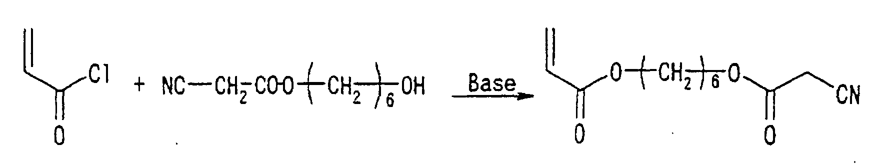

As the dehydrohalogenation agent, a basic material is used, and examples thereof include inorganic salts such as sodium carbonate and sodium hydrogencarbonate and organic bases such as pyridine and triethylamine. Of these, triethylamine is particularly preferable.

This reaction is preferably carried out in a solvent, and examples of the solvents include the same ones as previously described. Of the solvents, dichloromethane is particularly preferable.

The reaction temperature is in the range of usually -20 to 50°C, preferably 0 to 25°C, though it depends upon the types of the ester and the catalyst used. The acrylic acid halide and the hydroxyalkylcyanoacetic ester are used substantially in equimolar amounts. After the reaction is completed, a salt of the dehydrohalogenation agent and the hydrogen halide is usually deposited, so that the salt is removed by filtration or the like. Then, the reaction solution is concentrated to remove the solvent, followed by separating and purifying the desired terminal cyano group-containing acrylate by a known means such as column chromatography.

The compound of the formula (I-a) wherein X' is -COO- group and R¹ contains an isocyanate group can be synthesized by, for example, a reaction of a hydroxylalkyl acrylate with an alkylene diisocyanate, said reaction being represented by the following reaction formula.

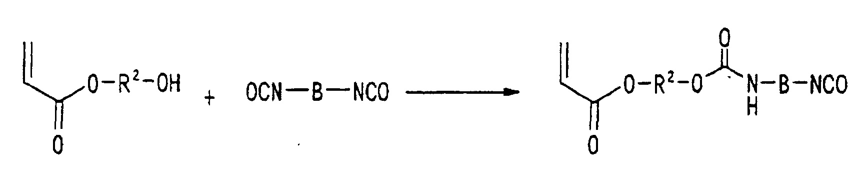

An example of the above reaction is a reaction of hydroxyethyl acrylate with hexamethylene diisocyanate, said reaction being represented by the following reaction formula.

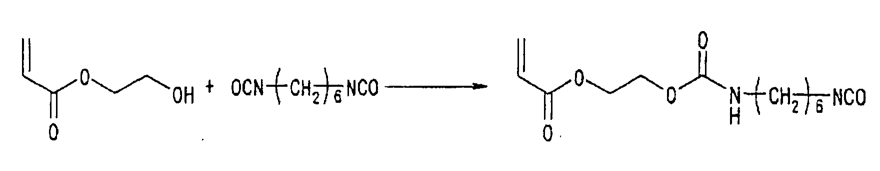

This reaction is preferably carried out in a solvent, and examples of the solvents employable for the reaction include the same ones as previously described. Of the solvents, toluene is particularly preferable.

The reaction temperature is in the range of usually 0 to 80°C, preferably 10 to 40°C, though it depends upon the types of the catalyst and the solvent used. The hydroxyalkyl acrylate and the alkylene diisocyanate are used substantially in equimolar amounts. After the reaction, the solvent is removed, followed by separating and purifying the desired terminal isocyanate group-containing acrylic acid by a known means such as column chromatography.

The compound of the formula (I-a) wherein X' is -COO- group or -CONH- group and R¹ contains a sulfonic acid group, a phosphoric acid group or the like can be synthesized by allowing a compound of the formula (I-a') wherein Q is a halogen atom to react with H₂N-R-L or HO-R-L (R is a group containing a part of the aforesaid R¹, a structure of R² or a structure of Y, and L is a sulfonic acid group or a phosphoric acid group such as -SO₃H or -P(=O)(OH)₂).

For example, an acrylic acid halide is allowed to react with a sulfonic acid group-containing alkylamine to synthesize the above compound, and an example of the reaction formula is given below.

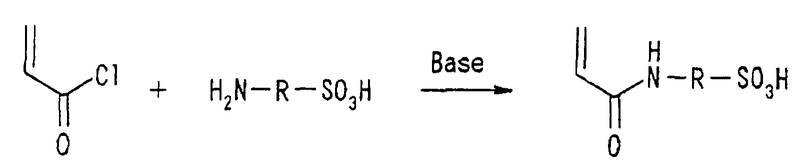

This reaction is carried out in the presence of a dehydrohalogenation agent. As the dehydrohalogenation agent, a basic material is used, and examples thereof include inorganic salts such as sodium carbonate and sodium hydrogencarbonate and organic bases such as pyridine and triethylamine. Of these, triethylamine is particularly preferable.

This reaction is preferably carried out in a solvent, and examples of the solvents employable for the reaction include the same ones as previously described. Of the solvents, dichloromethane is particularly preferable.

The reaction temperature is in the range of usually -20 to 50°C, preferably 0 to 25°C, though it depends upon the types of the reagent and the catalyst used. The acrylic acid halide and the sulfonic acid group-containing alkylamine are used substantially in equimolar amounts. After the reaction is completed, a salt of the dehydrohalogenation agent and the hydrogen halide is usually deposited, so that the salt is removed by filtration or the like. Then, the reaction solution is concentrated to remove the solvent, followed by separating and purifying the desired terminal sulfonic acid group-containing acrylamide by a known means such as column chromatography.

The compound of the formula (I-b) can be prepared by allowing a compound of the following formula (I-b') to react with NH₂-R²-OH and then with a halide containing a structure of the R¹ group such as an acid halide.

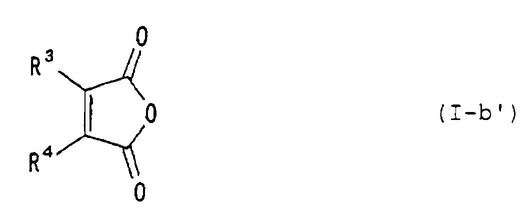

For example, a hydroxyalkyl maleimide is synthesized from maleic anhydride and a hydroxyalkylamine, and the hydroxyalkyl maleimide is allowed to react with, for example, atrimelliticanhydridehalide. Specifically, the compound of the formula (I-b) can be prepared in accordance with the process for synthesizing the compound of the formula (I-a).

The compound of the formula (I-c) can be prepared by allowing a compound of the above formula (I-b') to react with OH-R²-OH and then with a halide containing a structure of the R¹ group such as an acid halide. For example, maleic anhydride is allowed to react with an alkyldiol to synthesize a hydroxyalkyl maleate, and the hydroxyalkyl maleate is then allowed to react with, for example, a trimellitic anhydride halide. Specifically, the compound of the formula (I-c) can be prepared in accordance with the process for synthesizing the compound of the formula (I-a).

The compound of the formula (I-d) can be prepared by allowing a compound of the following formula (I-d') to react with NH₂-R²-OH and then with a halide containing a structure of the R¹ group such as an acid halide.

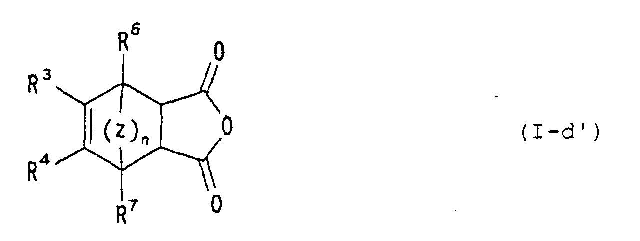

For example, an imide compound is synthesized from endomethylenetetrahydrophthalic anhydride and a hydroxyalkylamine, and the imide compound is then allowed to react with, for example, a trimellitic anhydride halide. Specifically, the compound of the formula (I-d) can be prepared in accordance with the process for synthesizing the compound of the formula (I-a).

The compound of the formula (I-e) can be prepared by allowing a compound of the above formula (I-d') to react with OH-R²-OH and then with a halide containing a structure of the R¹ group such as an acid halide. For example, endomethylenetetrahydrophthalic anhydride is allowed to react with an alkyldiol to synthesize a hydroxyalkyl endomethylenetetrahydrophthalate, and the hydroxyalkyl endomethylenetetrahydrophthalate is allowed to react with, for example, a trimellitic anhydride halide. Specifically, the compound of the formula (I-e) can be prepared in accordance with the process for synthesizing the compound of the formula (I-a).

The compound of the formula (I-f) can be prepared by allowing a compound of the following formula (I-f') to react with OH-R²-OH and then with a halide containing a structure of the R¹ group such as an acid halide. 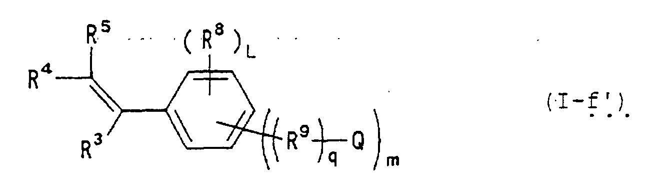 wherein Q is a halogen atom.

For example, 4-(chloromethyl)styrene is allowed to react with an alkyldiol to synthesize 4-(hydroxyalkoxymethyl)styrene, and the 4-(hydroxyalkoxymethyl)styrene is then allowed to react with, for example, a trimellitic anhydride halide. Specifically, the compound of the formula (I-f) can be prepared in accordance with the process for synthesizing the compound of the formula (I-a). This reaction route is represented by the following formula.

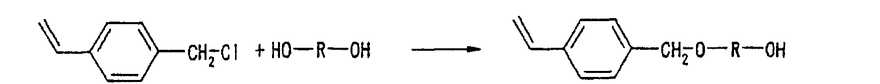

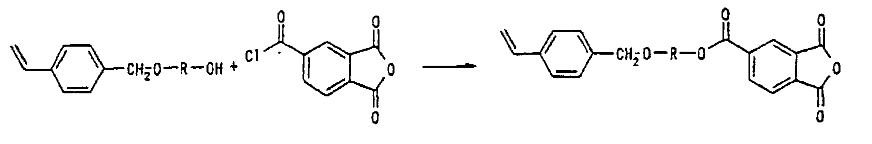

The compound of the formula (I-f) can be prepared by adding an alkylene oxide to a compound of the following formula (I-f") through addition reaction and then allowing the resulting compound to react with a halide containing a structure of the R¹ group such as an acid halide.

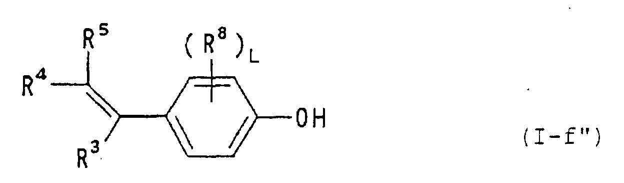

For example, an alkylene oxide is added to alkylated propenylphenol in a conventional manner, and then the resulting compound is allowed to react with, for example, atrimellitic anhydride halide. Specifically, the compound of the formula (I-f) can be prepared in accordance with the process for synthesizing the compound of the formula (I-a). This reaction route is represented by the following reaction formula.

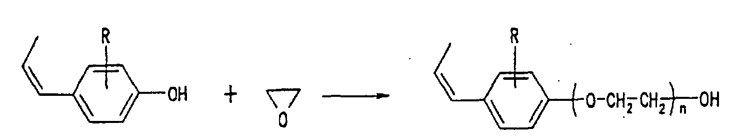

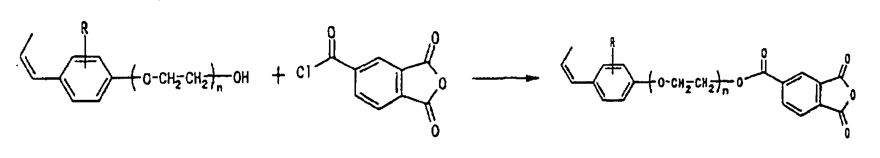

The reaction route to prepare the compound of the formula (I-f) containing an isocyanate group in R¹ by the similar process and the reaction route to prepare the compound of the formula (I-f) containing an sulfonic acid group in R¹ by the similar process are represented by, for example, the following formulas, respectively.

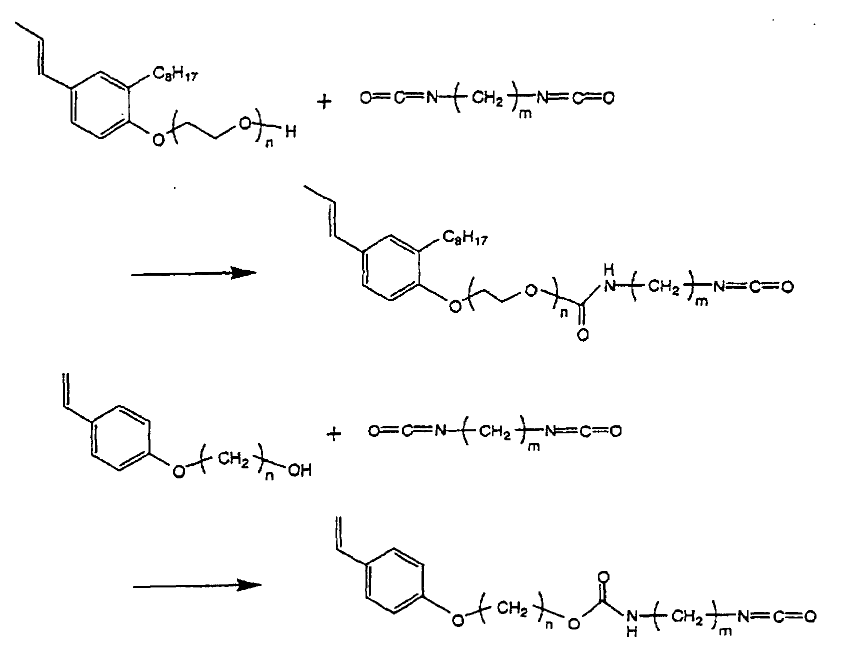

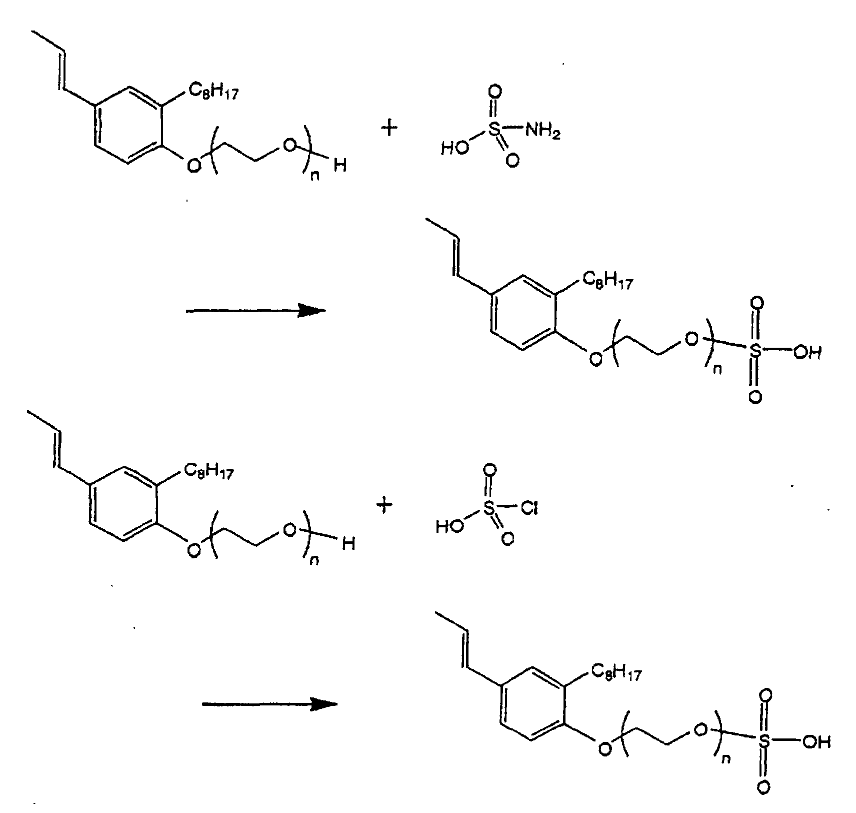

Next, synthesis of the polar group-containing unsaturated compound wherein R¹ is an epoxy group or any one of an alkyl group of 1 to 20 carbon atoms, a cycloalkyl group of 3 to 20 carbon atoms and an aryl group of 6 to 20 carbon atoms each of which contains an epoxy group is described.

When X' is -C(=O)O- group, the compound of the formula (I-a) can be prepared by allowing a compound of the following formula (I-a') to react with HO-R²-OH and then with a halide containing a structure of the R¹ group. 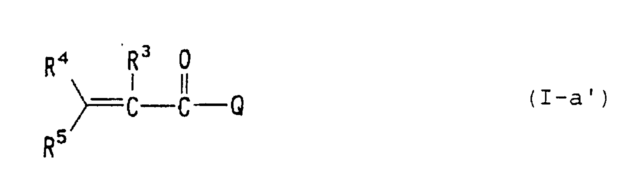 wherein Q is a halogen atom.

For example, an acrylic acid halide (or methacrylic acid halide) is allowed to react with an alkyldiol to synthesize a hydroxyalkyl acrylate, and the hydroxyalkyl acrylate is then allowed to react with, for example, epichlorohydrin, followed by intramolecular epoxidation reaction.

The reaction of the acrylic acid halide (or methacrylic acid halide) with the alkyldiol is preferably carried out in the presence of a dehydrohalogenation agent. As the dehydrohalogenation agent, a basic material is used, and examples thereof include inorganic salts such as sodium carbonate and sodium hydrogencarbonate and organic bases such as pyridine and triethylamine. Of these, triethylamine is preferable. This reaction is preferably carried out in a solvent, and examples of the solvents employable for the reaction include the same ones as previously described. Of these, dichloromethane is particularly suitable.

The reaction temperature is in the range of usually -20 to 50°C, preferably 0 to 25°C, though it depends upon the types of the diol and the catalyst used. It is preferable to use the acrylic acid halide and the diol substantially in equimolar amounts. After the reaction, a salt of the dehydrohalogenation agent and the hydrogen halide is usually deposited, so that the salt is removed by filtration or the like. Then, the reaction solution is concentrated to remove the solvent, followed by separating and purifying the desired hydroxyalkyl acrylate by a known means such as column chromatography.

The reaction of the hydroxyalkyl acrylate with the epichlorohydrin also is preferably carried out in the presence of a dehydrohalogenation agent. As the dehydrohalogenation agent, a basic material is used, and examples thereof include inorganic salts such as sodium carbonate and sodium hydrogencarbonate and organic bases such as pyridine and triethylamine. Of these, pyridine is particularly preferable. This reaction is preferably carried out in a solvent, and examples of the sol vents include the same ones as previously described. Of the solvents, dichloromethane is particularly preferably employed.

The reaction temperature is in the range of usually -20 to 50°C, preferably 0 to 25°C, though it depends upon the types of the diol and the catalyst used. It is preferable to use the hydroxyalkyl acrylate and the epichlorohydrin substantially in equimolar amounts. After the reaction, a salt of the dehydrohalogenation agent and the hydrogen halide is usually deposited, so that the salt is removed by filtration or the like. Then, the reaction solution is concentrated to remove the solvent, followed by separating and purifying the desired glycidyl alkyl acrylate by a known means such as column chromatography.

The glycidyl alkyl acrylate can also be synthesized by performing a reaction of a hydroxyalkyl acrylate with epichlorohydrin in the presence of an acid catalyst, followed by intramolecular epoxidation reaction using a dehydrohalogenation agent. An example of the reaction formula is given below.

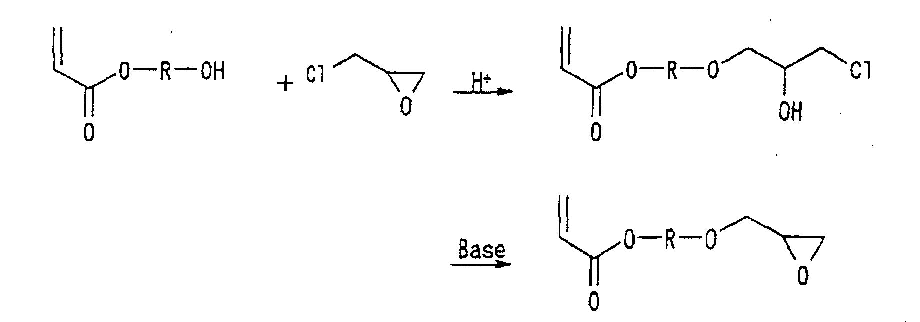

The reaction of the hydroxyalkyl acrylate with the epichlorohydrin can be carried out in the presence of an acid catalyst. Examples of the acid catalysts employable for the reaction include inorganic acids such as sulfuric acid and phosphoric acid, organic acids such as acetic acid and p-toluenesulfonic acid, and solid acids such as activated clay. Of these, p-toluenesulfonic acid is particularly preferable. This reaction is preferably carried out in a solvent, and examples of the solvents employable for the reaction include the same ones as previously described. Of those, toluene is particularly preferable.

The reaction temperature is in the range of usually 50 to 180°C, preferably 110 to 140°C, though it depends upon the types of the starting materials, the catalyst and the solvent used. After the reaction, the acid catalyst is removed by a method of using an adsorbent such as calcium oxide or magnesium oxide or a method of filtration or washing with water and so on, and then the solvent is distilled off, followed by separating and purifying the desired alkyl acrylate by a known means such as column chromatography.

Then, dehydrohalogenation reaction is carried out in the presence of a dehydorhalogenation agent. As the dehydrohalogenation agent, a basic material is used, and examples thereof include inorganic salts such as sodium carbonate and sodium hydrogencarbonate and organic bases such as pyridine and triethylamine. Of these, triethylamine is preferable. This reaction is preferably carried out in a solvent, and examples of the solvents include the same ones as previously described. Of the solvents, toluene is particularly preferable.

The reaction temperature is in the range of usually 50 to 180°C, preferably 110 to 140°C, though it depends upon the types of the solvent and the catalyst used. After the reaction, a salt of the dehydrohalogenation agent and the hydrogen halide is usually deposited, so that the salt is removed by filtration or the like. Then, the reaction solution is concentrated to remove the solvent; followed by separating and purifying the desired glycidyl alkyl acrylate by a known means such as column chromatography.

An example of the reaction is represented by the following reaction formula.

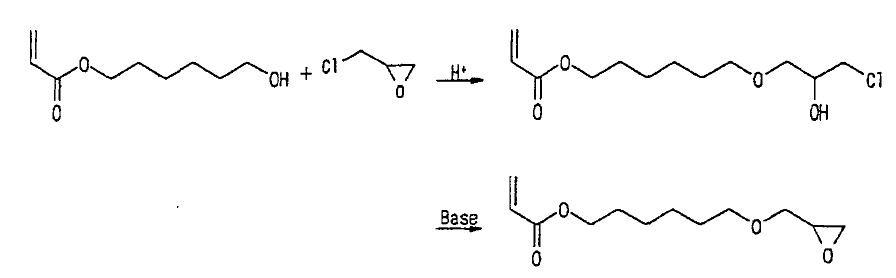

The glycidyl alkyl acrylate can also be synthesized by performing reaction of a carboxyalkyl acrylate with epichlorohydrin and then performing intramolecular epoxidation reaction using a dehydrohalogenation agent. An example of the reaction formula is given below.

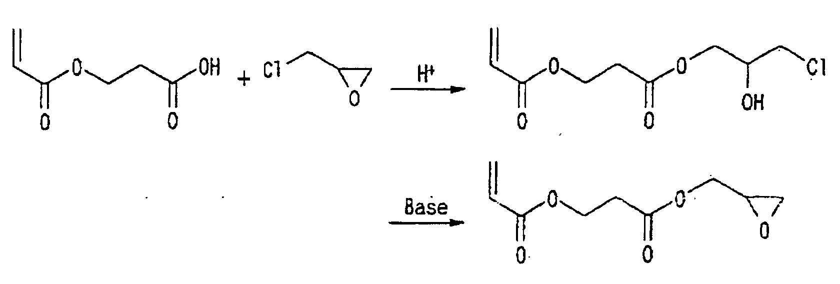

Synthesis of the glycidyl alkyl acrylate from the carboxyalkyl acrylate and the epichlorohydrin can be carried out in accordance with the aforesaid synthesis process.

The compound of the formula (I-b) can be prepared by allowing a compound of the following formula (I-b') to react with NH₂-R²-OH and then with a halide containing a structure of the R¹ group, followed by intramolecular epoxidation.

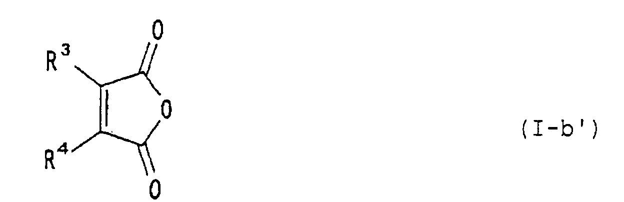

For example, a hydroxyalkyl maleimide is synthesized from maleic anhydride and a hydroxyalkylamine, and the hydroxyalkyl maleimide is allowed to react with, for example, epichlorohydrin, followed by intramolecular epoxidation. Specifically, the compound of the formula (I-b) can be prepared in accordance with the process for synthesizing the compound of the formula (I-a).

The compound of the formula (I-c) can be prepared by allowing a compound of the above formula (I-b') to react with OH-R²-OH and then with a halide containing the R¹ group, followed by intramolecular epoxidation. For example, maleic anhydride is allowed to react with an alkyldiol to synthesize a hydroxyalkyl maleate, and the hydroxyalkyl maleate is then allowed to react with, for example, epichlorohydrin, followed by intramolecular epoxidation. Specifically, the compound of the formula (I-c) can be prepared in accordance with the process for synthesizing the compound of the formula (I-a).

The compound of the formula (I-d) can be prepared by allowing a compound of the following formula (I-d') to react with NH₂-R²-OH and then with a halide containing a structure of the R¹ group, followed by intramolecular epoxidation.

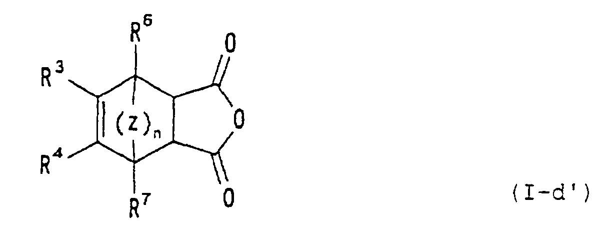

For example, an imide compound is synthesized from endomethylenetetrahydrophthalic anhydride and a nydroxyalkylamine, and the imide compound is then allowed to react with, for example, epichlorohydrin, followed by intramolecularepoxidation. Specifically, the compound of the formula (I-d) can be prepared in accordance with the process for synthesizing the compound of the formula (I-a).

The compound of the formula (I-e) can be prepared by allowing a compound of the above formula (I-d') to react with OH-R²-OH and then with a halide containing a structure of the R¹ group, followed by intramolecular epoxidation. For example, endomethylenetetrahydrophthalic anhydride is allowed to react with an alkyldiol to synthesize hydroxyalkyl endomethylenetetrahydrophthalate, and the hydroxyalkyl endomethylenetetrahydrophthalate is then allowed to react with, for example, epichlorohydrin, followed by intramolecular epoxidation. Specifically, the compound of the formula (I-e) can be prepared in accordance with the process for synthesizing the compound of the formula (I-a).

The compound of the formula (I-f) can be prepared by allowing a compound of the following formula (I-f') to react with OH-R²-OH and then with a halide containing a structure of the R¹ group, followed by intramolecular epoxidation. 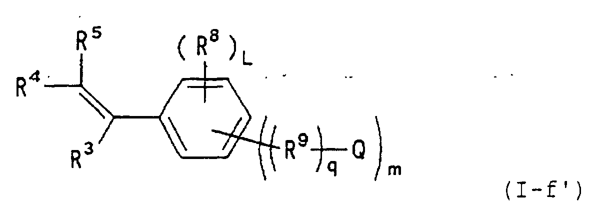 wherein Q is a halogen atom.

For example, 4-(chloromethyl)styrene is allowed to react with an alkyldiol to synthesize 4-(hydroxyalkoxymethyl)styrene, and the 4-(hydroxyalkoxymethyl)styrene is then allowed to react with, for example, epichlorohydrin, followed by intramolecular epoxidation. Specifically, the compound of the formula (I-f) can be prepared in accordance with the process for synthesizing the compound of the formula (I-a). The reaction route is represented by the following reaction formula.

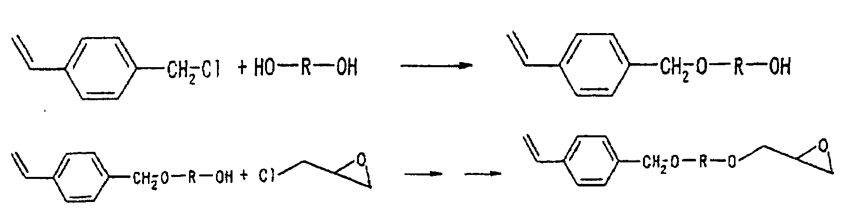

The compound of the formula (I-f) can be prepared by adding an alkylene oxide to a compound of the following formula (I-f") through addition reaction and then allowing the resulting compound to react with a halide containing a structure of the R¹ group.

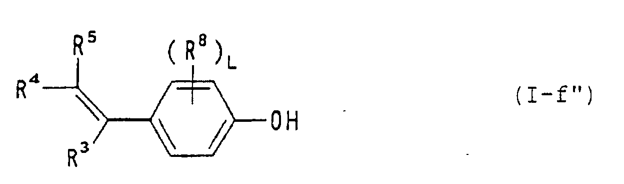

For example, an alkylene oxide is added to alkylated propenylphenol in a conventional manner, and the resulting compound is then allowed to react with, for example, epichlorohydrin. Specifically, the compound of the formula (I-f) can be prepared in accordance with the process for synthesizing the compound of the formula (I-a). The reaction route is represented by the following reaction formula.

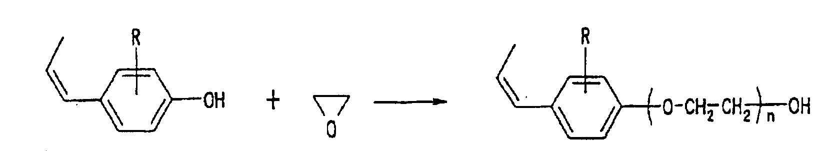

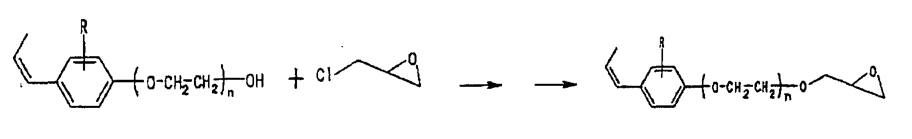

Specific examples of the polar group-containing unsaturated compounds represented by the formula (I) include a polar group-containing unsaturated imide compound, a polar group-containing vinylbenzene derivative and a glycidyl polyester acrylate compound which are described below.

### Polar group-containing unsaturated imide compound

The polar group-containing unsaturated imide compound is represented by the following formula (I-g): 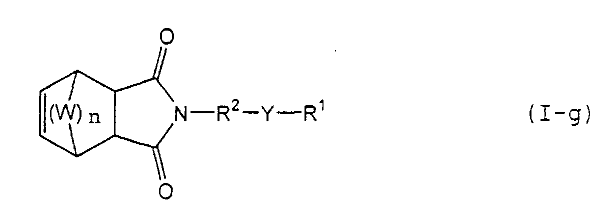 wherein W is -CH(R¹³)- (with the proviso that R¹³ is a hydrogen atom or an alkyl group of 1 to 5 carbon atoms),
n is 0 or 1,
R² is a straight-chain or branched alkylene group or an arylene group,
Y is -O₂C-, and
R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group.

In the formula (I-g), the straight-chain or branched alkylene group indicated by R² has carbon atoms of preferably 2 to 20 , more preferably 2 to 10, and the arylene group indicated by R² has carbon atoms of preferably 6 to 20, more preferably 6 to 10. Examples of the straight-chain or branched alkylene groups include -(CH₂)₂-, -(CH₂)₃-, -(CH₂)₄-, -(CH₂)₅-, -(CH₂)₆-, -CH(CH₃)CH₂-, -CH₂CH(CH₃)-, -CH(C₂H₅)CH₂- and -CH₂CH(C₂H₅)-. Examples of the arylene groups include o-phenylene, m-phenylene and p-phenylene.

The alkyl group containing a carboxylic anhydride group or a carboxyl group, that is indicated by R¹, has carbon atoms of preferably 1 to 20, more preferably 5 to 20; the cycloalkyl group has carbon atoms of preferably 3 to 20, more preferably 5 to 20; and the aryl group has carbon atoms of preferably 6 to 20. Examples of the alkyl groups, cycloalkyl groups and aryl groups containing a carboxylic anhydride group or a carboxyl group include a phthalic anhydride group and a phthalic acid group that is a ring opening group of the phthalic anhydride group.

R¹³ is a hydrogen atom or an alkyl group of 1 to 5 carbon atoms. Examples of the alkyl groups include methyl, ethyl, propyl and butyl. R¹³ is particularly preferably a hydrogen atom or methyl, and in this case, X is -CH₂- or -CH(CH₃)-.

In the formula (I-g), it is preferable that X is -CH₂-, n is 1, R² is a straight-chain or branched alkylene group, and R¹ is any one of an alkyl group, a cycloalkyl group and an aryl group each of which contains a carboxylic anhydride group or a carboxyl group.

In the polar group-containing unsaturated imide compound represented by the formula (I-g), the bond chain linking the double bond of the norbornene ring to the carboxylic anhydride group or the carboxylic group in R¹ is preferably constituted of 6 or more atoms.

Examples of the polar group-containing unsaturated imide compounds represented by the formula (I-g) include the following compounds (a) to (i).

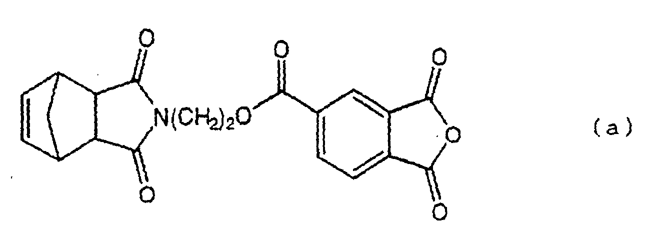

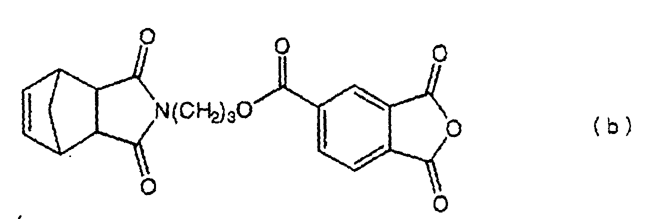

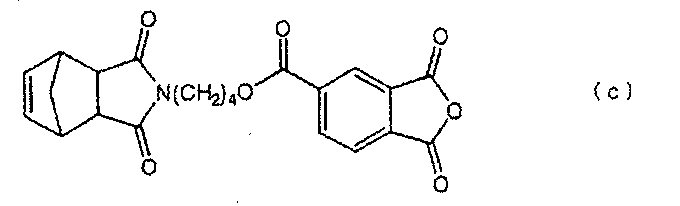

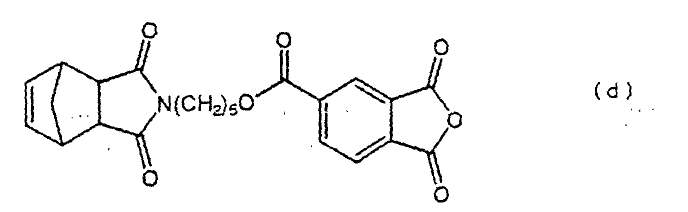

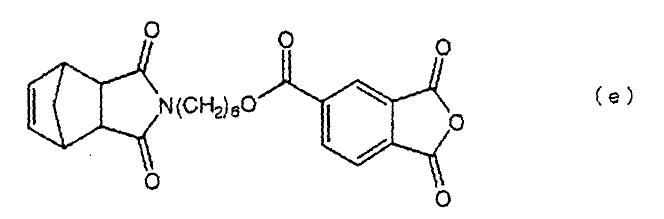

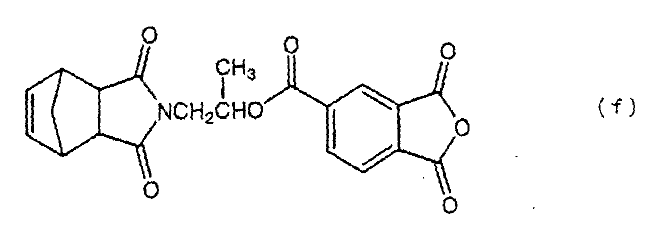

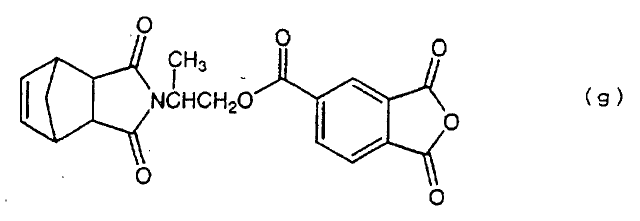

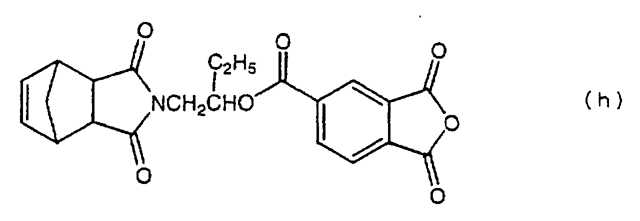

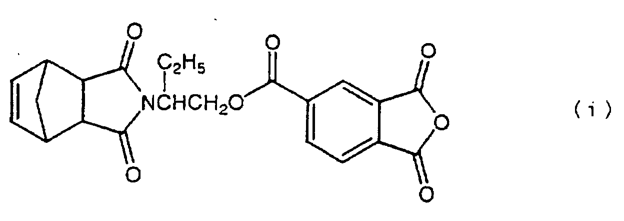

The polar group-containing unsaturated imide compound can be prepared by the following process.

For example, a carboxylic anhydride compound such as endomethylenetetrahydrophthalic anhydride or methylendomethylenetetrahydrophthalic anhydride is used as a starting material, and addition reaction of an amino alcohol to the acid anhydride group is performed to give an acid amide, followed by cyclodehydration reaction whereby an unsaturated imide compound having a hydroxyl group (unsaturated imide alcohol compound) that is an intermediate product is obtained. Then, the unsaturated imide alcohol compound is allowed to react with trimellitic anhydride chloride in the presence of a base to obtain the desired polar group-containing unsaturated imide compound. In another way to introduce the trimellitic anhydride group, dehydration reaction of the carboxylic acid with the unsaturated imide alcohol compound is performed using a dehydrating agent (reaction agent) suchas 2-halopyridinium salt-amine, dicyclohexylcarbodiimide, N,N'-carbonyldiimidazole or trifluoroacetic anhydride.

### Imidation reaction conditions

In the above process, the reaction of the carboxylic anhydride compound with the amino alcohol is carried out as follows. These materials are usually fed in a molar ratio of 1:1, or any one of them is used in an amount larger than that of the other by 10 to 20 % by mol, and water produced as a by-product is removed by azeotropic dehydration using a solvent such as toluene, xylene or benzene. In this reaction, a catalyst is used to accelerate the reaction, and examples of such catalysts include acid compounds such as sulfuric acid, phosphoric acid, p-toluenesulfonic acid and methanesulfonic acid, and acid ion-exchange resins such as Amberlyst A15.

The reaction is usually continued until any one of the materials disappears, and the reaction time is appropriately determined according to the substrate.

### Amino alcohol

Examples of the amino alcohols used as a starting material include 2-aminoethanol, 3-amino-1-propanol, 4-amino-1-butanol, 5-amino-1-pentanol, 6-amino-1-hexanol, 2-amino-1-propanol, 1-amino-2-propanol, 2-amino-1-butanol, 1-amino-2-butanol, 2-aminophenol, 3-aminophenol and 4-aminophenol.

### Reaction to introduce acid anhydride group

In order to introduce a trimellitic anhydride group as the acid anhydride group in the above process, the reaction is usually conducted as follows. Trimellitic anhydride chloride as a material and the unsaturated imide alcohol compound obtained by the previous reaction are fed in a molar ratio of 1:1, or any one of them is used in an amount larger than that of the other by 10 to 20 % by mol, and the reaction is conducted in a solvent and in the presence of a base under the conditions of a reaction temperature of 0 to 50°C and a pressure of atmospheric pressure to 1 MPa.

### Reaction solvent

The reaction solvent used herein can be appropriately selected from solvents of halogenated hydrocarbon type, aromatic hydrocarbon type, ester type, ether type, ketone type and nitrile type according to the solubility of the material and the reaction conditions. Examples of such solvents include dichloromethane, chloroform, 1,2-dichloroethane, benzene, toluene, xylene, ethyl acetate, diethyl ether, tetrahydrofuran, 1,4-dioxane, acetone and acetonitrile.

### Base

In the above process, a basic compound such as an amine is employable as a compound to accelerate the reaction. Example of the basic compounds include pyridine, triethylamine, trimethylamine and dimethylaminopyridine.

### Polar group-containing vinylbenzene derivative

The polar group-containing vinylbenzene derivative is represented by the following formula (I-h).

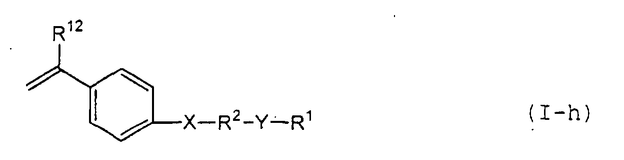

In the formula (I-h), R¹² is a hydrogen atom or a methyl group.

X¹ is an oxygen atom, and X² is a carbonyl group.

In the formula (I-h), R² is a single bond or a (poly)oxyalkylene group.

The (poly)oxyalkylene group is represented by the following formula:

-(R²¹O)q-

wherein R²¹ is an alkylene group, the number of carbon atoms in the alkylene group is preferably 1 to 8, more preferably 2 to 4, and q is an integer of preferably 1 to 10, more preferably 1 to 5.

The (poly)oxyalkylene group is, for example, a (poly)oxyalkylene group represented by the following formula (3):

-(CR²²R²³CR²⁴CR²⁵O)ₚ- (3)

wherein, R²², R²³, CR²⁴ and R²⁵ may be the same or different and are each a hydrogen atom or a methyl group, and p is an integer of preferably 1 to 10, more preferably 1 to 8, still more preferably 1 to 6.

Of such groups, preferably used is a (poly) oxyalkylene group represented by the following formula (1) or (2):

-(CH₂CH₂O)ₙ- (1)

wherein n is an integer of preferably 1 to 10, more preferably 1 to 8, still more preferably 1 to 5,

-(CH₂CHCH₃O)ₘ- (2)

wherein, in the formula (2), m is an integer of preferably 1 to 10, more preferably 1 to 8, still more preferably 1 to 5.

Examples of such (poly) oxyalkylene groups include the following groups.
- (CH₂)₂O -,
- CH₂CH₂OCH₂CH₂O -,
- (CH₂CH₂O)₂CH₂CH₂O -,
- (CH₂CH₂O)₃CH₂CH₂O -,
- (CH₂CH₂O)₄CH₂CH₂O -,
- CH₂CHCH₃O -,
- CH₂CHCH₃OCH₂CHCH₃O -,
- (CH₂CHCH₃O)₂CH₂CHCH₃O -,
- (CH₂CHCH₃O)₃CH₂CHCH₃O -,
- (CH₂CHCH₃O) ₄CH₂CHCH₃O -, and the like.

Also preferably used are oxyalkylene groups such as -(CH₂)₃O and -CHCH₃CH₂O-.

In the formula (I-h), R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group. The number of carbon atoms in the alkyl group containing a carboxylic anhydride group or a carboxyl group is preferably 1 to 20, more preferably 2 to 10, particularly preferably 3 to 8. The number of carbon atoms in the cycloalkyl group containing a carboxylic anhydride group or a carboxyl group is preferably 4 to 20, more preferably 6 to 15, particularly preferably 7 to 12. The number of carbon atoms in the aryl group containing a carboxylic anhydride group or a carboxyl group is preferably 7 to 20, more preferably 8 to 15, particularly preferably 8 to 12.

Examples of the alkyl groups include methyl, ethyl, n-propyl, isopropyl, n-butyl, isobutyl, sec-butyl, tert-butyl, neopentyl and n-hexyl. Examples of the cycloalkyl groups include cyclopentyl, cyclohexyl, cyclohexylmethyl and their derivatives. Examples of the aryl groups include phenyl, benzyl and their derivatives.

An example of the alkyl group of 1 to 20 carbon atoms, which contains a carboxylic anhydride group or a carboxyl group, is -CH₂CH₂-COOH derived from succinic anhydride.

Examples of the cycloalkyl groups of 1 to 20 carbon atoms, which contain a carboxylic anhydride group or a carboxyl group, include:

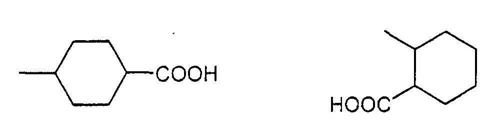

Examples of the aryl groups of 1 to 20 carbon atoms, which contain a carboxylic anhydride group or a carboxyl group, include a phthalic anhydride group represented by the following formula, a phthalic acid group that is a ring opening group of the phthalic anhydride group, derivatives thereof, and esters or metallic salts of these compounds.

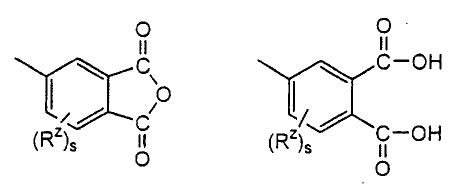

Examples of R^{Z} include an alkyl group of 1 to 10 carbon atoms, an aryl group of 6 to 10 carbon atoms, a cycloalkyl group of 3 to 10 carbon atoms, a COOH group, a derivative thereof, groups wherein an alkyl group of 1 to 10 carbon atoms, an aryl group and a cycloalkyl group of 3 to 10 carbon atoms are substituted with a COOH group and a derivative thereof, an OH group, a derivative thereof, and groups wherein an alkyl group of 1 to 10 carbon atoms, an aryl group of 6 to 10 carbon atoms and a cycloalkyl group of 3 to 10 carbon atoms are substituted with an OH group and a derivative thereof. When s is more than 1, R^{Z} and another R^{Z} may form a cyclic structure.

Examples of the alkyl groups of 1 to 10 carbon atoms, the aryl groups and the cycloalkyl groups include methyl, ethyl, n-propyl, isopropyl, n-butyl, isobutyl, sec-butyl, tert-butyl, neopentyl and n-hexyl.

An example of the aryl group is phenyl.

Examples of the cycloalkyl groups include cyclohexyl, cyclopentyl and cyclohexylmethyl.

Examples of the derivatives of the COOH group include an alkyl ester group and a salt of a metal such as Li, Na or K.

Examples of the derivatives of the OH group include an alkyl ether group and a silyl ether group.

More specifically, there can be mentioned the following groups.

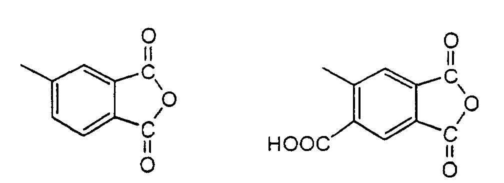

In the above formula (I-h), the bond chain linking the carbon of a carbonyl group of the carboxylic anhydride group or the carbon of a carbonyl group of the carboxyl group to the oxygen atom X¹ is desirably constituted of 5 or more atoms, preferably 7 or more atoms, more preferably 8 or more atoms. The upper limit of the number of atoms is not specifically restricted, but the number of atoms is usually 50 or less, preferably 30 or less.

The "bond chain" means a linkage of atomic bonds composed of the smallest number of atoms. Therefore, when R³ contains two or more carbonyl groups derived from the carboxyl group or the carboxylic anhydride group, the bond chain is so chosen that the number of atoms constituting the bond chain becomes the largest. For example, the number of atoms of the bond chain in the later-described compound (b) is 8 as shown below.

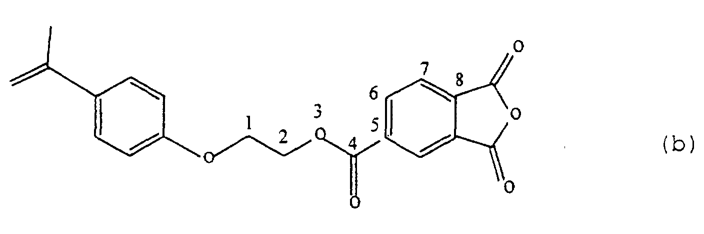

In the polar group-containing vinylbenzene compound represented by the formula (I-h), the bond chain linking the double bond of the vinyl group to the carboxylic anhydride group or the carboxyl group in R¹ is preferably constituted of 6 or more atoms.

Examples of the polar group-containing vinylbenzene derivatives represented by the formula (I-h) according to the invention include the following compounds (a) to (k).

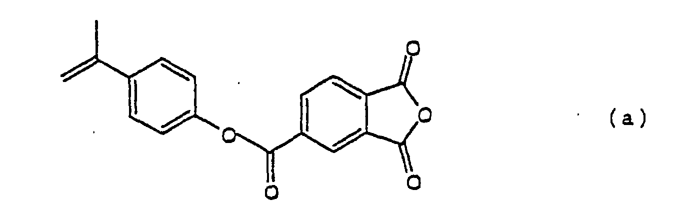

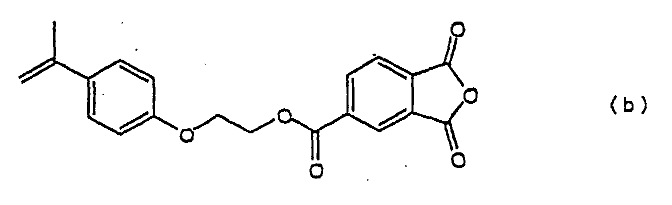

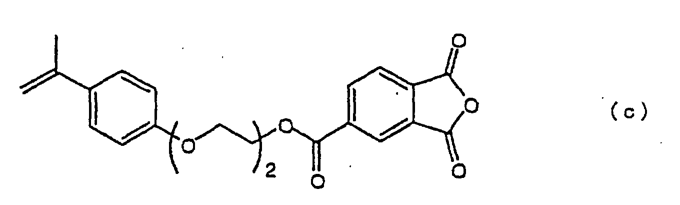

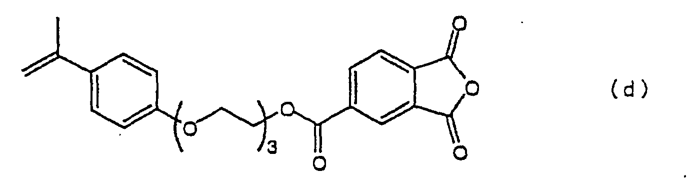

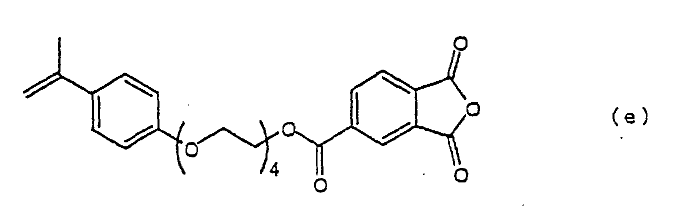

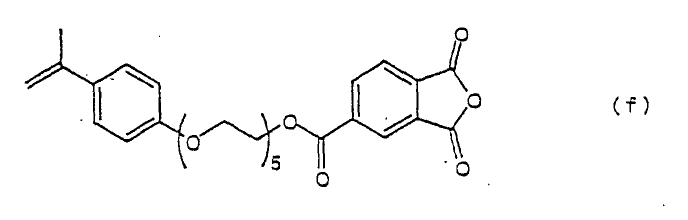

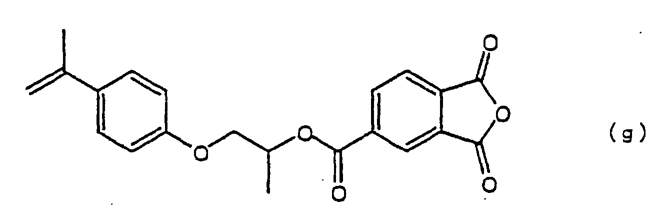

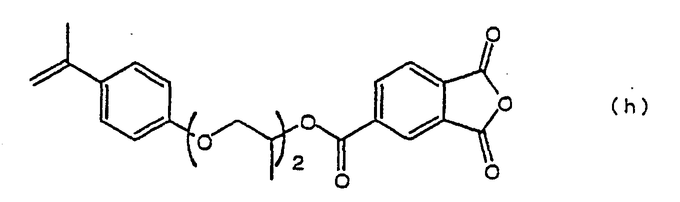

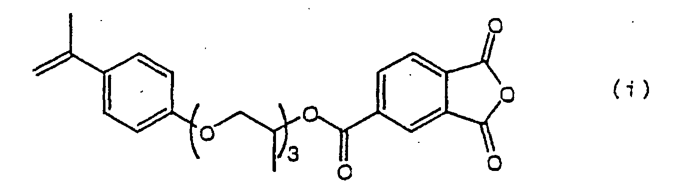

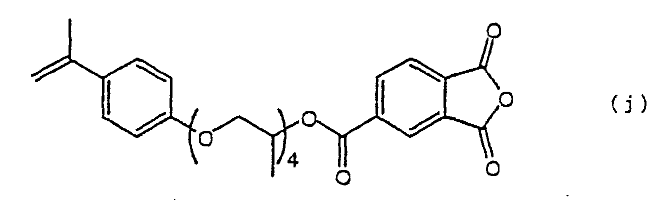

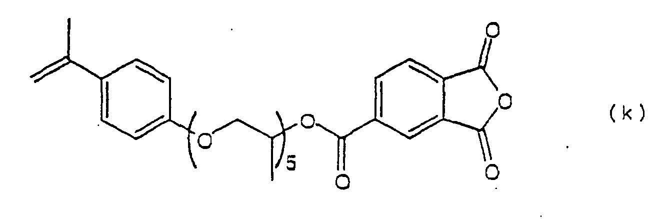

There is no specific limitation on the synthesis of the polar group-containing vinylbenzene derivatives, and they can be synthesized by the process described below.

When a phenol compound having a vinyl group is used as a starting material, the synthesis is carried out as follows. When R² in the formula (I-h) is a single bond, a phenol compound represented by the following formula (4) is brought into contact with a halide represented by the following formula (5) such as an acid halide, whereby polar group-containing vinylbenzene represented by the following formula (6) can be prepared (reaction step 2). 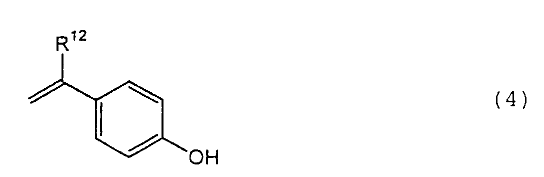 wherein R¹ is a hydrogen atom or a methyl group.

Hal - X² - R³ (5)

wherein Hal is a halogen atom, X² is a carbonyl group, R³ is any one of an alkyl group of 1 to 20 carbon atoms, a cycloalkyl group and an aryl group each of which contains a carboxylic anhydride group or a carboxyl group, and the bond chain linking the carbon of a carbonyl group of the carboxylic anhydride group or the carbon of a carbonyl group of the carboxyl group to X¹ is constituted of 5 or more atoms.

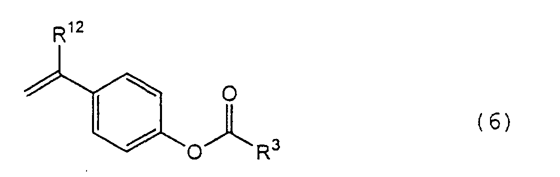

When R² in the formula (I-h) is a (poly)oxyalkylene group, an alkylene oxide represented by the formula R¹¹O is added to a phenol compound represented by the above formula (4) through addition reaction (reaction step 1) to obtain a compound represented by the following formula (7), and this compound is brought into contact with a compound represented by the above formula (5) (reaction step 2), whereby polar group-containing vinylbenzene represented by the following formula (8) can be prepared. 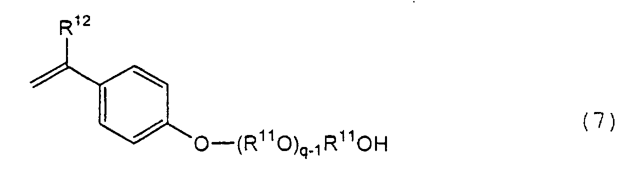 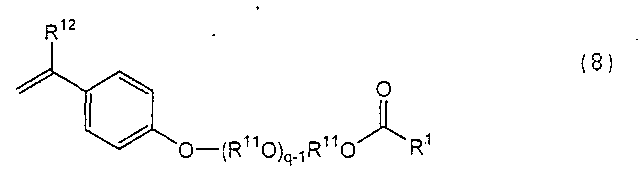 wherein, R¹² is a hydrogen atom or a methyl group, R¹¹ is an alkylene group of preferably 1 to 8 carbon atoms, more preferably 2 to 4 carbon atoms, q is an integer of preferably 1 to 10, more preferably 1 to 5, R¹ is any one of an alkyl group of 1 to 20 carbon atoms, a cycloalkyl group and an aryl group each of which contains a carboxylic anhydride group or a carboxyl group, and the bond chain linking the carbon of a carbonyl group of the carboxylic anhydride group or the carbon of a carbonyl group of the carboxyl group to X¹ is preferably constituted of 5 or more atoms.

When R² in the formula (I-h) is a covalent bond of an oxygen atom and a carbonyl group, p-isopropenylphenol as a starting material is allowed to react with trimellitic anhydride chloride in the presence of a base, whereby the desired polar group-containing vinylbenzene derivative can be obtained.

In case of the polar group-containing vinylbenzene derivative having a (poly)oxyalkylene group, an alkylene oxide such as ethylene oxide or propylene oxide is first added to p-isopropenylphenol through addition reaction, and the resulting (poly)alkylene glycol mono(p-isopropenylphenyl)ether is allowed to react with trimellitic anhydride chloride in the presence of a base, whereby the desired polar group-containing vinylbenzene derivative can be prepared.

In another way to introduce the trimellitic anhydride group, the carboxylic acid and the (poly)alkylene glycol mono(p-isopropenylphenyl)ether are subjected to dehydration reaction using a dehydrating agent (reaction agent) such as 2-halopyridinium salt-amine, dicyclohexylcarbodiimide, N,N'-carbonyldiimidazole or trifluoroacetic anhydride.

In the reaction to introduce an alkylene glycol chain into p-isopropenylphenol, an alkylene carbonate can be used instead of an alkylene oxide.

### Reaction step 1

The reaction of the phenol compound with the alkylene oxide or the alkylene carbonate in the process for preparing the polar group-containing vinylbenzene derivative is described below in more detail.

For the reaction of the phenol compound with the alkylene oxide or the alkylene carbonate in the process for preparing the polar group-containing vinylbenzene derivative, these starting materials are fed in a molar. ratio of 1:1, or the alkylene oxide or the alkylene carbonate is fed in large excess, and the reaction is conducted in the presence of a base under the conditions of a temperature of 0 to 200°C and a pressure of atmospheric pressure to 10 atm.

This reaction can be conducted using a large excess of the alkylene oxide or the alkylene carbonate without a solvent or with an appropriate solvent.

The base used in the reaction of the phenol compound with the alkylene oxide or the alkylene carbonate is, for example, a hydroxide or a carbonate of an alkali metal or an alkaline earth metal. Specific examples thereof include potassium hydroxide, sodium hydroxide, lithium hydroxide, magnesium hydroxide, calcium hydroxide, potassium carbonate, sodium carbonate, lithium carbonate, magnesium carbonate, calcium carbonate, potassium hydrogencarbonate, sodium hydrogencarbonate and lithium hydrogencarbonate. A solid base containing an alkali metal or an alkaline earth metal is also employable.

Examples of the solvents employable in the reaction of the phenol compound with the alkylene oxide or the alkylene carbonate include solvents of alcohol type, ketone type, amide type, aromatic hydrocarbon type, ether type, nitrile type and water. Specifically, there can be mentioned isopropanol, acetone, methyl ethyl ketone, methyl isobutyl ketone, dimethylformamide, toluene, xylene, tetrahydrofuran, 1,4-dioxane, acetonitrile and the like.

### Reaction step 2

The reaction (reaction step 2) of the (poly) alkylene glycol phenyl ether compound prepared as above or a starting phenol compound with trimellitic anhydride chloride can be conducted in a solvent using a base as a reaction accelerator.

For the reaction, the (poly)alkylene glycol phenyl ether compound or the starting phenol compound and the trimellitic anhydride chloride are fed in a molar ratio of 1:1 to 1:2, and the reaction is conducted under the conditions of a temperature of 0 to 50°C and a pressure of atmospheric pressure to 10 atm.

Examples of the bases used as the reaction accelerator include pyridine, triethylamine and dimethylaminopyridine.

The reaction solvent used herein can be appropriately selected from solvents of halogenated hydrocarbon type, aromatic hydrocarbon type, ether type, nitrile type, ketone type and ester type according to the solubility of the materials and the reaction conditions. Examples of such solvents include dichloromethane, chloroform, 1,2-dichloroethane, benzene, toluene, xylene, ethyl acetate, diethyl ether, dibutyl ether, tetrahydrofuran, 1,4-dioxane, acetone and acetonitrile.

The polar group-containing vinylbenzene derivative can be used for polymerization of polar group-containing derivative monomers, copolymerization with vinyl monomers such as ethylene and propylene, and graft modification of polyolefins. The resulting polymers are excellent in functions such as adhesion and compatibility with other resins.

Next, a polymer using the polar group-containing vinylbenzene derivative is described below.

The polar group-containing thermoplastic polymer according to the invention has a constituent unit represented by the following formula (I-h'): 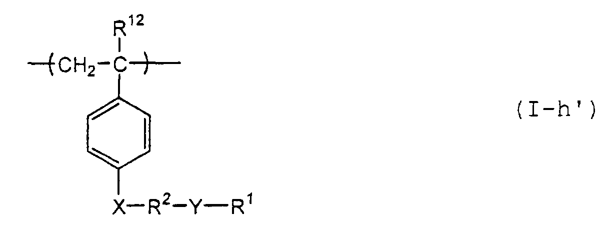 wherein R¹² is a hydrogen atom or a methyl group, X is an oxygen atom, Y is a carbonyl group, R² is a single bond or a (poly)oxyalkylene group, and R¹ is any one of an alkyl group of 1 to 20 carbon atoms, a cycloalkyl group and an aryl group each of which contains a carboxylic anhydride group or a carboxyl group.

The (poly)oxyalkylene group is represented by, for example, the following formula:

-(R²¹O)_{q}-

wherein R²¹ is an alkylene group, and q is an integer of 1 to 10. The number of carbon atoms in the alkylene group is preferably 1 to 8, more preferably 2 to 4.

The (poly)oxyalkylene group is, for example, a (poly)oxyalkylene group represented by the following formula (3):

-(CR²²R²³CR²⁴CR²⁵O)ₚ- (3)

wherein R²², R²³, CR²⁴ and R²⁵ may be the same or different and are each a hydrogen atom or a methyl group, and p is an integer of preferably 1 to 10, more preferably 1 to 8, still more preferably 1 to 6.

Of such groups, preferably used is an oxyalkylene group represented by the following formula (1) or (2):

-(CH₂CH₂O)ₙ- (1)

wherein n is an integer of preferably 1 to 10, more preferably 1 to 8, still more preferably 1 to 5,

-(CH₂CHCH₃O)ₘ- (2)

wherein m is an integer of preferably 1 to 10, more preferably 1 to 8, still more preferably 1 to 5, and CH₃ is a methyl group.

Examples of such (poly)oxyalkylene groups include the same groups as previously described.

In the formula (I-h'), R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group.

Examples of the alkyl groups include methyl, ethyl, n-propyl, isopropyl, n-butyl, isobutyl, sec-butyl, tert-butyl, neopentyl and n-hexyl. Examples of the cycloalkyl groups include cyclopentyl, cyclohexyl, cyclohexylmethyl and their derivatives. Examples of the aryl groups include phenyl, benzyl and their derivatives.

Examples of the alkyl groups of 1 to 20 carbon atoms, the cycloalkyl groups and the aryl groups containing a carboxylic anhydride group or a carboxyl group include the same groups as previously described with respect to R³ in the formula (I-h).

The polar group-containing thermoplastic polymer of the invention has a constituent unit represented by the aforesaid formula (I-h'), and in this polymer, the bond chain linking the carbon of a carbonyl group of the carboxylic anhydride group or the carbon of a carbonyl group of the carboxyl group to X¹ is desirably constituted of 5 or more atoms, preferably 7 or more atoms, still more preferably 8 or more atoms. The upper limit of the number of the atoms in the bond chain is not specifically restricted, but the number of atoms is usually 50 or less, preferably 30 or less.

### Glycidyl polyester acrylate compound

The glycidyl polyester acrylate compound has an acryloyl group as an unsaturated bond having a polymerizability and has a glycidyl group as a polar group.

This polar group-containing unsaturated compound is represented by the following formula (I-i).

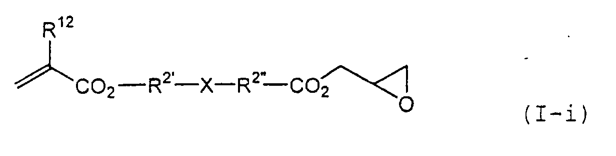

In the formula (I-i), R¹² is a methyl group (sometimes referred to as "Me") or a hydrogen atom.

R^{2'} is a straight-chain or branched alkylene group, an oxyalkylene group, an arylene group or a cycloalkylene group.

Examples of the alkylene group include groups of 2 to 10 carbon atoms, such as - (CH₂)₂-, - (CH₂)₃ -, - (CH₂)₄ -, - (CH₂)₅ -, - CH₂CHMe - and the like.

Examples of the oxyalkylene group include groups of 2 to 20 carbon atoms, such as - (CH₂)₂O -, - (CH₂)₃O -, - CH₂CHMeO -, - CHMeCH₂O -, - CH₂CH₂OCH₂CH₂O -, - (CH₂CH₂O)₂CH₂CH₂O -, - (CH₂CH₂O)₃CH₂CH₂O -, - CH₂CHMeOCH₂CHMeO -, - (CH₂CHMeO)₂CH₂CHMeO -, - (CH₂CHMeO)₃CH₂CHMeO - and the like.

As the arylene group, a phenylene group of 6 to 10 can be exemplified.

Examples of the cycloalkylene groups inlcude groups of 3 to 10 carbon atoms, such as - C₆H₁₀ -.

X is - CO₂ - or - O₂C -.

R2'' indicates liner or branched chain alkylene groups, oxyalkylene groups, arylene groups or cycloalkylene groups, such as - (CH₂)₂ - , - (CH₂)₃ -, - (CH₂)₄ - , - (CH₂)₅ -, - CH₂CHMe - and the like.

In the glycidylpolyester acrylate compounds represented by the above formula (I-i), it is preferable that R¹² be a halogen atom or a methyl group, R^{2'} indicate linear or branched chain alkylene group, oxyalene group or cycloalkylene group, Xbe -O₂C-, and R^{2''} be linear alkylene group.

Also preferably, R¹² is hydrogen atom, R^{2'} indicates linear alkylene group of 2 to 4 carbon atoms, X is - O₂C -, and R^{2''} is linear alkylene group of 2 to 4 carbon atoms.

Still preferably, R¹² is hydrogen atom, R^{2'} is ethylene, X is - O₂C - , and R" is ethylene.

In the glycidyl polyester acrylate compound represented by the formula (I-i), the bond chain linking the double bond in C=C (R¹²) - to the epoxy group is preferably constituted of 6 or more atoms.

Examples of such glycidyl polyester acrylate compounds represented by the formula (I-i) include the following compounds (a) to (t).

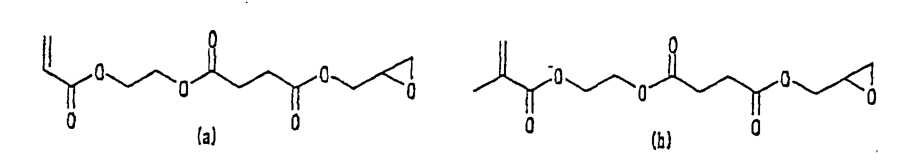

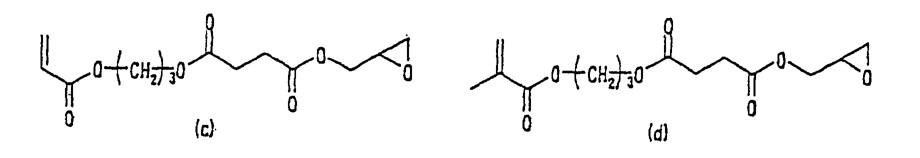

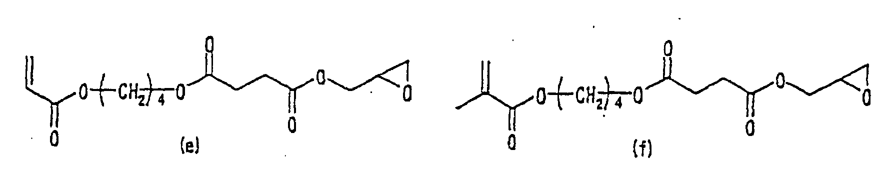

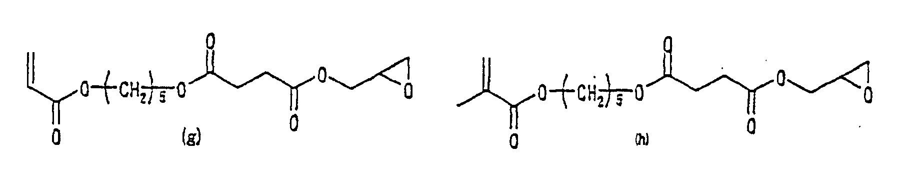

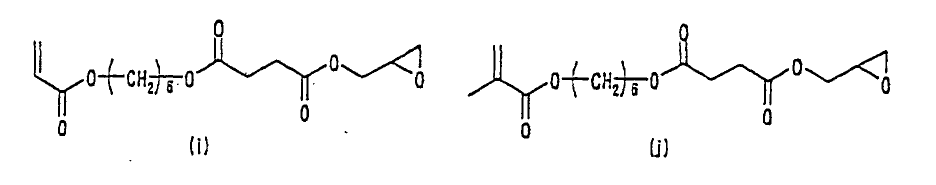

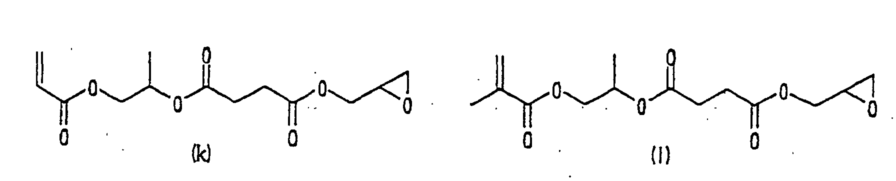

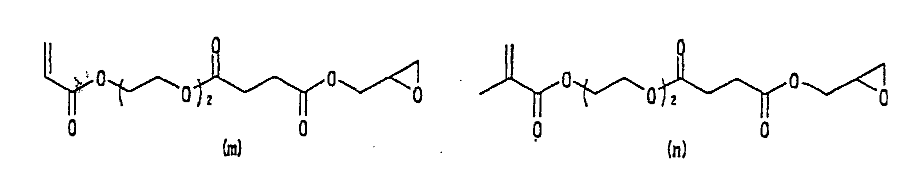

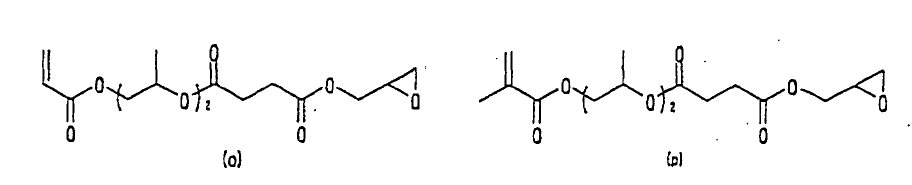

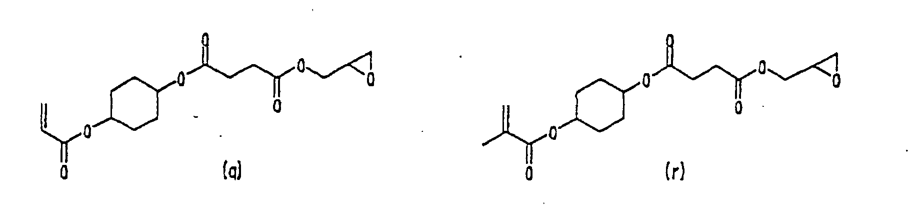

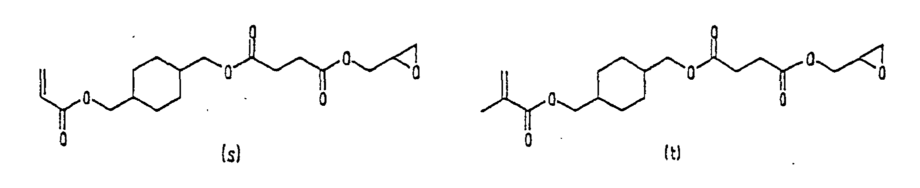

The glycidyl polyester acrylate compound has an epoxy group as a polar group in its molecule, so that if this compound is used for copolymerization with vinyl monomers such as ethylene and propylene or graft modification of polyolefins, the resulting polyolefins are imparted with functions such as adhesion and compatibility with other resins. Therefore, this compound is expected to be used as a material of various functional polyolefins, paints and adhesives.

Further, various groups can be introduced into R² and R¹⁴ each of which is a bond group present between the acryloyl group and the glycidyl group, according to the purpose such as development of novel properties or improvement of functions. Therefore, preparation of polyolefins imparted with properties suited to uses becomes feasible, and there is a possibility of a wide range of application.

The glycidyl polyester acrylate compound represented by the formula (I-i) can be prepared by the process described below.

Using acrylic acid, an alkanediol or the like as a starting material, a polyalkylene glycolmonoacrylate, i.e., a terminal hydroxyl group-containing acrylate compound, is first synthesized. The terminal group of the this terminal hydroxyl group-containing acrylate compound is changed to a terminal carboxyl group by the use of an acid anhydride such as succinic anhydride, and then the terminal carboxyl group is allowed to react with epichlorohydrin in the presence of a base to obtain the desired glycidyl group-containing acrylate compound.

### Synthesis of terminal hydroxyl group-containing acrylate compound

An alkylene oxide is added to acrylic acid or methacrylic acid through addition reaction to obtain an alkylene glycol monoacrylate having a hydroxyl group at the terminal. Instead of the alkylene oxide, an alkylene carbonate can be used.

Also by the reaction of an alkanediol with an acryloyl halide in a molar ratio of 1:1, a hydroxyalkyl acrylate can be obtained.

### Reaction conditions

For the reaction of the acrylic acid with the alkylene oxide or the alkylene carbonate in the above process, these starting materials are fed in a molar ratio of 1:1, or the alkylene oxide or the alkylene carbonate is fed in large excess, and the reaction is conducted in the presence of a base under the conditions of a temperature of 0 to 200°C, preferably 60 to 140°C, and a pressure of atmospheric pressure to 10 atm, preferably atmospheric pressure to 5 atm.

This reaction can be conducted using a large excess of the alkylene oxide or the alkylene carbonate without a solvent or with an appropriate solvent.

### Reaction solvent

The reaction solvent used herein can be selected from the same solvents as previously mentioned, such as solvents of alcohol type, ketone type, amide type, aliphatic hydrocarbon type, alicyclic hydrocarbon type, aromatic hydrocarbon type, ether type, nitrile type, sulfoxides and water, according to the solubility of the materials and the reaction conditions. Examples of such solvents include isopropanol, acetone, methyl ethyl ketone, methyl isobutyl ketone, dimethylformamide, n-pentane, n-hexane, cyclohexane, benzene, toluene, xylene, tetrahydrofuran, 1,4-dioxane, acetonitrile and dimethyl sulfoxide.

This reaction can be conducted using a large excess of the alkylene oxide or the alkylene carbonate without a solvent or with an appropriate solvent.

### Base

As the base, a hydroxide or a carbonate of an alkali metal or an alkaline earth metal is employable, and examples thereof include potassium hydroxide, sodium hydroxide, lithium hydroxide, magnesium hydroxide, calcium hydroxide, potassium carbonate, sodium carbonate, lithium carbonate, magnesium carbonate, calcium carbonate, potassium hydrogencarbonate, sodium hydrogencarbonate and lithium hydrogencarbonate. A solid base containing an alkali metal or an alkaline earth metal is also employable.

### Reaction of terminal hydroxyl group-containing acrylate compound with acid anhydride (succinic anhydride)

The reaction of the terminal hydroxyl group-containing acrylate compound prepared as above with the acid anhydride such as succinic anhydride is conducted in a solvent using a base as a reaction accelerator. An example of the reaction wherein succinic anhydride is used as the acid anhydride is described below.

### Reaction conditions

For the reaction, the terminal hydroxyl group-containing acrylate compound and the succinic anhydride are fed in a molar ratio of 1:1 to 1:3, preferably 1:1, and the reaction is conducted under the conditions of a temperature of preferably 0 to 50°C and a pressure of atmospheric pressure to 10 atm, preferably atmospheric pressure.

### Reaction solvent

The reaction solvent used herein can be selected from the same solvents as previously mentioned, such as solvents of halogenated hydrocarbon type, aliphatic hydrocarbon type, alicyclic hydrocarbon type, aromatic hydrocarbon type, ether type, nitrile type, ketone type, ester type and sulfoxides, according to the solubility of the materials and the reaction conditions.

### Base

Examples of the bases employable for accelerating the reaction in the above process include pyridine, triethylamine, dimethylaminopyridine and N-methylmorpholine.

### Reaction with epichlorohydrin

The reaction of the acryloyloxyalkylsuccinic ester compound prepared as above with epichlorohydrin is conducted in a solvent using a base as a reaction accelerator.

### Reaction conditions

For the reaction, the acryloyloxyalkylsuccinic ester and the epichlorohydrin are fed in a molar ratio of 1:1 to 1:10, more preferably 1:3, and the reaction is conducted under the conditions of a temperature of 0 to 200°C, preferably 100 to 150°C, and a pressure of atmospheric pressure to 10 atom, preferably atmospheric pressure to 5 atom.

### Base

Examples of the bases employable for accelerating the reaction in the above process include sodium carbonate, potassium carbonate, lithium carbonate, sodium hydrogencarbonate, sodium hydroxide, potassium hydroxide and lithium hydroxide.

### Reaction solvent

The reaction solvent used herein can be selected from the same solvents as previously mentioned, such as solvents of halogenated hydrocarbon type, aliphatic hydrocarbon type, alicyclic hydrocarbon type, aromatic hydrocarbon type, ether type, nitrile. type, ketone type, ester type and sulfoxides, according to the solubility of the materials and the reaction conditions.

Since the polar group-containing unsaturated compound represented by the formula (I) has a double bond, it can undergo radical reaction such as radical polymerization and is useful as a radical reaction compound. Therefore, if the compound is radical copolymerized with other monomers or used for graft reaction of polymers, properties of the resulting polymers can be changed (modified) . Further, the unsaturated compound of the invention is preferable as a polymer modifier.

Of the polymer modifiers of the invention comprising the unsaturated compounds, the compounds represented by the formulas (I-a) to (I-f) are preferable because polar group-containing thermoplastic polymers having a high content of polar groups and excellent adhesion properties can be obtained by graft polymerization.

Next, a polar group-containing thermoplastic polymer using the polar group-containing unsaturated compound is described.

### Polar group-containing thermoplastic polymer

Another embodiment of the polar group-containing thermoplastic polymer according to the invention has, in the polymer side chain, a structural part represented by the following formula (I').

-A'-X-R²-(Y)ₚ-R¹ (I')

In the formula (I'), A' is a group linking the polymer chain to X or a single bond. Specifically, A' is a group derived from the group indicated by A in the aforesaid formula (I) or a single bond. When the polar group-containing thermoplastic polymer is a polymer obtained by the use of the polar group-containing unsaturated compound represented by the formula (I) as a polymerization monomer, the C=C double bond in A becomes a part of the main chain of the polymer and the remainder of A becomes A'. When the polar group-containing thermoplastic polymer is a polymer obtained by graft polymerization of a thermoplastic resin using the polar group-containing unsaturated compound represented by the formula (I) as a graft monomer, the residue of A becomes A'.

X, R², Y, P and R¹ have the same meanings as those of X, R², Y, P and R¹ in the formula (I), respectively.

It is an important requisite that the bond chain linking the carbon atom of the polymer main chain, at which the side chain is bonded to the main chain, to at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group in R¹ is constituted of 6 or more atoms, preferably 7 or more atoms, more preferably 8 or more atoms, particularly preferably 9 or more atoms. The upper limit of the number of atoms is not specifically restricted, but the number of atoms is usually 55 or less, preferably 35 or less.

The bond chain linking C=O in the carboxylic acid group or the derivative thereof or C=O in the acid anhydride group in R¹ to X, or the bond chain linking the amino group, the cyano group, the isocyanate group, the sulfonic acid group, the metallic salt thereof, the phosphoric acid group, the metallic salt thereof or the epoxy group in R¹ to X is desirably constituted of 5 or more atoms, preferably 7 or more atoms, more preferably 8 or more atoms, particularly preferably 9 or more atoms. The upper limit of the number of atoms is not specifically restricted, but the number of atoms is usually 50 or less, preferably 30 or less.

In the polar group-containing thermoplastic polymer, it is preferable that, in the formula (I'), X is -COO-, -OCO-, -NHCO-, -CO-, -O- or -CONR¹⁰-, and R² is an alkylene group of 3 to 20 carbon atoms, an arylene group, a cycloalkylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

The polar group-containing thermoplastic polymer has a side chain of a specific structure wherein atoms of not less than a certain number are present between the polymer main chain and the functional group of the side chain, as shown in the formula (I'), and hence this polymer has high adhesion to metals or polar polymers.

The polar group-containing thermoplastic polymer has a side chain of a specific structure wherein atoms of not less than a certain number are present between X in the formula (I') and the functional group of the side chain, and hence this polymer has high adhesion to metals or polar polymers. When the polar group in R¹ is a carboxylic acid group, its derivative, an acid anhydride group, an amino group, an isocyanate group or an epoxy group, the polymer of the invention shows particularly excellent adhesion properties.

Synthesis of the polar group-containing thermoplastic polymer can be carried out by any of known processes. For example, a process of polycondensation of monomers having a structural part represented by the formula (I'), such as diol, dicarboxylic acid and diamine, a process of copolymerization of the polar group-containing unsaturated compound represented by the formula (I) and α-olefins such as ethylene and propylene or other polymerizable monomers using a transition metal catalyst, and a process of graft modification of a thermoplastic polymer with the polar group-containing unsaturated compound represented by the formula (I) in the presence or absence of a radical initiator are employable.

Of the above processes, the process of graft modification of a thermoplastic polymer with the polar group-containing unsaturated compound represented by the formula (I) in the presence or absence of a radical initiator is preferably employed because the reaction efficiency is high and a polar group-containing thermoplastic polymer can be efficiently obtained.

In the present invention, even if the polymer side chain does not have such a structural part as mentioned above, a polar group-containing thermoplastic polymer obtained by graft modifying a thermoplastic polymer with at least one of the unsaturated compounds of the invention exerts the effects of the invention.

### Thermoplastic polymer

The thermoplastic polymer employable for preparing the polar group-containing thermoplastic polymer in the invention is, for example, a polyolefin, a polystyrene, an acrylonitrile/butadiene/styrene copolymer (ABS), polyvinyl chloride, polyvinylidene chloride, polyvinyl acetate, an ethylene/(meth)acrylic ester copolymer or a diene rubber.

Examples of the polyolefins include olefin homopolymers, such as polyethylene, polypropylene, poly-1-butene, poly-4-methylpentene-1 and polymethylbutene; and olefin copolymers, such as an ethylene/α-olefin random copolymer, a propylene/ethylene random copolymer, a propylene/α-olefin random copolymer, a 4-methylpentene-1/α-olefin random copolymer, an ethylene/propylene/diene terpolymer and a propylene/ethylene/α-olefin terpolymer.

Of these, polyethylene, polypropylene, an ethylene/α-olefin random copolymer, an ethylene/propylene/dieneterpolymer, a propylene/ethylene random copolymer and a propylene/α-olefin random copolymer are preferable. When the polyolefin is a polyolefin obtained from an olefin of 3 or more carbon atoms, it may be an isotactic polymer, a syndiotactic polymer or an atactic polymer. As the catalyst for the preparation of the polyolefins, any of known catalysts such as Ziegler-Natta catalyst and metallocene catalyst may be used.

The ethylene homopolymer or the ethylene/α-olefin random copolymer preferably used in the invention desirably has a density of 850 to 960 kg/cm³ and a melt flow rate (MFR, ASTM D1238-65T, 190°C, load of 2.16 kg) of 0.01 to 100 g/10 min.

The polyolefins can be prepared by known processes such as high-pressure radical polymerization and medium- or low-pressure polymerization that is conducted in the presence of a transition metal compound catalyst such as Ziegler-Natta catalyst or metallocene catalyst.

Examples of the polystyrenes include a homopolymer of styrene, and a bipolymer of styrene and acrylonitrile, methyl methacrylate, α-methylstyrene or the like, e.g., an acrylonitrile/styrene copolymer.

As the ABS, preferably used is one containing 20 to 35 % by mol of constituent units derived from acrylonitrile, 20 to 30 % by mol of constituent units derived from butadiene and 40 to 60 % by mol of constituent units derived from styrene.

The polyvinyl chloride may be a homopolymer of vinyl chloride or may be a copolymer of vinyl chloride and vinylidene chloride, acrylic ester, acrylonitrile or propylene.

As the polyvinylidene chloride, a copolymer composed of not less than 85 % of vinylidene chloride and vinyl chloride, acrylonitrile, (meth)acrylic ester, allyl ester, unsaturated ether, styrene or the like is usually used.

The polyvinyl acetate may be a homopolymer of vinyl acetate or may be a copolymer of vinyl acetate and ethylene or vinyl chloride. Of these, an ethylene/vinyl acetate copolymer is preferable.

As the ethylene/(meth)acrylic ester copolymer, an ethylene/methyl acrylate copolymer, an ethylene/ethyl acrylate copolymer, an ethylene/methyl methacrylate copolymer or an ethylene/ethyl methacrylate is preferable.

Examples of the diene rubbers include conjugated polydienes such as an elastomer type styrene/butadiene copolymer that is known as polybutadiene, polyisoprene or SBR (styrene/butadiene rubber) . In such diene rubbers, at least a part of double bonds in the molecules may be hydrogenated.

The thermoplastic polymers mentioned above can be used singly or in combination of two or more kinds.

Of the above thermoplastic polymers, polyolefins are particularly preferable. The polyolefins are excellent particularly in mechanical properties and moldability, and show excellent low-temperature adhesion to metals and polar polymers.

### Radical initiator

Next, a radical initiator used for graft modification of a thermoplastic polymer with the aforesaid unsaturated compound is described.

The radical initiator used in the invention is, for example, an organic peroxide or an azo compound.

Examples of organic peroxydes include dicumylperoxyde, di-t-butylperoxyde, 2,5-dimethyl-2,5-bis(t-butylperoxy)hexane, 2,5-dimethyl-2,5-bis(t-butylperoxy)hexyne-3, 1,3-bis(t-butylperoxyisopropyl)benzene, 1,1-bis(t-butylperoxy)valerate, benzoylperoxyide, t-butylperoxybenzoate, acetylperoxyde, isobutylperoxyde, octanoylperoxide, decanoylperoxide, lauroylperoxyde, 3,5,5-trimethylhexanoylperoxide and 2,4-dichlorobenzoylperoxide, m-toluylperoxide and the like.
As azo compounds azoisobutylonitrile, dimethylazoisobutylonitrile can be exemplified.

The radical initiator is desirably used in an amount of usually 0.001 to 10 parts by weight, preferably 0.005 to 8 parts by weight, more preferably 0.01 to 5 parts by weight, based on 100 parts by weight of the thermoplastic polymer.

Although the radical initiator can be used by directly mixing it with the thermoplastic polymer, it may be used by dissolving it in a small amount of an organic solvent. As the organic solvent, any of organic solvents capable of dissolving the radical initiator can be used without any specific restriction. Examples of such organic solvents include aromatic hydrocarbon solvents, such as benzene, toluene and xylene; aliphatic hydrocarbon solvents, such as pentane, hexane, heptane, octane, nonane and decane; alicyclic hydrocarbon solvents, such as cyclohexane, methylcyclohexane and decahydronaphthalene; chlorinated hydrocarbons, such as chlorobenzene, dichlorobenzene, trichlorobenzene, methylene chloride, chloroform, carbon tetrachloride and tetrachloroethylene; alcohol type solvents, such as methanol, ethanol, n-propanol, isopropanol, n-butanol, sec-butanol and tert-butanol; ketone type solvents, such as acetone, methyl ethyl ketone and methyl isobutyl ketone; ester type solvents, such as ethyl acetate and dimethyl phthalate; and ether type solvents, such as dimethyl ether, diethyl ether, di-n-amyl ether, tetrahydrofuran and dioxyanisole.

### Other radical polymerizable compounds

In the present invention, to graft modify a thermoplastic polymer, the polar group-containing saturated compound may be used in combination with other radical polymerizable compounds within limits not detrimental to the objects of the invention.

The other radical polymerizable compound employable in the invention is at least one compound selected from a hydroxyl group-containing ethylenically unsaturated compound, an amino group-containing ethylenically unsaturated compound, an epoxy group-containing ethylenically unsaturated compound, an aromatic vinyl compound, an unsaturated carboxylic acid, its derivative, a vinyl ester compound, a nitrile group-containing unsaturated compound, vinyl chloride and the like.

In particular, examples of ethylene unsaturated compounds containing hydroxyl groups include (meth) acrylic esters, such as hydroxyethyl(meth)acrylate, 2-hydroxypropyl(meth)acrylate, 3-hydroxypropyl(meth)acrylate, 2-hydroxy-3-phenoxy-propyl(meth)acrlate, 3-chloro-2-hydroxypropyl(meth)acrylate, glycerine mono(meth)acrylate, pentaerythrytolmono(meth)acrylate, trimethylolpropanemono(meth)acrylate, tetramethylolethanemono(meth)acrylate, butanediolmono(meth)acrylate, polyethyleneglycolmono(meth)acrylate and 2-(6-hydroxyhexanoyloxy)ethylacrylate; 10-undecene-1-ol; 1-octene-3-ol; 2-methanolnorbornene; hydroxystyrene; hydroxyethylvinylether; hydroxybutylvinylether; N-methylolacrylamide; 2-(meth)acroyloxyethylacid phosphate; glycerin monoacrylether; allylalcohol; allyroxyethanol; 2-butene-1,4-diol; and glycerin monoalcohol.

The amino group-containing ethylenically unsaturated compound is a compound having an ethylenic double bond and an amino group. Such a compound is, for example, a vinyl monomer having at least one amino group or substituted amino group represented by the following formula: 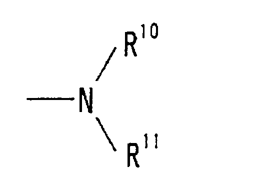 wherein R¹⁰ is a hydrogen atom, a methyl group or an ethyl group, and R¹¹ is a hydrogen atom, an alkyl group of 1 to 12 carbon atoms, preferably 1 to 8 carbon atoms, or a cycloalkyl group of 6 to 12 carbon atoms, preferably 6 to 8 carbon atoms. The alkyl group and the cycloalkyl group may have a substituent group.

Examples of the amino group-containing ethylenically unsaturated compounds include alkyl ester derivatives of acrylic acid or methacrylic acid, such as aminoethyl (meth)acrylate, propylaminoethyl (meth)acrylate, dimethylaminoethyl methacrylate, aminopropyl (meth)acrylate, phenylaminoethyl methacrylate and cyclohexylaminoethyl methacrylate; vinylamine derivatives, such as N-vinyldiethylamine and N-acetylvinylamine; allylamine derivatives, such as allylamine, methacrylamine, N-methylacrylamine, N,N-dimethylacrylamide and N,N-dimethylaminopropylacrylamide; acrylamide derivatives, such as acrylamide and N-methylacrylamide; aminostyrenes, such as p-aminostyrene; and other compounds, such as 6-aminohexylsuccinimide and 2-aminoethylsuccinimide.

The epoxy group-containing ethylenically unsaturated compound is a monomer having at least one polymerizable unsaturated bond and at least one epoxy group in one molecule. Examples of such epoxy group-containing unsaturated compounds include glycidyl acrylate, glycidyl methacrylate, mono and alkylglycidyl esters of dicarboxylic acids (number of carbon atoms of alkyl group in monoglycidyl ester: 1 - 12), such as mono and diglycidyl esters of maleic acid, mono and diglycidyl esters of fumaric acid, mono and diglycidyl esters of crotonic acid, mono and diglycidyl esters of tetrahydrophthalic acid, mono and diglycidyl esters of itaconic acid, mono and diglycidyl esters of butenetricarboxylic acid, mono and diglycidyl esters of citraconic acid, mono and diglycidyl esters of endo-cis-bicyclo[2.2.1]hept-5-ene-2,3-dicarboxylic acid (nadic acid™), mono and diglycidyl esters of endo-cis-bicyclo[2.2.1]hept-5-ene-2-methyl-2,3-dicarbo xylic acid (methylnadic acid™) and mono and diglycidyl esters of allylsuccinic acid, alkyl glycidyl esters of p-styrenecarboxylic acid, allyl glycidyl ether, 2-methylallyl glycidyl ether, styrene-p-glycidyl ether, 3,4-epoxy-1-butene, 3,4-epoxy-3-methyl-1-butene, 3,4-epoxy-1-pentene, 3,4-epoxy-3-methyl-1-pentene, 5,6-epoxy-1-hexene and vinylcyclohexene monoxide.

The aromatic vinyl compound is, for example, a compound represented by the following formula.

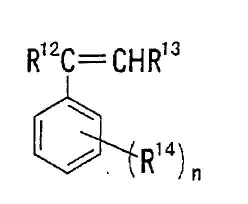

In the above formula, R¹² and R¹³ may be the same or different and are each a hydrogen atom or an alkyl group of 1 to 3 carbon atoms. Examples of the alkyl groups include methyl, ethyl, propyl and isopropyl. R¹⁴ is a hydrocarbon group of 1 to 3 carbon atoms or a halogen atom, and examples thereof include methyl, ethyl, propel, isopropyl, chlorine, bromine and iodine. n is an integer of usually 0 to 5, preferably 1 to 5.

Examples of the aromatic vinyl compounds include styrene, α-methylstyrene, o-methylstyrene, p-methylstyrene, m-methylstyrene, p-chlorostyrene, m-chlorostyrene and p-chloromethylstyrene, 4-vinylpyridine, 2-vinylpyridine, 5-ethyl-2-vinylpyridine, 2-methyl-5-vinylpyridine, 2-isopropenylpyridine, 2-vinylquinoline, 3-vinylisoquinoline, N-vinylcarbazole and N-vinylpyrrolidone.

Examples of the unsaturated carboxylic acids include unsaturated carboxylic acids, such as acrylic acid, methacrylic acid, maleic acid, fumaric acid, tetrahydrophthalic acid, itaconic acid, citraconic acid, crotonic acid, isocrotonic acid, norbornenedicarboxylic acid and bicyclo[2.2.1]hept-2-ene-5,6-dicarboxylic acid; and acid anhydrides or derivatives of these acids, such as acid halides, amides, imides and esters thereof.

Specific examples of such compounds include malenyl chloride, malenyl imide, maleic anhydride, itaconic anhydride, citraconic anhydride, tetrahydrophthalic anhydride, bicyclo[2.2.1]hept-2-ene-5,6-dicarboxylic anhydride, dimethyl maleate, monomethyl maleate, diethyl maleate, diethyl fumarate, dimethyl itaconate, diethyl citraconate, dimethyl tetrahydrophthalate, dimethyl bicyclo[2.2.1]hept-2-ene-5,6-dicarboxylate, acrylic acid, methyl acrylate, ethyl acrylate, propyl acrylate, butyl acrylate, 2-ethylhexyl acrylate, acryloyl chloride, acrylamide, methacrylic acid, methyl methacrylate, ethyl methacrylate, propyl methacrylate, butyl methacrylate, 2-ethylhexyl methacrylate, methacryloyl chloride, hydroxyethyl(meth)acrylate,hydroxypropyl (meth)acrylate, glycidyl (meth)acrylate, aminoethyl methacrylate, aminopropyl methacrylate, tetrahydrofurfuryl acrylate and tetrahyrofurfuryl methacrylate.

Of these, preferable are acrylic acid, methyl acrylate, ethyl acrylate, methacrylic acid, methyl methacrylate, ethyl methacrylate, acrylamide, methacrylamide, maleic anhydride, hydroxyethyl acrylate, hydroxyethyl methacrylate, glycidyl methacrylate, aminopropyl methacrylate, tetrahydrofurfuryl acrylate and tetrahydrofurfuryl methacrylate.

Examples of the vinyl ester compounds include vinyl acetate, vinyl propionate, vinyl n-butyrate, vinyl isobutyrate; vinyl pivalate, vinyl caproate, vinyl versatate, vinyl laurate, vinyl stearate, vinyl benzoate, vinyl p-t-butylbenzoate, vinyl salicylate and vinyl cyclohexanecarboxylate.

Examples of the nitrile group-containing unsaturated compounds include acrylonitrile, methacrylonitrile, fumaronitrile, allyl cyanide and cyanoethyl acrylate.

Of the above compounds, hydroxyethyl (meth)acrylate, 2-hydroxypropyl(meth)acrylate,aminoethyl (meth)acrylate, propylaminoethyl (meth) acrylate, glycidyl (meth) acrylate, styrene, (meth)acrylic acid, methyl (meth)acrylate, ethyl (meth)acrylate, tetrahydrofurfuryl (meth)acrylate, (meth)acrylamide and (meth)acrylonitrile are preferably employed.

When other radical polymerizable compounds are used, the amount thereof is in the range of usually 0.001 to 2 mol, preferably 0.05 to 1.5 mol, more preferably 0.01 to 1 mol, based on 1 mol of the unsaturated compound.

### Graft modification

The graft modification may be carried out by any of known methods within limits not detrimental to the objects of the invention.

For carrying out the graft modification, at least a part of the thermoplastic polymer may be in any of a solid state, a molten state or a dissolved state in an organic solvent.

When at least a part of the thermoplastic polymer is in a dissolved state in an organic solvent, the graft modification is carried out at a temperature of usually 70 to 200°C, preferably 80 to 190°C. As the organic solvent, any of organic solvents capable of dissolving the thermoplastic polymer can be used without specific restriction. When a polyolefin is used as the thermoplastic polymer, an aromatic hydrocarbon solvent, such as benzene, toluene or xylene, or an aliphatic hydrocarbon solvent, such as pentane, hexane or heptane, is used as the organic solvent.

When the thermoplastic polymer is in a molten state, the graft modification is carried out at a temperature of usually not lower than the melting point or the softening point of the thermoplastic polymer. When a polyolefin is used as the thermoplastic polymer, the reaction temperature is in the range of usually 80 to 300°C, preferably 80 to 250°C.

When an extruder is used in the graft modification, a mixture previously obtained from the unsaturated compound, the thermoplastic polymer and optionally a radical initiator may be fed from a hopper, or the thermoplastic polymer and optionally a radical initiator are fed from a hopper and then the unsaturated compound or optionally its solvent solution is fed through a feed opening provided at an arbitrary position between the hopper and the tip of the extruder.

In the present invention, a reducing material may be used for the graft modification of the thermoplastic polymer, within limits not detrimental to the objects of the invention. The reducing material has a function of increasing the graft quantity in the resulting graft modified thermoplastic polymer.

Examples of the reducing materials include iron(II) ion, chromium ion, cobalt ion, nickel ion., palladium ion, sulfite, hydroxylamine, hydrazine and compounds containing a group of -SH, SO₃H, -NHNH₂ or -COCH(OH)-.

Specific examples of the reducing materials include ferrous chloride, potassium bichromate, cobalt chloride, cobalt naphthenate, palladium chloride, ethanolamine, diethanolamine, N,N-dimethylaniline, hydrazine, ethyl mercaptan, benzenesulfonic acid and p-toluenesulfonic acid.

The reducing material is used in an amount of usually 0.001 to 5 parts by weight, preferably 0.1 to 3 parts by weight, based on 100 parts by weight of the thermoplastic polymer.

In the present invention, the unsaturated compound is used in an amount of usually 0.1 x 10⁻³ to 10 mol, preferably 0.2 x 10⁻³ to 6 mol, more preferably 1.0 × 10⁻³ to 2 mol, based on 100 g of the thermoplastic polymer.

In the polar group-containing thermoplastic polymer of the invention obtained by the graft modification mentioned above, the graft quantity of the polar group-containing unsaturated compound is in the range of usually 0.01 to 95 % by weight, preferably 0.03 to 90 % by weight, more preferably 0.05 to 85 % by weight.

An embodiment of the thermoplastic polymer composition according to the invention comprises 1 to 99 % by weight of a polar group-containing thermoplastic polymer (A) which has a structural part represented by the following formula (IV) in the polymer side chain and 99 to 1 % by weight of at least one thermoplastic polymer (B) which is different from the thermoplastic polymer (A).

-X-R²-(Y)ₚ-R¹ (IV)

wherein X is -COO-, -OCO-, -NHCO-, -CO-, -O-, -CONR¹⁰- or -NR¹⁰-, R¹⁰ is a hydrogen atom or a hydrocarbon group, and R¹⁰ may be bonded to a group to which X is linked or R² to form a cyclic structure (in this case, R¹⁰ may be a single bond),
R² is a divalent group containing a carbon atom,
Y is -COO-, -OCO-, -O-, -CONR¹¹-, -OCONR¹¹-or-NR¹¹CO-, R¹¹ is a hydrogen atom or a hydrocarbon group, and R¹¹ may be bonded to R² or R¹ to form a cyclic structure (in this case, R¹¹ may be a single bond),
p is 0 or 1,
R¹ is any one of an alkyl group of 1 to 20 carbon atoms, a cycloalkyl group and an aryl group each of which contains at least one group selected from the group consisting of a carboxylic acid group, a derivative thereof, an acid anhydride group, an amino group, a cyano group, an isocyanate group, an epoxy group, a sulfonic acid group, a metallic salt thereof, a phosphoric acid group and a metallic salt thereof, and
the bond chain linking C=O in the carboxylic acid group, the derivative thereof or the acid anhydride group in R¹ to X, or the bond chain linking the amino group, the cyano group, the isocyanate group, the epoxy group, the sulfonic acid group, the metallic salt thereof, the phosphoric acid group or the metallic salt thereof in R¹ to X is constituted of 5 or more atoms.

Another embodiment of the thermoplastic polymer composition according to the invention comprises 1 to 99 % by weight of a polar group-containing thermoplastic polymer (A) which has been graft modified with at least one unsaturated compound represented by the following formula (V) and 99 to 1 % by weight of at least one thermoplastic polymer (B) which is different from the thermoplastic polymer (A).

A-X-R²-(Y)ₚ-R¹ (V)

wherein A is a group containing a double bond,
X is -COO-, -OCO-, -NHCO-, -CO-, -O-, -CONR¹⁰-or-NR¹⁰-, R¹⁰ is a hydrogen atom or a hydrocarbon group, and R¹⁰ may be bonded to A or R² to form a cyclic structure (in this case, R¹⁰ may be a single bond),
R² is a divalent group containing a carbon atom,
Y is -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
p is 0 or 1,
R¹ is any one of an alkyl group of 1 to 20 carbon atoms, a cycloalkyl group and an aryl group each of which contains at least one group selected from the group consisting of a carboxylic acid group, a derivative thereof, an acid anhydride group, an amino group, a cyano group, an isocyanate group, an epoxy group, a sulfonic acid group, a metallic salt thereof, a phosphoric acid group and a metallic salt thereof, and
the bond chain linking C=O in the carboxylic acid group, the derivative thereof or the acid anhydride group in R¹ to X, or the bond chain linking the amino group, the cyano group, the isocyanate group, the epoxy group, the sulfonic acid group, the metallic salt thereof, the phosphoric acid group or the metallic salt thereof in R¹ to X is constituted of 5 or more atoms.

### Thermoplastic polymer composition

The thermoplastic polymer composition according to the invention comprises the polar group-containing thermoplastic polymer (A) and at least one thermoplastic polymer (B) which is different from the polymer (A).

### Thermoplastic polymer (B)

The thermoplastic polymer (B) is at least one thermoplastic polymer (B) which is different from the polar group-containing thermoplastic polymer (A), and is preferably a polymer containing, as main recurring units, recurring units derived from a monomer containing a polar group.

The expression "polymer containing, as main recurring units, recurring units derived from a monomer containing a polar group" means that, in the polymer, recurring units derived from a polar group-containing monomer containing an atom or atomic group of oxygen, nitrogen, sulfur, phosphorus, fluorine, chlorine, bromine or iodine are contained as constituent units and the content of the recurring units is usually not less than 50 % by mol.

Preferred examples of such polymers include polyamide resin, polyester resin, acrylic resin, polyphenylene sulfide resin, polycarbonate resin, polyacetal resin, polyphenylene oxide resin, polyarylate resin and polysulfone resin. Of these, polyamide resin, polyester resin and polyphenylene sulfide resin are preferable.

The thermoplastic polymer (B) may be used singly or in combination of plural kinds.

Examples of the polyamide resins include polycondensates of aliphatic, alicyclic or aromatic diamines, such as hexamethylenediamine, decamethylenediamine, dodecamethylenediamine, 2,2,4- or 2,4,4-trimethylhexamethylenediamine, 1,3- or 1,4-bis(aminomethyl)cyclohexane, bis (p-aminocyclohexylmethane) and m- or p-xylenediamine, with aliphatic, alicyclic or aromatic dicarboxylic acids, such as adipic acid, suberic acid, sebacic acid, cyclohexanedicarboxylic acid, terephthalic acid and is ophthalic acid; condensates of aminocarboxylic acids such as ε-aminocaproic acid and 11-aminoundecanoic acid; ring opening polymers of lactams such as ε-caprolactam and ω-laurolactam; copolymerization polyamides composed of these components; and mixtures of these polyamides.

Specific examples of the polyamides include nylon 6, nylon 66, nylon 610, nylon 9, nylon 11, nylon 12, nylon 6/66, nylon 66/610 and nylon 6/11. Of these, nylon 6 and nylon 66 are preferable.

The molecular weight of the polyamide used in the invention is not specifically restricted, and the relative viscosity thereof (nr, JIS K 6810, measured in 98% sulfuric acid) is desired to be not less than 1.5, preferably not less than 2.0.

The polyester resins are polycondensates of dicarboxylic acids, such as aromatic dicarboxylic acids (e.g., terephthalic acid, isophthalic acid, 2,6-naphthalenedicarboxylic acid, diphenyldicarboxylic acid, diphenoxyethanedicarboxylic acid), aliphatic dicarboxylic acids (e.g., succinic acid, glutaric acid, adipic acid, sebacic acid, azelaic acid, decanedicarboxylic acid) and alicyclic dicarboxylic acids (e.g., cyclohexanedicarboxylic acid), with diol compounds, such as aliphatic glycols (e.g., ethylene glycol, propylene glycol, tetramethylene glycol, heptamethylene glycol, diethylene glycol, triethylene glycol, tetraethylene glycol, trimethylene glycol (propanediol), pentanediol, neopentyl glycol, hexamethylene glycol, dodecamethylene glycol), alicyclic glycols (e.g., cyclohexanedimethanol) and aromatic diols (e.g., bisphenols, hydroquinone), or polyfunctional hydroxy compounds (e.g., trimethylolmethane, trimethylolethane, trimethylolpropane).

Specific examples of the polyesters include aromatic polyesters, such as polyethylene terephthalate, polyethylene naphthalate and polybutylene terephthalate, polycaprolactone, and polyhydroxy butyrate. Of these, polyethylene terephthalate is preferable.

Examples of polyacetals include polyformaldehyde (polyoxymethylene), polyacetaldehyde, polypropionaldehyde and polybutylaldehyde. Of these, polyformaldehyde is particularly preferable.

Examples of the acrylic resins include polyacrylic acid, polyacrylic ester such as polymethyl acrylate, polymethacrylic ester such as polymethyl methacrylate, and (meth)acrylate/α-methylstyrene copolymer. Of these, polymethyl methacrylate (PMMA) is preferable.

Examples of the polycarbonates include polycarbonates obtained from bis(4-hydroxyphenyl)methane, 1,1-bis(4-hydroxyphenyl)ethane, 2,2-bis(4-hydroxyphenyl)propane and 2,2-bis (4-hydroxyphenyl)butane. Of these, polycarbonate obtained from 2,2-bis(4-hydroxyphenyl)propane is preferable.

As the polyphenylene oxide, poly(2,6-dimethyl-1,4-phenylene oxide) is preferable.

The thermoplastic resin composition of the invention can be obtained by blending the polar group-containing thermoplastic polymer (A) with the thermoplastic polymer (B) other than the polymer (A) by melt kneading or the like.

In the thermoplastic resin composition, the polar group-containing thermoplastic polymer (A) is desirably contained in an amount of 1 to 99 % by weight, preferably 3 to 95 % by weight, more preferably 5 to 90 % by weight, and the thermoplastic polymer (B) other than the polymer (A) is desirably contained in an amount of 99 to 1 % by weight, preferably 97 to 5 % by weight, more preferably 95 to 10 % by weight.

To obtain the thermoplastic resin composition, the polar group-containing thermoplastic polymer (A) and at least one kinds of the thermoplastic polymer (B) other than the polymer (A) are mixed in the above amounts, then melt kneaded and granulated or pulverized. For the mixing, a Henschel mixer, a V-type blender, a ribbon blender, a tumbling blender or the like is employed. For the melt kneading, a single-screw extruder, a twin-screw extruder, a kneader, a Banbury mixer or the like is employed.

To the thermoplastic resin composition of the invention, heat stabilizers, weathering stabilizers, antistatic agents, lubricants, slip agents, nucleating agents, flame retardants, oils, pigments, dyes, and inorganic or organic fillers, such as glass fiber, carbon fiber, potassium titanate fiber, wollastonite, calcium carbonate, calcium sulfate, talc, glass flake, barium sulfate, clay, kaolin, silica powder, mica, calcium silicate, aluminum hydroxide, magnesium hydroxide, aluminum oxide and magnesium oxide, may be added.

The thermoplastic resin composition of the invention can be used in various fields wherein polyolefin resins and engineering plastics have been heretofore used, for example, fields of food containers, electric parts (those for cleaners, washing machines, etc.), electronic parts, automobile parts (e.g., bumper, interior trim, exterior trim) and mechanical parts, without specific restriction. In particular, the composition is favorably used for extrusion sheets, unstretched films, stretched films, filaments, extrusion molded products, injection molded products and blow molded products.

### Molded product

The molded product according to the invention is obtained by molding the above-mentioned thermoplastic resin composition. The molding method is not specifically restricted, and known methods such as extrusion molding, injection molding, blow molding, extrusion blow molding, inflation molding and mold stamping are adoptable. The molded products are divided into some groups, such as extrusion molding products, injection molded products, blow molded products, extrusion molded products, injection blow molded products, inflation molded products and mold stamping products, according to the molding methods.

### Extrusion molded product

There is no specific limitation on the shape of the extrusion molded product of the invention and the type of the manufactured article, and examples of the extrusion molded products include sheets, films, pipes, hoses, electric wire coverings and filaments. The molded products are favorably used particularly as sheets, films and filaments.

Such molded products can be produced by the use of known extrusion molding machines, and they can be produced by extruding a molten thermoplastic resin composition from a T-die using, for example, a single-screw extruder, a kneading extruder, a ram extruder or a gear extruder. As the extrusing conditions, known conditions are adoptable.

A stretched film can be produced by stretching the extrusion sheet or film using a known stretching machine. Examples of stretching methods include tentering (lengthwise-crosswise stretching, crosswise-lengthwise stretching), simultaneous biaxial stretching and monoaxial stretching. The stretch ratio of the stretched film is desired to be usually 20 to 70 times in case of biaxially stretched film and 2 to 10 times in case of monoaxially stretched film. The thickness of the stretched film is desired to be usually 5 to 200 µm.

From the thermoplastic resin composition of the invention, an inflation film can also be produced.

The sheet, unstretched film, and stretched film formed from the thermoplastic resin composition of the invention have excellent rigidity, heat rigidity, transparency, gloss, surface hardness, heat resistance, aging resistance, moisture resistance and gas barrier properties. Therefore, they can be favorably used for press through packages for packaging medical tablets or capsules, and packaging films.

The filament comprising the thermoplastic resin composition of the invention can be produced by extruding a molten thermoplastic resin composition through a spinneret. The filament thus obtained may be stretched. The stretching is made to such an extent that at least monoaxial molecular orientation of the thermoplastic resin composition is imparted to the filament, and the stretch ratio is desired to be usually 5 to 10 times.

The filament has excellent rigidity.

### Injection molded product

The injection molded product comprising the thermoplastic resin composition of the invention can be produced by a known injection molding machine. As the molding conditions, known conditions are adoptable. The injection molded product has excellent rigidity, heat rigidity, gloss, surface hardness, heat resistance, aging resistance, impact resistance, chemical resistance and abrasion resistance, so that it can be widely used in the fields of automobile interior trim, automobile exterior trim, housings of electrical appliances, and containers.

### Blow molded product

The blow molded product comprising the thermoplastic resin composition of the invention can be produced by a known blow molding machine. As the molding conditions, known conditions are adoptable.

In case of, for example, extrusion blow molding, the thermoplastic resin composition is extruded from a die at a resin temperature of 100 to 300 °C to give a tubular parison, then the parison is held in a mold having an intended shape, then air is blown into the parison, and the parison is fitted into the mold at a resin temperature of 130 to 300°C to obtain a hollow molded product. The stretch ratio is desired to be 1.5 to 5 times in the crosswise direction.

In case of, for example, injection blow molding, the thermoplastic resin composition is injected into a mold at a resin temperature of 100 to 300°C to give a parison, then the parison is held in the mold having an intended shape, then air is blown into the parison, and the parison is fitted into the mold at a resin temperature of 120 to 300°C to obtain a hollow molded product. The stretch ratio is desired to be 1.1 to 1.8 times in the lengthwise direction and 1.3 to 2.5 times in the crosswise direction.

The blow molded product has excellent rigidity, heat rigidity, transparency, gloss, surface hardness and gas barrier properties.

### Mold stamping product

The thermoplastic resin composition of the invention can be used as a substrate material in a method (mold stamping) of producing a composite integrally molded product through simultaneous press molding of a skin material and the substrate material. The mold stamping product obtained by this molding method is favorably used as automobile interior trim, such as door trim, rear package trim, sheet back garnish and instrument panel.

The mold stamping product has excellent rigidity, heat rigidity, gloss and surface hardness.

### EFFECT OF THE INVENTION

By the present invention, a novel polar group-containing unsaturated compound useful for modifying polymers or obtaining polar group-containing thermoplastic polymers is provided. The unsaturated compound can be used as a polymer modifier for introducing a polar group into a thermoplastic polymer or the like. The polar group-containing thermoplastic polymer of the invention has high adhesion to metals and polar group polymers. By the use of this polymer, an adhesive resin is provided.

By the present invention, further, a thermoplastic resin composition excellent in mechanical properties such as impact resistance and tensile properties is also provided. The molded products obtained by the use of this composition are excellent not only in mechanical properties such as impact resistance and tensile properties but also in rigidity, heat rigidity, transparency, gloss, surface hardness, oil resistance and gas barrier properties.

### EXAMPLE

The present invention is further described with reference to the following examples, but it should be construed that the invention is in no way limited to those examples.

Property values used in the present invention were measured by the following methods.

### Modification quantity of polar group-containing unsaturated compound

With respect to polymers modified with unsaturated compounds containing carboxylic acids, carboxylic acid derivatives or acid anhydrides, the modification quantity is determined from the acid value obtained by the following method. With respect to polymers modified with other polar group-containing unsaturated compounds, the modification quantity is determined from a nitrogen, oxygen or sulfur content obtained by the elemental analysis.

### Acid value

The sample is accurately weighed into a conical flask. To the sample, 100 ml of a mixed solvent of p-xylene and dimethyl sulfoxide (volume ratio: 9/1) is added, and they are heated to 110°C to dissolve the sample in the solvent.

After the sample is completely dissolved, the solution is allowed to stand for cooling for about 1 minute in the flask, and 1 ml of a phenolphthalein indicator is added. The solution is titrated with an ethanol solution. (concentration: 0.1 mol/l) of KOH to find the titration end point at which a slight red color which has developed by the last drop does not disappear for 30 seconds or more. The operation using no sample is carried out as a blank test.

Based on the results obtained above, the acid value of the sample is determined from the following formula.

Acid value (mg-KOH/g) = (A-B)×0.1×F×56.1/S

A: consumption (ml) of KOH solution required for titration of sample
B: consumption (ml) of KOH solution required for blank test
F: factor of KOH solution (0.1 mol/l)
S: weight (9) of sample

### Strength of adhesion to Al

### Preparation of film

On a press plate, an aluminum sheet of 0.1 mm thickness and an aluminum sheet of 100 µm thickness from the center of which a square of 15 cm× 15 cm has been cut away are superposed in this order, and on the center (cut portion) of the aluminum sheet, 3.3 g of a sample (modified polymer) is placed. Then, on the aluminum sheet, a polyethylene terephthalate sheet, an aluminum sheet and a press plate are further superposed in this order.

The sample interposed between the press plates is placed in a hot press at 200°C and preheated for about 7 minutes. In order to remove bubbles from the sample, an operation of pressure-application (50 kg/cm²-G)/pressure-release is repeated several times. Then, the pressure is increased to 100 kg/cm²-G, and the sample is heated for 2 minutes under pressure. After the pressure is released, the press plates are taken out of the pressing machine, then transferred into a different pressing machine wherein the pressing zone is maintained at 0°C, and then cooled for 4 minutes under a pressure of 100 kg/cm²-G. After the pressure is released, the sample is taken out. The resulting laminate of aluminum and the modified polymer is used for measuring the strength of adhesion to Al.

### Measurement of strength of adhesion to Al

The laminate of aluminum and the modified polymer is cut into a strip of 15cm×2cm. On the polymer surface of the strip, an aluminum sheet of 15cm×2cm (thickness: 50 µm) is placed, and they are bonded by the use of a heat sealing tester having been set at an upper heater temperature of 200°C and a lower heater temperature of 70°C. The resulting laminate is cut into a strip of 15 mm width, and the upper aluminum sheet (thickness: 50 µm) is peeled fromthe modified polymer film at a peel angle of 180° to measure a peel strength by a tensile tester.

### Impact resistance, Izod impact strength (notched)

The impact strength at 23°C was measured in accordance with ASTM D 256.

### Tensile test

A Dumbbell specimen punched from a pressed sheet of 1 mm thickness was subjected to a tensile test under the conditions of a temperature of 23°C, a span of 30 mm and a stress rate of 30 mm/min.

### Synthesis Example 1

### Synthesis of 4-(6-acryloxyhexyl)trimellitic anhydride

To 800 ml of a dichloromethane solution containing 66 g of 1,6-hexanediol and 78 ml of triethylamine, 200 ml of dichloromethane containing 50 g of acrylic acid chloride was dropwise added over a period of 3 hours with ice cooling. The mixture was stirred for 2 hours with ice cooling and then further stirred at room temperature for 12 hours to perform reaction. After the reaction was completed, the reaction mixture was washed with a 0.1M hydrochloric acid aqueous solution, distilled water and a saturated aqueous salt solution. Then, the organic layer was dried over anhydrous sodium sulfate. After the solvent was distilled off under reduced pressure, the residue was purified by column chromatography (developing solvent: hexane/ethyl acetate = 10/1, then hexane/ethyl acetate = 1/1) to obtain 6-hydroxyhexyl acrylate (41 g).

To 600 ml of a dichloromethane solution containing 40 g of 6-hydroxyhexyl acrylate and 24 ml of pyridine, 600 ml of a dichloromethane solution containing 59 g of trimellitic anhydride chloride was dropwise added over a period of 2 hours with ice cooling. The mixture was stirred for 2 hours with ice cooling and then further stirred at room temperature for 12 hours to perform reaction. After the reaction was completed, the reaction mixture was washed with a 0.1M hydrochloric acid aqueous solution and a saturated aqueous salt solution. Then, the organic layer was dried over anhydrous sodium sulfate. After the solvent was distilled off under reduced pressure, the residue was purified by column chromatography (developing solvent: hexane/ethyl acetate = 1/1) to obtain 4-(6-acryloxyhexyl)trimellitic anhydride (light yellow oily compound, 64 g). The results of ¹H-NMR (270 MHz) analysis of the resulting polar group-containing unsaturated compound (m-1) and the structural formula of the compound are given below.

### Analytical results

¹H-NMR (ppm): 8.65 (1H, s), 8.58 (1H, dd), 8.12 (1H, d), 6.42 (1H, d), 6.12 (1H, dd), 5.83 (1H, d), 4.10-4.45 (4H, m), 1.46-1.71 (8H, m)
IR (cm⁻¹): 2950, 1855, 1784, 1723

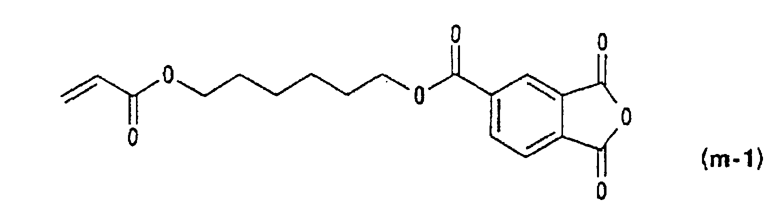

### Synthesis Example 2

### Synthesis of 4-(3-acryloxy-2,2-dimethylpropyl)trimellitic anhydride

To 800 ml of a dichloromethane solution containing 115 g of 2,2-dimethyl-1,3-propanediol and 78 ml of triethylamine, 200 ml of dichloromethane containing 50 g of acrylic acid chloride was dropwise added over a period of 3 hours with ice cooling. The mixture was stirred for 2 hours with ice cooling and then further stirred at room temperature for 12 hours to perform reaction. After the reaction was completed, the reaction mixture was washed with a 0.1M hydrochloric acid aqueous solution, distilled water and a saturated aqueous salt solution. Then, the organic layer was dried over anhydrous sodium sulfate. After the solvent was distilled off under reduced pressure, the residue was purified by column chromatography (developing solvent: hexane/ethyl acetate = 10/1, then hexane/ethyl acetate = 1/1) to obtain 3-hydroxy-2,2-dimethylpropyl acrylate (40 g).

To 600 ml of a dichloromethane solution containing 40 g of the resulting 3-hydroxy-2,2-dimethylpropyl acrylate and 24 ml of pyridine, 600 ml of a dichloromethane solution containing 59 g of trimellitic anhydride chloride was dropwise added over a period of 2 hours with ice cooling. The mixture was stirred for 2 hours with ice cooling and then further stirred at room temperature for 12 hours to perform reaction. After the reaction was completed, the reaction mixture was washed with a 0.1M hydrochloric acid aqueous solution and a saturated aqueous salt solution. Then, the organic layer was dried over anhydrous sodium sulfate. After the solvent was distilled off under reduced pressure, the residue was purified by column chromatography (developing solvent: hexane/ethyl acetate = 1/1) to obtain 4-(3-acryloxy-2,2-dimethylpropyl)trimellitic anhydride (64 g). The results of ¹H-NMR (270 MHz) analysis of the resulting polar group-containing unsaturated compound (m-2) and the structural formula of the compound are given below.

### Analytical results

Property: light yellow oily compound
¹H-NMR (ppm): 8.62 (1H, s), 8.55 (1H, dd, J₁=7.92Hz, J₂=1.32Hz), 8.13 (1H, d, J=7.92Hz), 6.39 (1H, d, J=17.31Hz), 6.15 (1H, dd, J₁=17.31Hz, J₂=10.39Hz), 5.85 (1H, d, J=10.39Hz), 4.26 (2H, s), 4.10 (2H, s), 1.13 (6H, s)

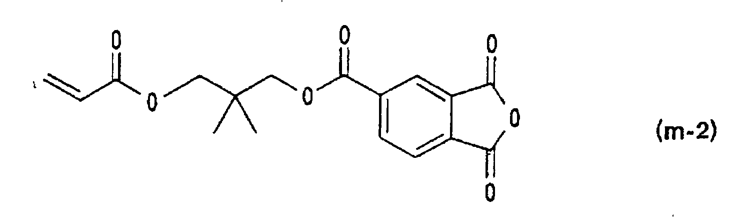

### Synthesis Example 3

### Synthesis of acryloxypolyethylene glycol trimellitic anhydride

In a 500 ml three-necked flask equipped with a Dimroth condenser and a 500 ml dropping funnel, 10 g (26 mmol) of polyethylene glycol monoacrylate (Blenmer AE-350, available from Nippon Oils & Fats Co., Ltd.) and 200 ml of methylene chloride were placed, and 3.1 g (40 mmol) of pyridine was slowly added with ice bath cooling. Then, 8.2 g (40 mmol) of trimellitic anhydride chloride was further slowly added with ice bath cooling. Thereafter, the mixture was stirred for 3 hours with ice bath cooling. The resulting precipitate was filtered off, and the filtrate was washed with dilute hydrochloric acid, water and a saturated aqueous salt solution. The organic layer was dried over anhydrous sodium sulfate, then the solvent was concentrated under reduced pressure, and the resulting product was purified by silica gel column chromatography to obtain 6.2 g (yield: 58 %) of acryloxypolyethylene glycol trimellitic anhydride.

The structural formula of the resulting polar group-containing unsaturated compound and the reaction formula are given below.

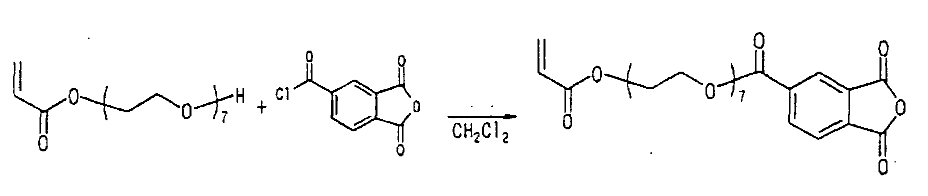

### Synthesis Example 4

In a 500 ml three-necked flask equipped with a Dimroth condenser and a 500 ml dropping funnel, 20 g (172 mmol) of hydroxyethyl acrylate and 1000 ml of methylene chloride were placed, and 16.3 g (207 mmol) of pyridine was slowly added with ice bath cooling. Then, 43.5 g (207 mmol) of trimellitic anhydride chloride was further slowly added with ice bath cooling. Thereafter, the mixture was stirred for 3 hours with ice bath cooling. The resulting precipitate was filtered off, then the solvent was concentrated under reduced pressure, and the resulting product was purified by silica gel column chromatography to obtain 41.2 g (yield: 83 %) of 2-acryloxyethyltrimellitic anhydride.

The structural formula of the resulting unsaturated compound (m-3) is given below.

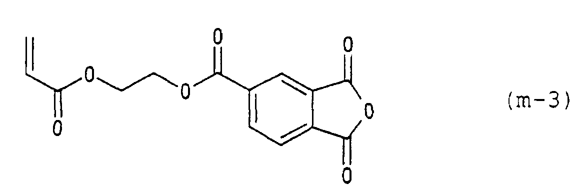

### Synthesis Example 5

In a 1 liter three-necked flask equipped with a Dimroth condenser and a 500 ml dropping funnel, 20.2 g (0.10 mol) of dodecanediol, 7.9 g (0.10 mol) of pyridine and 300 ml of methylene chloride were placed, and they were stirred at 45°C. Then, 100 ml of a methylene chloride solution containing 11.1 g (0.12 mol) of acrylic acid chloride was slowly dropwise added over a period of 1 hour through the dropping funnel. After the dropwise addition, the mixture was stirred for 6 hours at 45°C. After the reaction, the resulting precipitate was filtered off, and the filtrate was washed with water, dilute hydrochloric acid, water and a saturated aqueous salt solution. The organic layer was dried over anhydrous sodium sulfate, then the solvent was concentrated under reduced pressure, and the resulting product was subjected to silica gel column chromatography. From a hexane/ethyl acetate fraction, 5.7 g (yield: 22 %) of 12-hydroxydodecyl acrylate was obtained.

In a 500 ml three-necked flask equipped with a Dimroth condenser and a 500 ml dropping funnel, 4.6 g (22 mmol) of trimellitic anhydride chloride and 200 ml of methylene chloride were placed, and a solution containing 5 g (20 mmol) of 12-hydroxydodecyl acrylate, 1.8 g (22 mmol) of pyridine and 100 ml of methylene chloride was slowly dropwise added over a period of 30 minutes with ice bath cooling. Thereafter, the mixture was stirred for 3 hours in an ice bath. The resulting precipitate was filtered off, and the solvent was concentrated under reduced pressure, and the resulting product was purified by silica gel column chromatography to obtain 7.7 g (yield: 90 %) of 12-acryloxydodecyl trimellitic anhydride.

The structural formula of the resulting polar group-containing unsaturated compound (m-4) is given below.

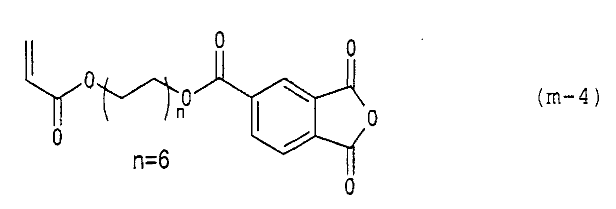

### Synthesis Example 6

In a 1000 ml three-necked flask equipped with a Dimroth condenser and a 500 ml dropping funnel, 180 g (0.855 mmol) of trimellitic anhydride chloride and 500 ml of methylene chloride were placed, and a solution containing 152 g (0.854 mol) of polyethylene glycol monoacrylate (AE-90, available from Nippon Oils & Fats Co., Ltd.), 67.5 g (0.854 mol) of pyridine and 200 ml of methylene chloride was dropwise added over a period of 1 hour with ice bath cooling. Thereafter, the mixture was stirred for 3 hours in an ice bath. The resulting precipitate was filtered off, then the solvent was concentrated under reduced pressure, and the resulting product was purified by silica gel column chromatography to obtain 254.2 g (yield: 89 %) of trimellitic anhydride polyethylene glycol monoacrylate ester.

The structural formula of the resulting polar group-containing unsaturated compound (m-5) is given below.

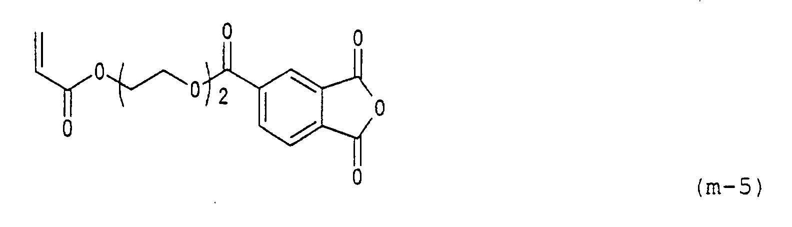

### Synthesis Example 7

In a 1000 ml three-necked flask equipped with a Dimroth condenser and a 500 ml dropping funnel, 31.5 g (0.15 mol) of trimellitic anhydride chloride and 190 ml of methylene chloride were placed, and a solution containing 42.4 g (0.15 mol) of (AE-200, available from Nippon Oils & Fats Co., Ltd.), 13.4 g (0.15 mol) of pyridine and 150 ml of methylene chloride was added over a period of 1 hour with ice bath cooling. Thereafter, the mixture was stirred for 3 hours in an ice bath. The resulting precipitate was filtered off, then the solvent was concentrated under reduced pressure, and the resulting product was purified by silica gel column chromatography to obtain 61.9 g (yield: 90 %) of trimellitic anhydride polyethylene glycol monoacrylate ester.

The structural formula of the resulting polar group-containing unsaturated compound (m-6) is given below.

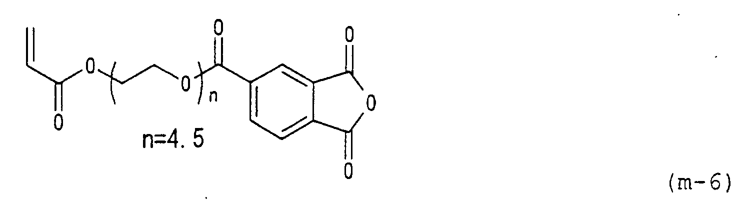

### Example 1

In 460 ml of chlorobenzene as a reaction solvent, 50 g of a propylene homopolymer (PP-1, MFR (measured at a temperature of 230°C under a load of 2.16 kg in accordance with ASTM D1238): 0.5 g/10 min) was dissolved at 140°C. Then, to the chlorobenzene solution, a chlorobenzene solution of the polar group-containing unsaturated compound (m-1) (concentration: 1.25 g (3.6×10⁻³ mol) /20 ml) obtained in Synthesis Example 1 and a chlorobenzene solution of dicumyl peroxide (DPC) (concentration: 0.5 g/20 ml) were slowly fed through different feed tubes over a period of 4 hours.

After the feeding was completed, the reaction was further continued for 2 hours at 140°C, and the reaction solution was cooled to room temperature to precipitate a polymer. The precipitated polymer was filtered, then repeatedly washed with acetone and vacuum dried at 80°C for one day and night to obtain the desired modified propylene homopolymer (MP-1). Measurement of an acid value of the modified propylene homopolymer resulted in 1.1 mg-KOH/g. From this result, the graft quantity of the polar group-containing unsaturated compound (m-1) in the modified propylene homopolymer (MP-1) was found to be 0.34 % by weight.

The strength of Al-adhesion of the modified propylene homopolymer (MP-1) was measured by the aforesaid method. The result is set forth in Table 1.

### Example 2

A modified propylene homopolymer (MP-2) was obtained in the same manner as in Example 1, except that a chlorobenzene solution containing, as the polar group-containing unsaturated compound, the polar group-containing unsaturated compound (m-2) (1.2 g (3.6×10⁻³ mol)/20 ml) obtained in Synthesis Example 2 was used.

Measurement of an acid value of the modified propylene homopolymer resulted in 1.2 mg-KOH/g. From this result, the graft quantity of the polar group-containing unsaturated compound (m-2) in the modified propylene homopolymer (MP-2) was found to be 0.36 % by weight.

The strength of Al-adhesion of the modified propylene homopolymer (MP-2) was measured by the aforesaid method. The result is set forth in Table 1.

### Example 3

In 170 ml of toluene as a reaction solvent, 50 g of a propylene homopolymer (PP-1) was dissolved at 140°C. Then, to the toluene solution, a toluene solution of the polar group-containing unsaturated compound (m-1) (2.5 g/40 ml) obtained in Synthesis Example 1 and a toluene solution of dicumyl peroxide (DPC) (1 g/40 ml) were slowly fed through different feed tubes over a period of 4 hours.

After the feeding was completed, the reaction was further continued for 2 hours at 140°C, and the reaction solution was cooled to room temperature to precipitate a polymer. The precipitated polymer was filtered, then repeatedly washed with acetone and vacuum dried at 80°C for one day and night to obtain the desired modified propylene homopolymer.

Measurement of an acid value of the modified propylene homopolymer resulted in 1.9 mg-KOH/g. From this result, the graft quantity in the modified propylene homopolymer was found to be 0.59 % by weight. The strength of Al-adhesion of the modified propylene homopolymer was measured by the aforesaid method. The result is set forth in Table 2, in which PP-2 designates a propylene homopolymer (MFR (230°C) : 14 g/10 min).

### Examples 4 - 9

A modified propylene homopolymer was obtained in the same manner as in Example 3, except that a polar group-containing unsaturated compound and a propylene homopolymer shown in Table 2 were used.

The graft quantity in the modified propylene homopolymer and the strength of Al-adhesion of the homopolymer were measured by the aforesaid methods. The results are set forth in Table 2.

### Comparative Example 1

A modified propylene homopolymer (MP-3) was obtained in the same manner as in Example 1, except that a chlorobenzene solution containing maleic anhydride (0.5 g (5.1×10⁻³ mol)/20 ml) instead of the polar group-containing unsaturated compound (m-1) was used.

Measurement of an acid value of the modified propylene homopolymer resulted in 2.3 mg-KOH/g. From this result, the graft quantity of the maleic anhydride in the modified propylene homopolymer (MP-3) was found to be 0.20 % by weight.

The strength of Al-adhesion of the modified propylene homopolymer (MP-3) was measured by the aforesaid method. The result is set forth in Table 1.

### Comparative Example 2

A modified propylene homopolymer was obtained in the same manner as in Example 3, except that a toluene solution containing maleic anhydride (2.0 g/40 ml) instead of the polar group-containing unsaturated compound (m-1) was used.

Measurement of an acid value of the modified propylene homopolymer resulted in 5.5 mg-KOH/g. From this result, the graft quantity of the maleic anhydride in the modified propylene homopolymer was found to be 0.48 % by weight. The strength of Al-adhesion of the modified propylene homopolymer was measured by the aforesaid method. The result is set forth in Table 2.

### Comparative Example 3

A modified propylene homopolymer was obtained in the same manner as in Example 8, except that a toluene solution containing maleic anhydride (7.2 g/40 ml) instead of the polar group-containing unsaturated compound (m-1) was used.

Measurement of an acid value of the modified propylene homopolymer resulted in 31.4 mg-KOH/g. From this result, the graft quantity of the maleic anhydride in the modified propylene homopolymer was found to be 2.75 % by weight. The strength of Al-adhesion of the modified propylene homopolymer was measured by the aforesaid method. The result is set forth in Table 2.

### Example 10

In 460 ml of chlorobenzene as a reaction solvent, 50 g of an ethylene/1-hexene copolymer (LLDPE-1, 1-hexene content: 5.9 % by mol, density: 0.91 g/cm³, MFR (measured at a temperature of 190 °C under a load of 2.16 kg in accordance with ASTM D1238): 4.0 g/10 min) was dissolved at 140°C. Then, to the chlorobenzene solution, a chlorobenzene solution of the polar group-containing unsaturated compound (m-1) (2.0 g (5.8×10⁻³ mol)/20 ml) obtained in Synthesis Example 1 and a chlorobenzene solution of dicumyl peroxide (DPC) (0.2 g/20 ml) were slowly fed through different feed tubes over a period of 4 hours.

After the feeding was completed, the reaction was further continued for 2 hours at 140°C, and the reaction solution was cooled to room temperature to deposit a polymer. The deposited polymer was filtered, then repeatedly washed with acetone and vacuum dried at 80°C for one day and night to obtain the desired modified ethylene/1-hexene copolymer (MP-4). Measurement of an acid value of the modified ethylene/1-hexene copolymer resulted in 5.9mg-KOH/g. From this result, the graft quantity of the polar group-containing unsaturated compound (m-1) in the modified ethylene/1-hexene copolymer (MP-4) was found to be 1.82 % by weight.

The strength of Al-adhesion of the modified ethylene/1-hexene copolymer (MP-4) was measured by the aforesaid method. The result is set forth in Table 1.

### Example 11

In 460 ml of toluene as a reaction solvent, 50 g of an ethylene/1-hexene copolymer (LLDPE-1) was dissolved at 140°C. Then, to the toluene solution, a toluene solution of the polar group-containing unsaturated compound (m-1) (2.5 g/40 ml) obtained in Synthesis Example 1 and a toluene solution of dicumyl peroxide (DPC) (0.25 g/40 ml) were slowly fed through different feed tubes over a period of 4 hours.

After the feeding was completed, the reaction was further continued for 2 hours at 140°C, and the reaction solution was cooled to room temperature to deposit a polymer. The deposited polymer was filtered, then repeatedly washed with acetone and vacuum dried at 80°C for one day and night to obtain the desired modified ethylene/1-hexene copolymer.

Measurement of an acid value of the modified ethylene/1-hexenecopolymerresultedin2.9mg-KOH/g. From this result, the graft quantity in the modified ethylene/1-hexene copolymer (m-1) was found to be 0.90 % by weight. The strength of Al-adhesion of the modified ethylene/1-hexene copolymer was measured by the aforesaid method. The result is set forth in Table 2.

### Example 12

A modified ethylene copolymer was obtained in the same manner as in Example 11, except that a toluene solution containing, as the polar group-containing unsaturated compound, the polar group-containing unsaturated compound (m-3) (2.5 g/40 ml) was used.

The graft quantity in the modified ethylene copolymer and the strength of Al-adhesion of the copolymer were measured by the aforesaid methods. The results are set forth in Table 2.

### Comparative Example 4

A modified ethylene/1-hexene copolymer (MP-5) was obtained in the same manner as in Example 10, except that a chlorobenzene solution containing maleic anhydride (0.5 g (5.1×10⁻³mol)/20ml) instead of the polar group-containing unsaturated compound (m-1) was used.

Measurement of an acid value of the modified ethylene/1-hexenecopolymerresultedin4.3mg-KOH/g. From this result, the graft quantity of the maleic anhydride in the modified ethylene/1-hexene copolymer (MP-5) was found to be 0.38 % by weight. The strength of Al-adhesion of the modified ethylene/1-hexene copolymer (MP-5) was measured by the aforesaid method. The result is set forth in Table 1.

### Comparative Example 5

A modified ethylene/1-hexene copolymer was obtained in the same manner as in Example 11, except that a toluene solution containing maleic anhydride (0.5 g/40 ml) instead of the polar group-containing unsaturated compound (m-1) was used.

Measurement of an acid value of the modified ethylene/1-hexene copolymer resulted in 6.8 mg-KOH/g. From this result, the graft quantity of the maleic anhydride in the modified ethylene/1-hexene copolymer was found to be 0.6 % by weight. The strength of Al-adhesion of the modified ethylene/1-hexene copolymer was measured by the aforesaid method. The result is set forth in Table 2.

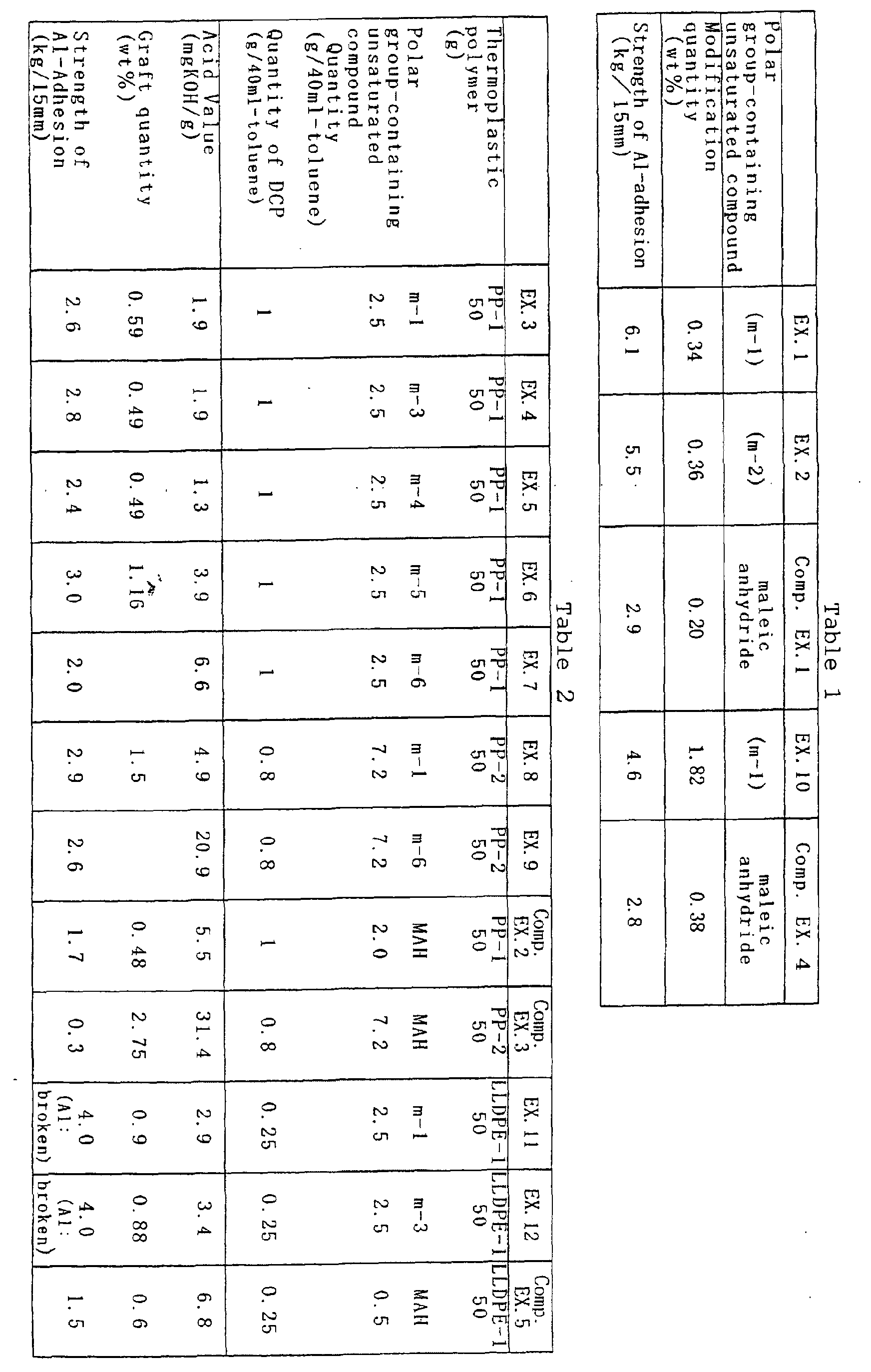

### Synthesis Example 8

In a 500 ml three-necked flask equipped with a Dimroth condenser, 20 g (0.09 mol) of β-acryloyloxyethyl hydrogensuccinate, 8.0 g (0.06 mol) of potassium carbonate and 100 ml of acetone were placed, and they were stirred for 1 hour with refluxing under heating. The acetone was distilled off under reduced pressure, and the residue was dried under reduced pressure. Thereafter, 100 ml of toluene was added, and with stirring, 23.6 g (0.25 mol) of epichlorohydrin was dropwise added over a period of 20 minutes at room temperature using the dropping funnel. After the dropwise addition, the mixture was stirred for 20 minutes with refluxing under heating. After the reaction, potassium chloride produced was filtered off, and the residue was washed three times with 20 ml of ethyl acetate. The filtrate and the washing liquid were together washed with water and a saturated aqueous salt solution, and the organic layer was dried over anhydrous sodium sulfate. Then, the solvent was concentrated under reduced pressure, and the resulting solution was subjected to silica gel chromatography. From a hexane/ethyl acetate fraction, 7.0 g (yield: 28 %) of acryloyloxyethyl glycidyl succinate (m-7) was obtained. The results of ¹H-NMR (270 MHz) analysis of the resulting polar group-containing unsaturated compound (m-7) and the structural formula of the compound are given below.

### Analytical results

¹H-NMR (ppm) : 6.44 (1H, d), 6.15 (1H, dd), 5.88 (1H, d), 4.42 (1H, d), 4.36 (4H, m), 3.95 (1H, dd), 3.21 (1H, m), 2.85 (1H, dd), 2.69 (4H, m), 2.68 (1H, d)
IR(cm⁻¹): 2960, 1737, 1638, 1272, 1157

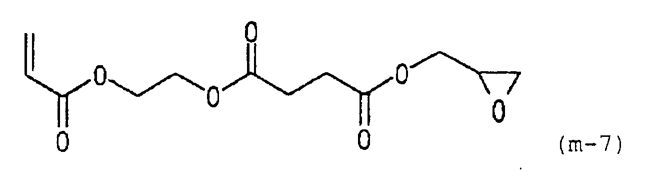

### Example 13

In 460 ml of chlorobenzene as a reaction solvent, 50 g of a propylene homopolymer (PP-1) was dissolved at 140°C. Then, to the chlorobenzene solution, a chlorobenzene solution of the polar group-containing unsaturated compound (m-7) (molecular weight: 346) (1.25 g (3.6×10⁻³ mol)/20 ml) obtained in Synthesis Example 8 and a chlorobenzene solution of dicumyl peroxide (DPC) (0.5 g/20 ml) were slowly fed through different feed tubes over a period of 4 hours. After the feeding was completed, the reaction was further continued for 2 hours at 140°C, and the reaction solution was cooled to room temperature to deposit a polymer. The deposited polymer was filtered, then repeatedly washed with acetone and vacuum dried at 80°C for one day and night to obtain the desired modified propylene homopolymer (MP-6).

From the elemental analysis of the modified propylene homopolymer (MP-6), the graft quantity of the compound (m-7) in the modified propylene homopolymer was found to be 0.3 % by weight. The strength of Al-adhesion of the modified propylene homopolymer (MP-6) was measured by the aforesaid method. The result is set forth in Table 3.

### Comparative Example 6

A modified propylene homopolymer (MP-7) was obtained in the same manner as in Example, except that a chlorobenzene solution containing glycidyl methacrylate (molecular weight: 142) (0.5 g (3.5×10⁻³ mol)/20 ml) instead of the polar group-containing unsaturated compound (m-7) was used.

From the elemental analysis of the modified propylene homopolymer (MP-7), the graft quantity of the glycidyl methacrylate in the modified propylene homopolymer was found to be 0.3 % by weight. The strength of Al-adhesion of the modified propylene homopolymer (MP-7) was measured by the aforesaid method. The result is set forth in Table 3.

### Example 14

In 170 ml of toluene as a reaction solvent, 50 g of an ethylene/1-hexene copolymer (LLDPE-1) was dissolved at 140°C. Then, to the toluene solution, a toluene solution of the polar group-containing unsaturated compound (m-7) (2.5 g/40 ml) obtained in Synthesis Example 8 and a toluene solution of dicumyl peroxide (DPC) (0.25 g/40 ml) were slowly fed through different feed tubes over a period of 4 hours.

After the feeding was completed, the reaction was further continued for 2 hours at 140°C, and the reaction solution was cooled to room temperature to deposit a polymer. The deposited polymer was filtered, then repeatedly washed with acetone and vacuum dried at 80°C for one day and night to obtain the desired modified ethylene/1-hexene copolymer.

From the elemental analysis of the modified ethylene/1-hexene copolymer, the graft quantity of the compound (m-7) in the modified ethylene/1-hexene copolymer was found to be 0.97 % by weight. The strength of A1-adhesion of the modified ethylene/1-hexene copolymer was measured by the aforesaid method, and as a result, it was 4.0 kg/15 mm. The result is set forth in Table 3.

### Example 15

A modified ethylene/1-octene copolymer was obtained in the same manner as in Example 14, except that an ethylene/1-octene copolymer (1-octene content: 21 % by mol, density: 0.888 g/cm³, MFR (190°C): 4.0 g/10 min) was used as a polyolefin.

From the elemental analysis of the obtained modified ethylene/1-octene copolymer, the graft quantity of the compound (m-7) in the modified ethylene/1-octene copolymer was found to be 1.21 % by weight. The strength of Al-adhesion of the modified ethylene/1-octene copolymer was measured by the aforesaid method, and as a result, it was 4.0 kg/15 mm. The result is set forth in Table 3.

**Table 3**

| | Ex. 13 | Comp. Ex. 6 | Ex. 14 | EX.15 |
|---|---|---|---|---|
| Thermoplastic polymer | P P-1 | P P-1 | LLDPE-1 | ethylene/octene copolymer |
| Polar group-containing unsaturated compound | (m-7) | glycidyl methacrylate | (m-7) | (m-7) |
| Modification quantity (wt%) | 0.3 | 0.3 | 0.97 | 1.21 |
| Strength of Al-Adhesion (kg/15mm) | 3.0 | 1.5 | 4.0 | 4.0 |

### Example 16

### Preparation of polar group-containing thermoplastic resin composition

To 10 % by weight of the modified propylene homopolymer (MP-1) obtained in Example 1, 30 % by weight of nylon 6 (NY 6, relative viscosity: 2.35) and 60 % by weight of a propylene homopolymer (PP-3, MFR (230°C, load of 2.16 kg) : 2.2 g/10 min) were added, and the mixture was melt kneaded by a twin-screw extruder of 20 mm diameter at 250°C to obtain a polar group-containing thermoplastic resin composition.

The polar group-containing thermoplastic resin composition was press molded under the prescribed conditions, and the properties of the molded product were measured by the aforesaid test methods. The results are set forth in Table 4.

### Example 17

### Preparation of polar group-containing thermoplastic resin composition

To 10 % by weight of the modified propylene homopolymer (MP-2) obtained in Example 2, 30 % by weight of nylon 6 (NY 6, relative viscosity: 2.35) and 60 % by weight of a propylene homopolymer (PP-3) were added, and the mixture was melt kneaded by a twin-screw extruder of 20 mm diameter at 250°C to obtain a polar group-containing thermoplastic resin composition.

The polar group-containing thermoplastic resin composition was press molded under the prescribed conditions, and the properties of the molded product were measured by the aforesaid test methods. The results are set forth in Table 4.

### Comparative Example 7

To 30 % by weight of nylon 6 (NY 6, relative viscosity: 2.35), 70 % by weight of a propylene homopolymer (PP-3) was added, and the mixture was melt kneaded by a twin-screw extruder of 20 mm diameter at 250°C to obtain a polar group-containing thermoplastic resin composition.

The polar group-containing thermoplastic resin composition was press molded under the prescribed conditions, and the properties of the molded product were measured by the aforesaid test methods. The results are set forth in Table 4.

### Example 18

### Preparation of acid anhydride group-containing unsaturated compound-modified ethylene/1-hexene copolymer

A modified ethylene/1-hexene copolymer (MP-8) was obtained in the same manner as in Example 1, except that an ethylene/1-hexene copolymer (EHC-1, 1-hexene content: 5.1 % by mol, density: 906 kg/m³, MFR (190°C, load of 2.16 kg): 3.9 g/10 min) was used instead of the propylene homopolymer (PP-1).

Measurement of an acid value of the modified ethylene/1-hexene copolymer (MP-8) resulted in 5.9mg-KOH/g. From this result, the graft quantity of the polar group-containing unsaturated compound (m-1) in the modified ethylene/1-hexene copolymer (MP-8) was found to be 1.8 % by weight.

### Preparation of polar group-containing thermoplastic resin composition

To 20 % by weight of the modified ethylene/1-hexene copolymer (MP-8) obtained above, 80 % by weight of nylon 6 (NY 6, relative viscosity: 2.35) was added, and the mixture was melt kneaded by a twin-screw extruder of 20 mm diameter at 250°C to obtain a polar group-containing thermoplastic resin composition.

The polar group-containing thermoplastic resin composition was press molded under the prescribed conditions, and the properties of the molded product were measured by the aforesaid test methods. The results are set forth in Table 4.

### Example 19

### Preparation of acid anhydride group-containing unsaturated compound-modified ethylene/propylene copolymer

A modified ethylene/propylene copolymer (MP-9) was obtained in the same manner as in Example 1, except that an ethylene/propylenecopolymer(EPC-1, propylenecontent: 62 % by mol, density: 859 kg/m³, MFR (190°C, load of 2.16 kg): 2.1 g/10 min) was used instead of the propylene homopolymer (PP-1).

Measurement of an acid value of the modified ethylene/propylene copolymer (MP-9) resulted in 5.2 mg-KOH/g. From this result, the graft quantity of the polar group-containing unsaturated compound (m-1) in the modified ethylene/propylene copolymer (MP-9) was found to be 1.6 % by weight.

### Preparation of polar group-containing thermoplastic resin composition

To 20 % by weight of the modified ethylene/propylene copolymer (MP-9) obtained above, 80 % by weight of nylon 66 (NY66, relative viscosity: 2.70) was added, and the mixture was melt kneaded by a twin-screw extruder of 20 mm diameter at 280°C to obtain a polar group-containing thermoplastic resin composition.

The polar group-containing thermoplastic resin composition was press molded under the prescribed conditions, and the properties of the molded product were measured by the aforesaid test methods. The results are set forth in Table 4.

### Comparative Example 8

To 80 % by weight of nylon 6 (relative viscosity: 2.35), 20 % by weight of an ethylene/1-hexene copolymer (EHC-1) was added, and the mixture was melt kneaded by a twin-screw extruder of 20 mm diameter at 250°C to obtain a polar group-containing thermoplastic resin composition.

The polar group-containing thermoplastic resin composition was press molded under the prescribed conditions, and the properties of the molded product were measured by the aforesaid test methods. The results are set forth in Table 4.

### Example 20

### Preparation of epoxy group-containing unsaturated compound-modified propylene homopolymer

A modified propylene homopolymer (MP-10) was obtained in the same manner as in Example 1, except that an epoxy group-containing unsaturated compound (polar group-containing unsaturated compound (m-8)) represented by the following formula was used instead of the polar group-containing unsaturated compound (m-1).

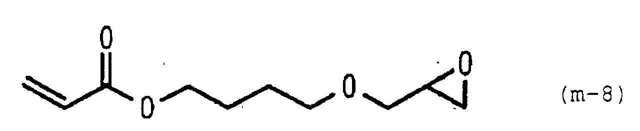

Measurement of an oxygen content in the modified propylene homopolymer (MP-10) by elemental analysis resulted in 842 ppm. From this result, the modification quantity of the epoxy group-containing unsaturated compound (m-8) in the modified propylene homopolymer was found to be 0.3 % by weight.

### Preparation of polar group-containing thermoplastic resin composition

To 10 % by weight of the modified propylene homopolymer (MP-10) obtained above, 30 % by weight of nylon 6 (NY 6, relative viscosity: 2.35) and 60 % by weight of a propylene homopolymer (PP-3) were added, and the mixture was melt kneaded by a twin-screw extruder of 20 mm diameter at 250°C to obtain a polar group-containing thermoplastic resin composition.

The polar group-containing thermoplastic resin composition was press molded under the prescribed conditions, and the properties of the molded product were measured by the aforesaid test methods. The results are set forth in Table 5.

### Example 21

### Preparation of polar group-containing thermoplastic resin composition

To 10 % by weight of the modified propylene homopolymer (MP-10) obtained in Example 20, 30 % by weight of polyethylene terephthalate (PET, intrinsic viscosity: 0.77 dl/g) and 60 % by weight of a propylene homopolymer (PP-3) were added, and the mixture was melt kneaded by a twin-screw extruder of 20 mm diameter at 280°C to obtain a polar group-containing thermoplastic resin composition.

The polar group-containing thermoplastic resin composition was press molded under the prescribed conditions, and the properties of the molded product were measured by the aforesaid test methods. The results are set forth in Table 5.

### Example 22

A modified ethylene/propylene copolymer (MP-11) was obtained in the same manner as in Example 1, except that the epoxy group-containing unsaturated compound (polar group-containing unsaturated compound (m-8)) was used instead of the polar group-containing unsaturated compound (m-1) and the ethylene/propylene copolymer (EPC-1) was used instead of the propylene homopolymer (PP-1).

Measurement of an oxygen content in the modified ethylene/propylene copolymer (MP-11) by elemental analysis resulted in 2246 ppm. From this result, the modification quantity of the polar group-containing unsaturated compound (m-8) in the modified ethylene/propylene copolymer (MP-11) was found to be 0.8 % by weight.

### Preparation of polar group-containing thermoplastic resin composition

To 30 % by weight of the modified ethylene/propylene copolymer (MP-11) obtained above, 70 % by weight of polyethylene terephthalate (PET, intrinsic viscosity: 0.77 dl/g) was added, and the mixture was melt kneaded by a twin-screw extruder of 20 mm diameter at 280°C to obtain a polar group-containing thermoplastic resin composition.

The polar group-containing thermoplastic resin composition was press molded under the prescribed conditions, and the properties of the molded product were measured by the aforesaid test methods. The results are set forth in Table 5.

### Comparative Example 9

To 30 % by weight of polyethylene terephthalate (PET, intrinsic viscosity: 0.77 dl/g), 70 % by weight of a propylene homopolymer (PP-3) was added, and the mixture was melt kneaded by a twin-screw extruder of 20 mm diameter at 280°C to obtain a polar group-containing thermoplastic resin composition.

The polar group-containing thermoplastic resin composition was press molded under the prescribed conditions, and the properties of the molded product were measured by the aforesaid test methods. The results are set forth in Table 5.

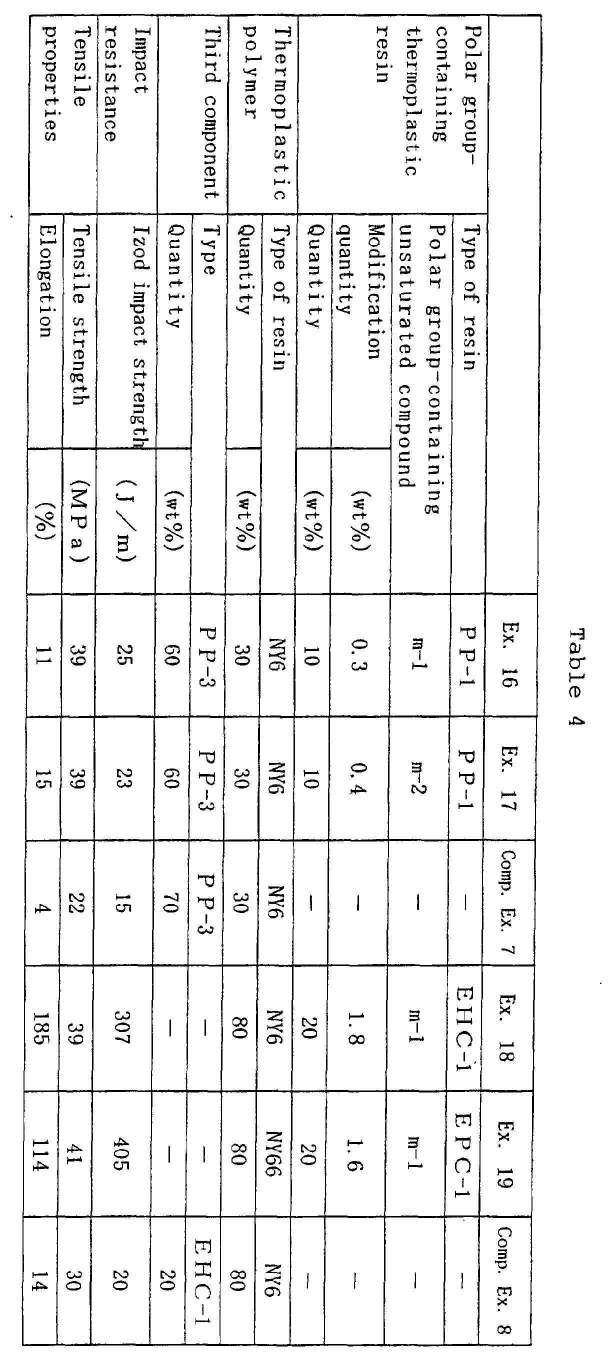

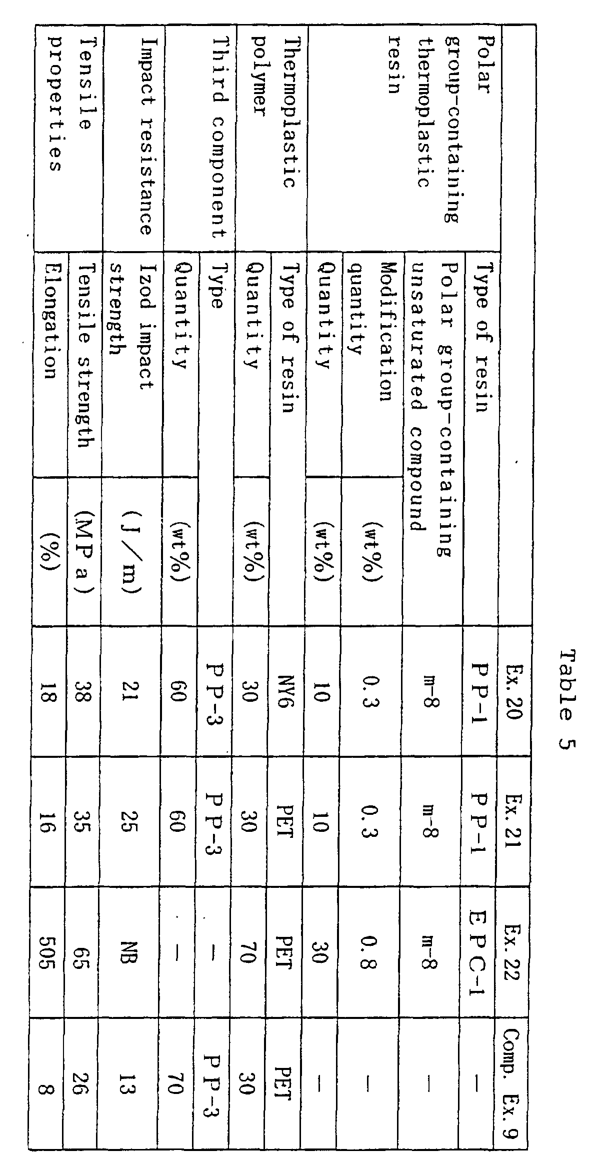

### Synthesis Example 9

### Synthesis of 4-(3-nadiimidopropyl)trimellitic anhydride

A xylene solution containing endomethylenetetrahydrophthalic anhydride (5-norbornene-2,3-dicarboxylic acid, 1.64 g, 0.01 mol), 3-amino-1-propanol (0.75 g, 0.01 mol) and sulfuric acid (20 mg) was stirred under heating, and the reaction was performed for 3 hours while water produced as a by-product was removed by azeotropic distillation. Then, the solvent was distilled off to obtain 3-nadiimido-1-propanol (2.53 g, 0.0099 mol).

Then, to a methylene chloride solution (20 ml) of trimellitic anhydride chloride (2.17 g, 0.010 mol), a methylene chloride solution (20 ml) containing 3-nadiimido-1-propanol (2.53 g, 0.0099 mol) and pyridine (0.83 ml) was dropwise added over a period of 50 minutes with ice cooling. After stirring for 2 hours at room temperature, the reaction mixture was concentrated and purified by silica gel column chromatography to obtain 4-(3-nadiimidopropyl)trimellitic anhydride (3.51 g, 0.00888 mol). The results of ¹H-NMR (270 MHz) analysis of the resulting polar group-containing unsaturated compound and the structural formula of the compound were given below.

### Analytical results

¹H-NMR (ppm): 8.65 (s, 1H), 8.57 (dd, 1H), 8.12 (dd, 1H), 6.15 (m, 2H), 4.32 (t, 2H), 3.55 (t, 2H), 3.40 (m, 2H), 3.28 (m, 2H), 1.85 (m, 2H), 1.74 (m, 2H), 1.56 (m, 2H)

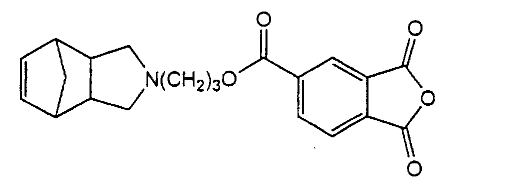

### Synthesis Example 10

### Synthesis of p-isopropenylphenyltrimellitic anhydride

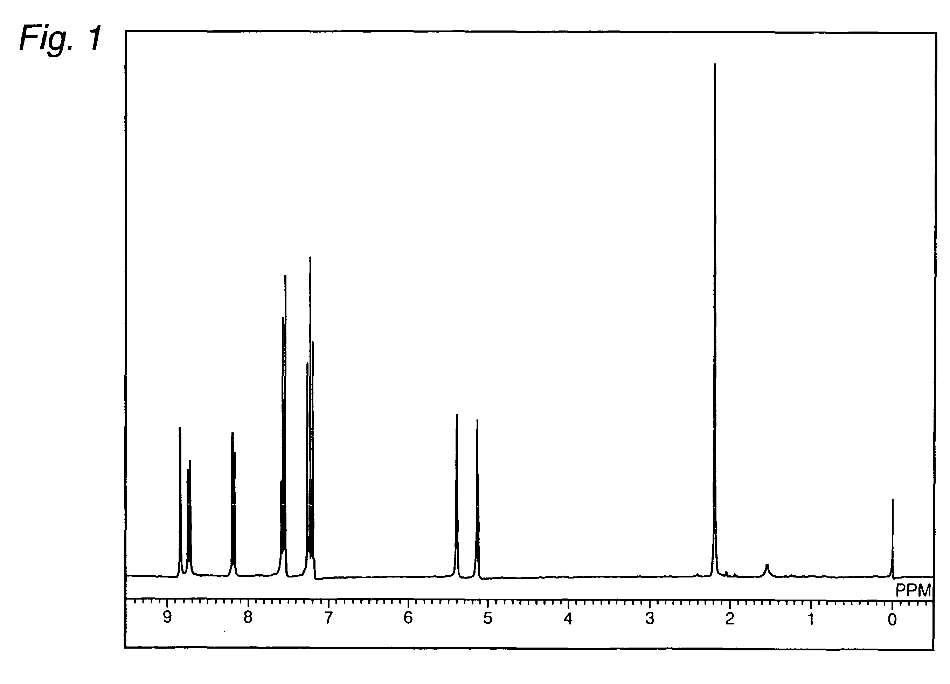

To a methylene chloride solution (50ml) of trimellitic anhydride chloride (2.11 g, 0.0100 mol), a methylene chloride solution (50 ml) containing p-isopropenylphenol (1.34 g, 0.0100 mol) and pyridine (0.80 ml) was dropwise added over a period of 10 minutes with ice cooling. After stirring for 6 hours at room temperature, the reaction mixture was concentrated, then purified by silica gel column chromatography and recrystallized from ethyl acetate to obtain of p-isopropenylphenyltrimellitic anhydride (1.54 g, 0.000500 mol). The results of ¹H-NMR (270 MHz) analysis of the resulting polar group-containing unsaturated compound are given below and shown in Fig. 1. The structural formula of the compound is given below.

### Analytical results

¹H-NMR (ppm): 8.83 (s, 1H), 8.71 (dd, 1H), 8.19 (dd, 1H), 7.54 (m, 2H), 7.23 (m, 2H), 5.39 (s, 1H), 5.14 (s, 1H), 2.18 (s, 3H)

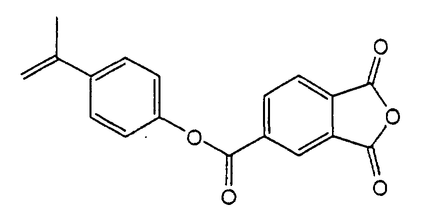

### Synthesis Example 11

### Synthesis of p-isopropenylphenoxyethyltrimellitic anhydride

A N,N-dimethylformamide solution (50 ml) containing p-isopropenylphenol (4.03 g, 0.0300 mol), ethylene carbonate (5.84 g, 0.00663 mol) and potassium carbonate (4.98 g) was placed in a flask and stirred for 12 hours under heating. To the reaction mixture, water (200 ml) was added, and the mixture was filtered. The resulting white powder was washed several times with water to obtain p-(2-hydroxyethoxy)isopropenylbenzene (4.47 g, 0.025 mol).

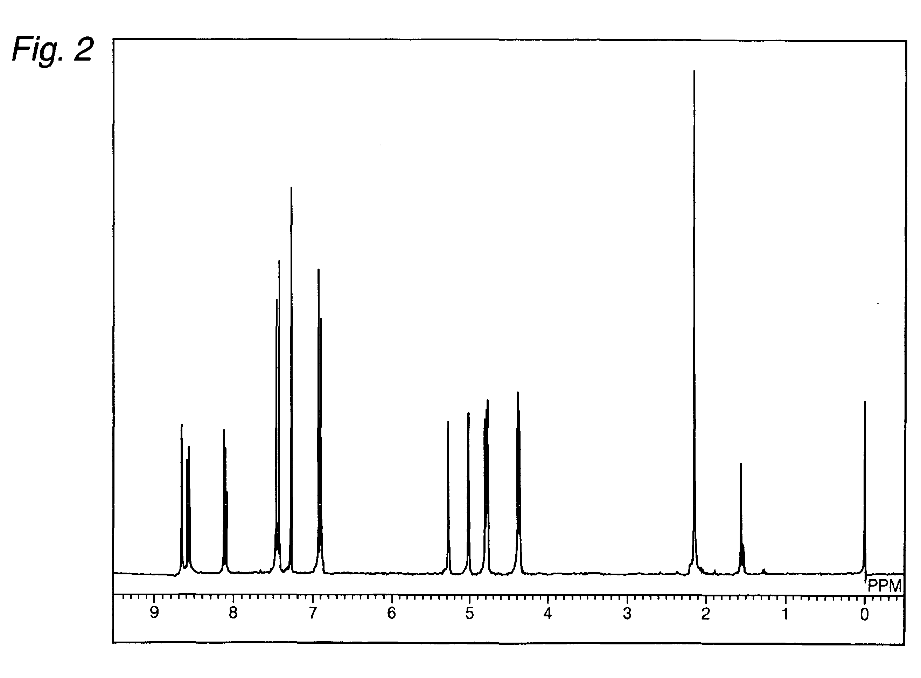

To a methylene chloride solution (50ml) of trimellitic anhydride chloride (5.47 g, 0.0260 mol), a methylene chloride solution (50 ml) containing p-(2-hydroxyethoxy)isopropenylbenzene (4.41 g, 0.0247 mol) and pyridine (2.10 ml) was dropwise added over a period of 10 minutes with ice cooling. After stirring for 6 hours at room temperature, the reaction mixture was concentrated and purified by silica gel column chromatography to obtain p-isopropenylphenoxyethyltrimellitic anhydride (8.10 g, 0.0230 mol). The results of ¹H-NMR (270 MHz) analysis of the resulting polar group-containing unsaturated compound are given below and shown in Fig. 2. The structural formula of the compound is given below.

### Analytical results

¹H-NMR (ppm) : 8.65 (s, 1H), 8.57 (dd, 1H), 8.10 (dd, 1H), 7.40 (m, 2H), 6.91 (m, 2H), 5.28 (s, 1H), 5.01 (s, 1H), 4.77 (t, 2H), 4.36 (t, 2H), 2.12 (s, 3H)

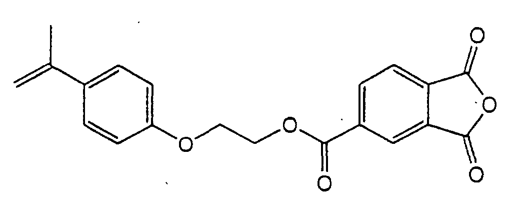

### Synthesis Example 12

### Synthesis of acryloyloxyethyl glycidyl succinate

To an acetone solution (100 ml) of β-acryloyloxyethyl hydrogensuccinate (20g, 0.092 mol, commercially available), potassium carbonate (6.6 g, 0.048 mol) was added, and the mixture was refluxed under heating. The solvent was distilled off. To the residue, 100 ml of toluene was added, and epichlorohydrin (20 ml, 0.26 mol) was further dropwise added at room temperature over a period of 20 minutes. The mixture was stirred for 1 hour with refluxing under heating and then cooled to room temperature. The reaction mixture was concentrated and purified by silica gel column chromatography to obtain acryloyloxyethyl glycidyl succinate (6.0 g, 0.022 mol)

### Analytical results

¹H-NMR (ppm): 6.43 (dd, 1H), 6.14 (dd, 1H), 5.87 (dd, 1H), 4.43 (d, 1H), 4.36 (m, 4H), 3.95 (dd, 1H), 3.20 (dd, 1H), 2.84 (dd, 1H), 2.69 (m, 4H), 2.65 (dd, 1H)

## Claims

1. A polar group-containing thermoplastic polymer obtained by graft modifying a thermoplastic polymer with at least one kind of a polar group-containing unsaturated compound represented by the following formula (I):
A-X-R²-(Y)ₚ-R¹ (I)
wherein A is a group containing a double bond,
X is -COO-, -OCO-, -NHCO-, -CO-, -O-, -CONR¹⁰-or-NR¹⁰, R¹⁰ is a hydrogen atom or a hydrocarbon group, and R¹⁰ may be bonded to A or R² to form a cyclic structure (in this case, R¹⁰ may be a single bond),
R² is a divalent group containing a carbon atom or a single bond,
Y is -CO-, -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
p is 0 or 1,
R¹ is at least one polar group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group, or any one of an alkyl group of 1 to 20 carbon atoms, a cycloalkyl group of 3 to 20 carbon atoms and an aryl group of 6 to 20 carbon atoms each of which contains at least one group selected from these polar groups, and
the bond chain linking the double bond in A to at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group in R¹ is constituted of 6 or more atoms.

2. Thepolargroup-containingthermoplasticpolymer as claimed in claim 1, wherein, in the formula (I), the bond chain linking C=O in the carboxylic acid group or the derivative thereof or C=O in the acid anhydride group in R¹ to X, or the bond chain linking the amino group, the cyano group, the isocyanate group, the sulfonic acid group, the metallic salt thereof, the phosphoric acid group, the metallic salt thereof or the epoxy group in R¹ to X is constituted of 5 or more atoms.

3. The polar group-containing thermoplastic polymer as claimed in claim 1 or 2, wherein, in the formula (I), R¹ is at least one polar group selected from the group consisting of a carboxylic acid group, a derivative thereof, an acid anhydride group, an amino group, a cyano group, an isocyanate group, a sulfonic acid group, a metallic salt thereof, a phosphoric acid group and a metallic salt thereof, or any one of an alkyl group of 1 to 20 carbon atoms, a cycloalkyl group of 3 to 20 carbon atoms and an aryl group of 6 to 20 carbon atoms each of which contains at least one group selected from these polar groups.

4. The polar group-containing thermoplastic polymer as claimed in claim 1 or 2, wherein, in the formula (I), R¹ is an epoxy group, or any one of an alkyl group of 1 to 20 carbon atoms, a cycloalkyl group of 3 to 20 carbon atoms and an aryl group of 6 to 20 carbon atoms each of which contains an epoxy group.

5. The polar group-containing thermoplastic polymer as claimed in claim 4, wherein, in the formula (I), when X is a group other than -NR¹⁰-, R² is a divalent group containing a carbon atom, and when X is -NR¹⁰-, R² is an alkylene group, a cycloalkylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

6. The polar group-containing thermoplastic polymer as claimed in any one of claims 1 to 5, wherein, in the formula (I), A is a group containing a double bond selected from the following groups: 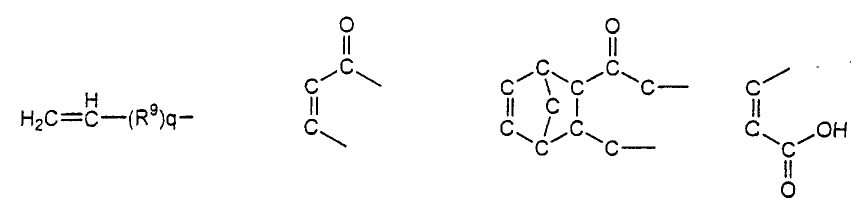 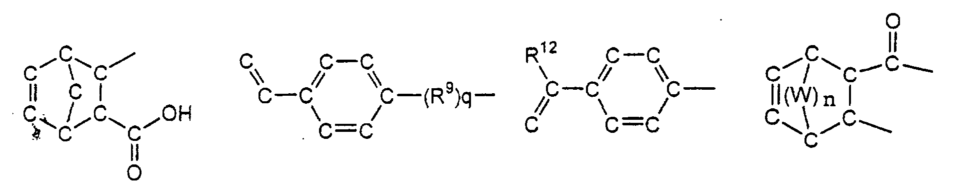 wherein R⁹ is an alkylene group of 1 to 10 carbon atoms, an arylene group of 6 to 10 carbon atoms or a cycloalkylene group of 3 to 10 carbon atoms,
q is 0 or 1,
R¹² is a hydrogen atom or a methyl group,
W is -CH(R¹³)- (with the proviso that R¹³ is a hydrogen atom or an alkyl group of 1 to 5 carbon atoms), and
n is 0 or 1.

7. The polar group-containing thermoplastic polymer as claimed in any one of claims 1 to 6, wherein the compound (I) is a compound represented by any one of the following formulas (I-a) to (I-f): 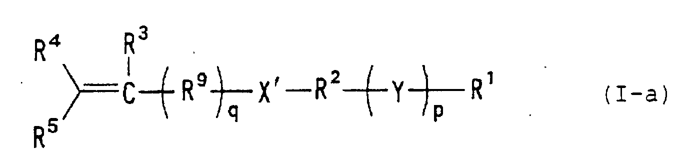 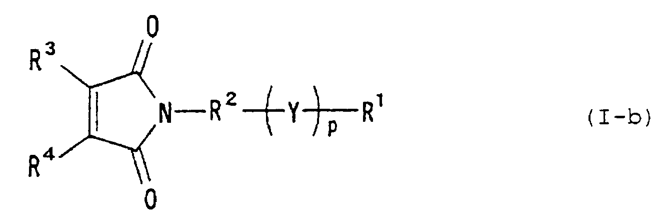 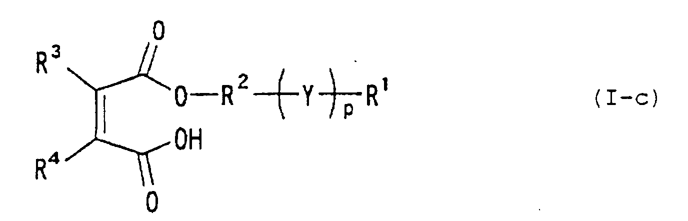 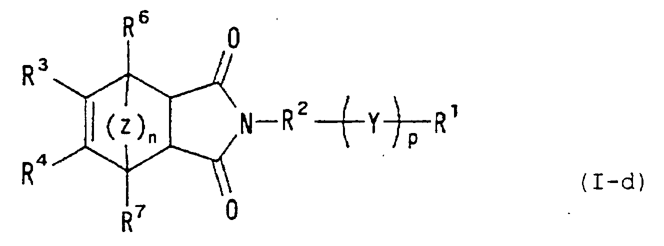 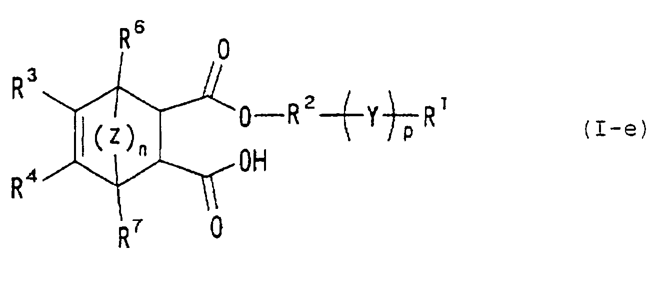 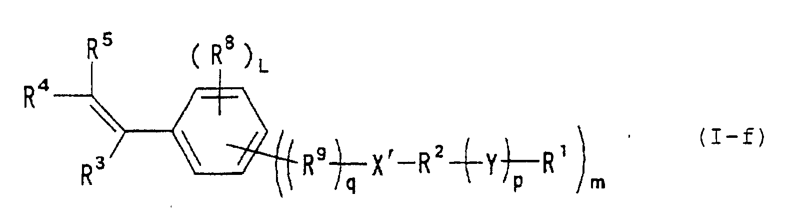 wherein R³, R⁴, R⁵, R⁶, R⁷ and R⁸ are each a hydrogen atom, an alkyl group of 1 to 10 carbon atoms or an aryl group of 6 to 15 carbon atoms,
R⁹ is an alkylene group of 1 to 10 carbon atoms, an arylene group of 6 to 10 carbon atoms or a cycloalkylene group of 3 to 10 carbon atoms,
p is 0 or 1,
q is 0 or 1,
Z is -CH₂- or -O-,
n is an integer of 0 to 2, and in case of n = O, there is no linkage between the carbon to which R⁶ is bonded and the carbon to which R⁷ is bonded,
X' is -COO-, -OCO-, -NHCO-, -CO-, -O- or -CONR¹⁰-, R¹⁰ is a hydrogen atom or a hydrocarbon group, and R¹⁰ may be bonded to a group to which X' is linked or R² to form a cyclic structure (in this case, R¹⁰ may be a single bond),
R² is a divalent group containing a carbon atom,
Y is -CO-, -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
R¹ is any one of an alkyl group of 1 to 20 carbon atoms, a cycloalkyl group of 3 to 20 carbon atoms and an aryl group of 6 to 20 carbon atoms each of which contains at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group,
L is an integer of 0 to 4,
m is an integer of 1 to 5, L+m = 5, and in case of L≥2, each R⁸ may be the same or different, and
the bond chain linking the double bond R⁴R⁵C=CR³ in the formula (I-a), the double bond R⁴C=CR³ in the formula (I-b), the double bond R⁴C=CR³ in the formula (I-c), the double bond R⁴C=CR³ in the formula (I-d), the double bond R⁴C=CR³ in the formula (I-e) or the double bond R⁴R⁵C=CR³ in the formula (I-f) to at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group in R¹ is constituted of 6 or more atoms.

8. The polar group-containing thermoplastic polymer as claimed in claim 7, wherein, in the formulas (I-a) to (I-f), the bond chain linking C=O in the carboxylic acid group, the derivative thereof or the acid anhydride group in R¹ to each of X' in the formula (I-a), N in the formula (I-b), COO of -COOR²- in the formula (I-c), N in the formula (I-d), COO of -COOR²- in the formula (I-e) and X' in the formula (I-f), or the bond chain linking the amino group, the cyano group, the isocyanate group, the sulfonic acid group, the metallic salt thereof, the phosphoric acid group, the metallic salt thereof or the epoxy group in R¹ to each of X' in the formula (I-a), N in the formula (I-b), COO of -COOR²- in the formula (I-c), N in the formula (I-d), COO of -COOR²- in the formula (I-e) and X' in the formula (I-f) is constituted of 5 or more atoms.

9. The polar group-containing thermoplastic polymer as claimed in any one of claims 1 to 8, wherein, in the formula (I), R² is a single bond, an alkylene group, a cycloalkylene group, an arylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

10. The polar group-containing thermoplastic polymer as claimed in any one of claims 1 to 8, wherein, in the formula (I), R² is an alkylene group of 2 to 20 carbon atoms, a cycloalkylene group of 3 to 20 carbon atoms, an arylene group of 6 to 20 carbon atoms, a polyoxyalkylene group of 2 to 20 carbon atoms, -R^{a}COOR^{b}-or-R^{a}OCOR^{b}- (with the proviso that R^{a} and R^{b} may be the same or different and are each a group selected from an alkylene group of 2 to 20 carbon atoms, an arylene group of 6 to 20 carbon atoms, a cycloalkylene group of 3 to 20 carbon atoms and a polyoxyalkylene group of 2 to 20 carbon atoms).

11. The polar group-containing thermoplastic polymer as claimed in any one of claims 1 to 10, which is a polymer selected from a polyolefin, a polyester, a polyamide and a polystyrene.

12. A polar group-containing thermoplastic polymer having, in the polymer side chain, a structural part represented by the following formula (I'):
-A'-X-R²-(Y)ₚ-R¹ (I')
wherein A' is a group linking the polymer chain to X or a single bond,
X is -COO-, -OCO-, -NHCO-, -CO-, -O-, -CONR¹⁰- or -NR¹⁰-, R¹⁰ is a hydrogen atom or a hydrocarbon group, and R¹⁰ may be bonded to A or R² to form a cyclic structure (in this case, R¹⁰ may be a single bond),
R² is a divalent group containing a carbon atom or a single bond,
Y is -CO-, -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
p is 0 or 1,
R¹ is any one of an alkyl group of 1 to 20 carbon atoms, a cycloalkyl group of 3 to 20 carbon atoms and an aryl group of 6 to 20 carbon atoms each of which contains at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group, and
the bond chain linking the carbon atom of the polymer main chain, at which the side chain is bonded to the main chain, to at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group in R¹ is constituted of 6 or more atoms.

13. The polar group-containing thermoplastic polymer as claimed in claim 12, wherein, in the formula (I'), the bond chain linking C=O in the carboxylic acid group or the derivative thereof or C=O in the acid anhydride group in R¹ to X, or the bond chain linking the amino group, the cyano group, the isocyanate group, the sulfonic acid group, the metallic salt thereof, the phosphoric acid group, the metallic salt thereof or the epoxy group in R¹ to X is constituted of 5 or more atoms.

14. The polar group-containing thermoplastic polymer as claimed in claim 12 or 13, wherein, in the formula (I'), X is -COO-, -OCO-, -NHCO-, -CO-, -O- or -CONR¹⁰-, and R² is an alkylene group of 3 to 20 carbon atoms, an arylene group, a cycloalkylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

15. An adhesive resin comprising the polar group-containing thermoplastic polymer of any one of claims 1 to 14.

16. A polar group-containing thermoplastic polymer composition comprising:
(A) the polar group-containing thermoplastic polymer of any one of claims 1 to 14, and
(B) a thermoplastic polymer other than the polar group-containing thermoplastic polymer (A),
wherein the polar group-containing thermoplastic polymer (A) is contained in an amount of 1 to 99 % by weight and the thermoplastic polymer (B) is contained in an amount of 99 to 1 % by weight.

17. The polar group-containing thermoplastic polymer composition as claimed in claim 16, wherein the thermoplastic polymer (B) is at least one polymer selected from an olefin polymer and a diene rubber.

18. The polar group-containing thermoplastic polymer composition as claimed in claim 16 or 17, wherein the thermoplastic polymer (B) is a thermoplastic polymer containing, as main recurring units, recurring units derived from a monomer containing a polar group.

19. The polar group-containing thermoplastic polymer composition as claimed in claim 16 or 17, wherein the thermoplastic polymer (B) is a thermoplastic polymer selected from the group consisting of a polyamide resin, a polyester resin, an acrylic resin, a polyphenylene sulfide resin, a polycarbonate resin, a polyacetal resin, a polyphenylene oxide resin, a polyarylate resin and a polysulfone resin.

20. A molded product obtained by molding the polar group-containing thermoplastic polymer composition of any one of claims 16 to 19.

21. An adhesive resin composition comprising the polar group-containing thermoplastic polymer composition of any one of claims 16 to 19.

22. A polar group-containing unsaturated compound represented by the following formula (I):
A-X-R²-(Y)ₚ-R¹ (I)
wherein A is a group containing a double bond,
X is -COO-, -OCO-, -NHCO-, -CO-, -O-, -CONR¹⁰- or -NR¹⁰-, R¹⁰ is a hydrogen atom or a hydrocarbon group, and R¹⁰ may be bonded to A or R² to form a cyclic structure (in this case, R¹⁰ may be a single bond),
R² is a divalent group containing a carbon atom or a single bond,
Y is -CO-, -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
p is 0 or 1,
R¹ is any one of an alkyl group of 1 to 20 carbon atoms, a cycloalkyl group of 3 to 20 carbon atoms and an aryl group of 6 to 20 carbon atoms each of which contains at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group, and
the bond chain linking the double bond in A to at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group in R¹ is constituted of 6 or more atoms.

23. The polar group-containing unsaturated compound as claimed in claim 22, wherein, in the formula (I), the bond chain linking C=O in the carboxylic acid group or the derivative thereof or C=O in the acid anhydride group in R¹ to X, or the bond chain linking the amino group, the cyano group, the isocyanate group, the sulfonic acid group, the metallic salt thereof, the phosphoric acid group, the metallic salt thereof or the epoxy group in R¹ to X is constituted of 5 or more atoms.

24. The polar group-containing unsaturated compound as claimed in claim 22 or 23, wherein, in the formula (I), R¹ is an epoxy group, or any one of an alkyl group of 1 to 20 carbon atoms, a cycloalkyl group of 3 to 20 carbon atoms and an aryl group of 6 to 20 carbon atoms each of which contains an epoxy group.

25. The polar group-containing unsaturated compound as claimed in any one of claims 22 to 24, wherein, in the formula (I), when X is a group other than -NR¹⁰-, R² is a divalent group containing a carbon atom, and when X is -NR¹⁰-, R² is an alkylene group, a cycloalkylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

26. The polar group-containing unsaturated compound as claimed in claim 22 or 23, wherein, in the formula (I), R¹ is any one of an alkyl group of 1 to 20 carbon atoms, a cycloalkyl group and an aryl group each of which contains at least one group selected from a carboxylic acid group, a derivative thereof and an acid anhydride group.

27. The polar group-containing unsaturated compound as claimed in any one of claims 22 to 26, wherein, in the formula (I), A is a group containing a double bond selected from the following groups: 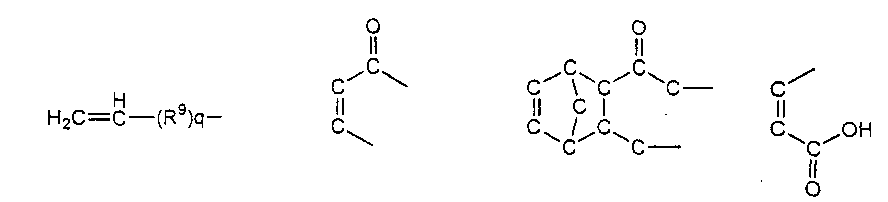 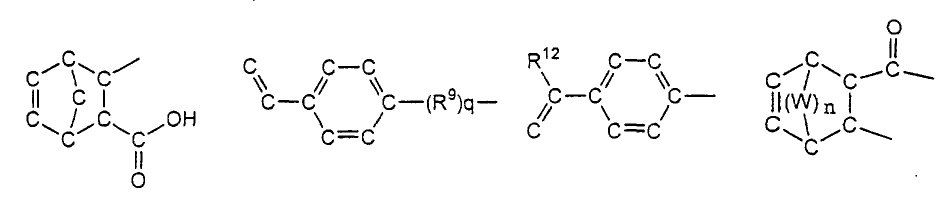 wherein R⁹ is an alkylene group of 1 to 10 carbon atoms, an arylene group of 6 to 10 carbon atoms or a cycloalkylene group of 3 to 10 carbon atoms,
q is 0 or 1,
R¹² is a hydrogen atom or a methyl group,
W is -CH(R¹³) - (with the proviso that R¹³ is a hydrogen atom or an alkyl group of 1 to 5 carbon atoms), and
n is 0 or 1.

28. The polar group-containing unsaturated compound as claimed in any one of claims 22 to 27, which is a compound represented by any one of the following formulas (I-a) to (I-f): 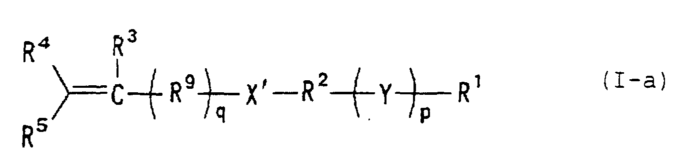 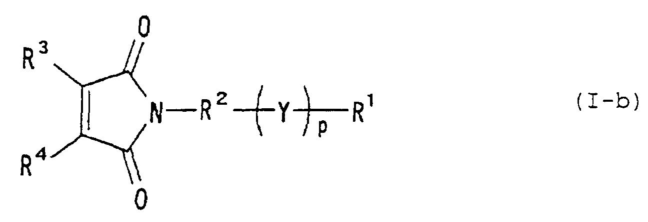 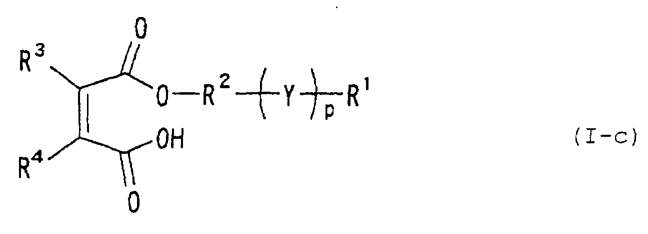 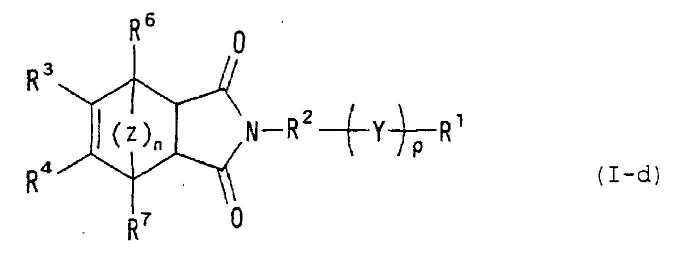 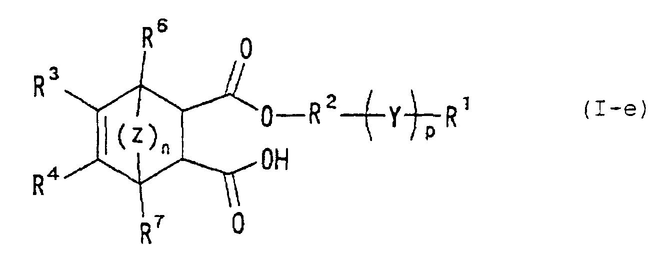 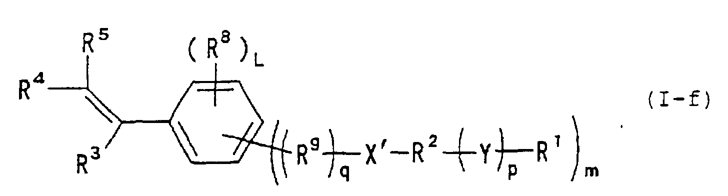 wherein R³, R⁴, R⁵, R⁶, R⁷ and R⁸ are each a hydrogen atom, an alkyl group of 1 to 10 carbon atoms or an aryl group of 6 to 10 carbon atoms,
R⁹ is an alkylene group of 1 to 10 carbon atoms, an arylene group of 6 to 10 carbon atoms or a cycloalkylene group of 3 to 10 carbon atoms,
p is 0 or 1,
q is 0 or 1,
Z is -CH₂- or -O-,
n is an integer of 0 to 2, and in case of n = 0, there is no linkage between the carbon to which R⁶ is bonded and the carbon to which R⁷ is bonded,
X' is -COO-, -OCO-, -NHCO-, -CO-, -O- or -CONR¹⁰-, R¹⁰ is a hydrogen atom or a hydrocarbon group, and R¹⁰ may be bonded to a group to which X' is linked or R² to form a cyclic structure (in this case, R¹⁰ may be a single bond),
R² is a divalent group containing a carbon atom,
Y is -CO-, -COO-, -OCO-, -O-, -CONR¹¹-, -OCONR¹¹- or -NR¹¹CO-, R¹¹ is a hydrogen atom or a hydrocarbon group, and R¹¹ may be bonded to R² or R¹ to form a cyclic structure (in this case, R¹¹ may be a single bond),
R¹ is any one of an alkyl group of 1 to 20 carbon atoms, a cycloalkyl group of 3 to 20 carbon atoms and an aryl group of 6 to 20 carbon atoms each of which contains at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group,
L is an integer of 0 to 4,
m is an integer of 1 to 5, L+m = 5, and in case of L≥2, each R⁸ may be the same or different, and
the bond chain linking the double bond R⁴R⁵C=CR³ in the formula (I-a), the double bond R⁴C=CR³ in the formula (I-b), the double bond R⁴C=CR³ in the formula (I-c), the double bond R⁴C=CR³ in the formula (I-d), the double bond R⁴C=CR³ in the formula (I-e) or the double bond R⁴R⁵C=CR³ in the formula (I-f) to at least one group selected from the group consisting of a carboxylic acid group, a derivative thereof, an acid anhydride group, an amino group, a cyano group, an isocyanate group, a sulfonic acid group, a metallic salt thereof, a phosphoric acid group, a metallic salt thereof and an epoxy group in R¹ is constituted of 6 or more atoms.

29. The polar group-containing unsaturated compound as claimed in claim 28, wherein, in the formulas (I-a) to (I-f), the bond chain linking C=O in the carboxylic acid group, the derivative thereof or the acid anhydride group in R¹ to each of X' in the formula (I-a), N in the formula (I-b), COO of -COOR²- in the formula (I-c), N in the formula (I-d), COO of -COOR²- in the formula (I-e) and X' in the formula (I-f), or the bond chain linking the amino group, the cyano group, the isocyanate group, the sulfonic acid group, the metallic salt thereof, the phosphoric acid group, the metallic salt thereof or the epoxy group in R¹ to each of X' in the formula (I-a), N in the formula (I-b), COO of -COOR²- in the formula (I-c), N in the formula (I-d), COO of -COOR²- in the formula (I-e) and X' in the formula (I-f) is constituted of 5 or more atoms.

30. The polar group-containing unsaturated compound as claimed in any one of claims 22 to 29, wherein, in the formula (I), R² is a single bond, an alkylene group, a cycloalkylene group, an arylene group, a polyoxyalkylene group, -R^{a}COOR^{b}- or -R^{a}OCOR^{b}- (with the proviso that R^{a} and R^{b} may be the same or different and are each a group selected from an alkylene group, an arylene group, a cycloalkylene group and a polyoxyalkylene group).

31. The polar group-containing unsaturated compound as claimed in any one of claims 22 to 29, wherein, in the formula (I), R² is an alkylene group of 2 to 20 carbon atoms, a cycloalkylene group of 3 to 20 carbon atoms, an arylene group of 6 to 20 carbon atoms, a polyoxyalkylene group of 2 to 20 carbon atoms, -R^{a}COOR^{b}- or -R^{a}OCOR^{b}- (with the proviso that R^{a} and R^{b} may be the same or different and are each a group selected from an alkylene group of 2 to 20 carbon atoms, an arylene group of 6 to 20 carbon atoms, a cycloalkylene group of 3 to 20 carbon atoms and a polyoxyalkylene group of 2 to 20 carbon atoms).

32. The polar group-containing unsaturated compound as claimed in claim 22, which is a polar group-containing unsaturated imide compound represented by the following formula (I-g): 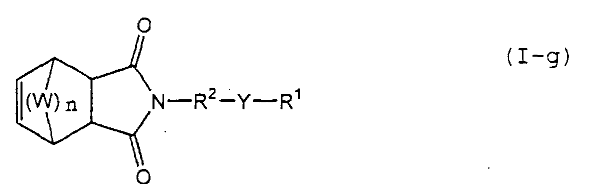 wherein W is -CH(R¹³)- (with the proviso that R¹³ is a hydrogen atom or an alkyl group of 1 to 5 carbon atoms),
n is 0 or 1,
R² is a straight-chain or branched alkylene group or an arylene group,
Y is -O₂C-, and
R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group.

33. The polar group-containing unsaturated compound as claimed in claim 32, wherein, in the formula (I-g), R¹ is a phthalic anhydride group.

34. The polar group-containing unsaturated compound as claimed in claims 32 or 33, wherein, in the formula (I-g), W is -CH₂-, n is 1, R² is a straight-chain or branched alkylene group, and R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group.

35. The polar group-containing unsaturated imide compound as claimed in any one of claims 32 to 34, wherein, in the formula (I-g), R² is a straight-chain alkylene group of 2 to 10 carbon atoms.

36. The polar group-containing unsaturated compound as claimed in claim 22, which is a polar group-containing vinylbenzene derivative represented by the following formula (I-h): 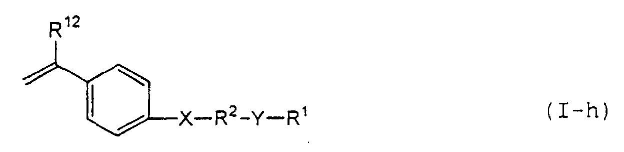 wherein R¹² is a hydrogen atom or a methyl group,
X is an oxygen atom
R² is single bond or a (poly)oxyalkylene group,
Y is a carbonyl group, and
R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group.

37. The polar group-containing unsaturated compound as claimed in claim 36, wherein, in the formula (I-h), R¹² is a methyl group.

38. The polar group-containing unsaturated compound as claimed in claim 36 or 37, wherein the (poly)oxyalkylene group indicated by R² is represented by the following formula (1) or (2) :
-(CH₂CH₂O)ₙ- (1)
wherein n is an integer of 1 to 10,
-(CH₂CHCH₃O)ₘ- (2)
wherein m is an integer of 1 to 10.

39. The polar group-containing unsaturated compound as claimed in any one of claims 36 to 38, wherein, in the formula (I-h), R¹ is any one of an alkyl group of 1 to 20 carbon atoms, a cycloalkyl group of 3 to 20 carbon atoms and an aryl group of 6 to 20 carbon atoms each of which contains a phthalic anhydride group.

40. A polar group-containing thermoplastic polymer having a recurring unit represented by the following formula (I-h'): 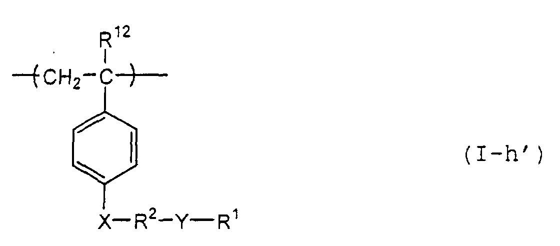 wherein R¹² is a hydrogen atom or a methyl group,
X is an oxygen atom,
R² is single bond or a (poly)oxyalkylene group,
Y is a carbonyl group,
R¹ is any one of an alkyl group of 1 to 20 carbon atoms, a cycloalkyl group of 3 to 20 carbon atoms and an aryl group of 6 to 20 carbon atoms each of which contains a carboxylic anhydride group or a carboxyl group, and
the bond chain linking the carbon of a carbonyl group in the carboxylic anhydride group or the carbon of a carbonyl group in the carboxyl group to X is constituted of 5 or more atoms.

41. The polar group-containing unsaturated compound as claimed in claim 22, which is a glycidyl polyester acrylate compound represented by the following formula (I-i): 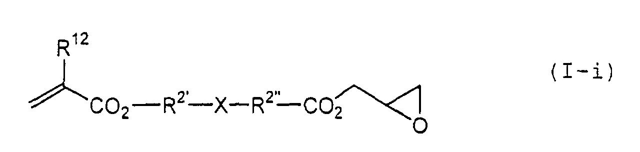 wherein R¹² is a hydrogen atom or a methyl group,
R^{2'} is a straight-chain or branched alkylene group, an oxyalkylene group, an arylene group or a cycloalkylene group,
X is -CO₂- or -O₂C-, and
R²" is a straight-chain or branched alkylene group, an oxyalkylene group, an arylene group or a cycloalkylene group.

42. The polar group-containing unsaturated compound as claimed in claim 41, wherein, in the formula (I-i), R¹² is a hydrogen atom or a methyl group, R^{2'} is a straight-chain or branched alkylene group, an oxyalkylene group or a cycloalkylene group, X is -O₂C-, and R^{2"} is a straight-chain alkylene group.

43. The polar group-containing unsaturated compound as claimed in claim 41, wherein, in the formula (I-i), R¹² is a hydrogen atom, R^{2'} is a straight-chain alkylene group of 2 to 4 carbon atoms, X is -O₂C-, and R^{2"} is a straight-chain alkylene group of 2 to 4 carbon atoms.

44. The polar group-containing unsaturated compound as claimed in claim 41, wherein, in the formula (I-i), R¹² is a hydrogen atom, R^{2'} is ethylene, X is -O₂C-, and R^{2"} is ethylene.

45. A process for preparing a polar group-containing unsaturated compound, comprising:
allowing acrylic acid or methacrylic acid to react with an alkylene oxide or an alkylene carbonate or allowing an acryloyl halide to react with an alkanediol to obtain a terminal hydroxyl group-containing acrylate,
allowing a terminal hydroxyl group of the terminal hydroxyl group-containing acrylate to react with an acid anhydride to obtain a carboxyl group,
then allowing to react with epichlorohydrin in the presence of a base to obtain the polar group-containing unsaturated compound of any one of claims 41 to 44.

46. A compound for radical reaction, comprising the polar group-containing unsaturated compound of any one of claims 22 to 45.

47. A polymer modifier comprising the polar group-containing unsaturated compound of any one of claims 22 to 45.
